# EUROPEAN PATENT APPLICATION

(11) **EP 3 040 959 A1**
(43) Date of publication of application: **06.07.2016**
(21) Application number: 15203231.4
(22) Date of filing: 31.12.2015
(51) Int. Cl.: G09B 1/00, G09B 23/28

(54) **SURGICAL SIMULATOR SYSTEM AND METHOD**

(30) Priority: 29.12.2014 US 201462097504 P; 29.12.2015 US 201514983037; 29.12.2015 WO PCT/US2015/067919; 29.12.2015 IN 4919MU2015
(71) Applicant: Help Me See Inc., New York, NY 10018 (US)
(72) Inventor: Thazhathu, Mohan, New York, NY 11780-2167 (US); Ueltschi, James, Florida, FL 32963-3512 (US)
(74) Representative: Boult Wade Tennant

(57) **Abstract**

A surgical simulator comprising a haptic arm capable of simulating forces generated during surgery from interactions between a surgical tool and tissue operated upon. The simulator further comprises a visual display capable of depicting a three-dimensional image of the simulated surgical tool and a physics-based computer model of the tissue. The haptic arm controls the movement and orientation of the simulated tool in the three-dimensional image, and provides haptic feedback forces to simulate forces experienced during surgery. Methods for simulating surgery and training users of the simulator are also described.

## Description

### FIELD OF THE INVENTION

This invention relates to the field of surgical simulation, and particularly to a surgical simulator system and method of surgery with haptic force feedback.

### BACKGROUND OF THE INVENTION

In many parts of the world, people suffer from blindness even though it could be cured. The blind cannot see to work, to care for themselves, or care for anyone else and in most cases do not have access to proper medical care. Many of the blind die as a result of their blindness. Much of the blindness in the world today could be cured because blindness is often caused by cataracts that can be removed from the eye to restore sight. Sadly, the resources are simply not available to provide this cure. 30 million people are blind today who could be cured. In ten years the number will double unless something is done.

Compared to other types of disabilities, the impact of blindness is particularly destructive and can be economically devastating. In most cases, loss of sight from cataracts occurs gradually over many years and results in reduced quality of life, reduced disposable income for the family, and increased reliance on family care takers (often children who should be in school). General health is impacted as a result of increased risk for injuries, inability to see injuries in order to care for them properly (like cuts and scrapes that can get infected), and reduced ability to maintain proper nutrition. In developing nations, resources for the blind are scarce or non-existent, white cane policies (providing awareness and safety for the blind) and other disability legislation are often lacking, and the family carries the burden alone. As a result, cataract blindness is also associated with extreme poverty and increased risk of death.

The fact that cataracts are common also makes blindness an expected disability in developing nations, especially among the poor. It is understood to be part of the aging process and is accepted as such. Information about cataracts and cataract surgery is lacking or incorrect. Patients who have gone to traditional healers or poorly trained surgeons are often given bad information and poor treatment. Surgical treatment is feared and many people would rather stay blind than undergo surgery. As a result, blind patients in developing nations often come to believe that their only choice is to live with the blindness, longing to be free of the dark world in which they have been forced to live, but unable to do anything about it.

Phacoemulsification (PE) cataract surgery and conventional extracapsular cataract extraction (ECCE) surgery, with its variants, are the two primary cataract surgery techniques used universally. A popular variant of ECCE surgery that can be performed without sutures is known as manual small incision cataract surgery (MSICS). Although PE cataract surgery is considered the gold standard for cataract removal, it requires expensive machinery and an uninterrupted power source. The overall cost and maintenance of machinery and supplies for PE makes it cost-prohibitive for regions with inadequate infrastructure. PE also has a higher rate of intraoperative complications when it is performed on patients who have advanced cataracts.

MSICS and ECCE are widely practiced outside North America and Europe. A distinct advantage of MSICS over ECCE is that a smaller incision is utilized to remove the cataractous natural lens and implant an intraocular lens (IOL). The smaller incision is also fashioned to be self-sealing and sutureless. This translates into shorter healing times, significantly less astigmatism, reduced intraoperative risk, and overall shorter post-operative recovery. ECCE and MSICS are not dependent on any powered machinery other than an operating microscope. When comparing different surgical techniques and circumstances of application all over the world, MSICS is often the preferred technique for high volume cataract surgery. MSICS can be used to safely and cost effectively restore vision in developing nations where most of the blind live and with the same quality that would be expected in the high tech world. Though there are many variations on the technique, the basic idea of MSICS revolves around properly producing and utilizing a tunnel large enough to deliver even dense cataracts but stable enough to be self-sealing and have minimal impact on the curvature of the cornea.

Even though MSICS is a well proven alternative to address the problem of cataract blindness in developing nations, there is a lack of developing nation eye surgeons skilled in the technique. In some Sub-Saharan African countries for example, there is on average, one ophthalmologist per million individuals. To deal with the burden of global cataract blindness, there is an urgent need to train a substantial number of surgeons in the technique of MSICS. Global cataract blindness rates can be successfully decreased by increasing the number and skill level of available surgeons. A significant part of this training need can be met by high-quality, high efficiency simulation based training with no patient risk.

There is therefore a need for a surgical simulator system and method that allows the user to master manual small incision cataract surgery or MSICS.

### SUMMARY OF INVENTION

An object of the present invention is to provide surgical simulator systems and methods that provide visual, haptic, and audio cues to a user to simulate an MSIC surgery.

A further object of the present invention is to provide simulation systems and methods that model tissue, use of surgical tools, and interactions between the surgical tools and the modeled tissue, and allow a user to practice and become proficient in a surgical procedure.

Another object of the present invention is to provide simulation systems and methods that gradually introduce a comprehensive array of realistic patient factors and surgical complications so that the trainee experiences the live feel of surgery, including the many variables and errors that can occur.

Yet another object of the present invention is to provide simulation systems and methods that allow a trainee's performance to be monitored and evaluated for feedback, remedial instruction, scoring and progression.

A further object of the present invention is to provide simulation systems and methods that use a mesh model to build and display visuals using rasterization or ray tracing, while a physical model that influences the properties, such as collision detection and tissue deformation, runs in parallel.

### BRIEF DESCRIPTION OF THE DRAWINGS

For a fuller understanding of the invention, reference is had to the following description of the accompanying Figures. Like reference numbers are used to refer to like and corresponding elements of the various Figures.
FIG. 1 is a depiction of a simulated surgical environment.
FIG. 2 is a chart depicting the interaction between a simulated surgical environment and computer components.
FIG. 3a is a chart illustrating hardware components of a simulator.
FIG. 3b is a chart illustrating software components of a simulator
FIG. 3c is a chart illustrating computer components of a simulator.
FIG. 3d is a chart illustrating an exemplary connection between a simulator, a central server and instructor clients.
FIGs. 4a to 4f depict simulated physical eye models.
FIGs. 5a to 5c depict simulated images visible through a simulated microscope.
FIG. 6a is a table of exemplary tools that may be simulated by a haptic right arm.
FIG. 6b is a table of exemplary tools that may be simulated by a haptic left arm.
FIGs. 7a and 7b depicts the steps for performing MSIC surgery and the tools that may be simulated by haptic arms.

### DESCRIPTION OF THE INVENTION

The invention may be understood more readily by reference to the following detailed description of a preferred embodiment of the invention. However, techniques, systems, and operating structures in accordance with the invention may be embodied in a wide variety of forms and modes, some of which may be quite different from those in the disclosed embodiment. Consequently, the specific structural and functional details disclosed herein are merely representative, yet in that regard, they are deemed to afford the best embodiment for purposes of disclosure and to provide a basis for the claims herein, which define the scope of the invention. Further, it will be apparent to those of skill in the art that numerous changes may be made in such details without departing from the spirit and the principles of the invention. It must be noted that, as used in the specification and the appended claims, the singular forms "a", "an", and "the" include plural referents unless the context clearly indicates otherwise.

The present invention is described in the context of MSIC surgery. However, the present invention may be used to simulate other types of surgical procedures performed on the human eye and on other parts of the human body. The present invention may also be used to simulate veterinary surgery.

The present invention may stem the tide of cataract blindness by significantly increasing the number of effective MSICS surgeons in the world. The surgical simulator of the present invention provides MSICS training on a large scale. The provided training is comprehensive, trainee advancement is based on performance, and successful completion results in the opportunity for the trainee to become part of the global network of cataract surgeons. The training assumes no previous knowledge of or experience with eye care. Success in the training program is performance based - the trainees prove that they have the basic language, intellectual, and motor skills needed to master MSICS surgery by demonstrating the required performance in order to progress through the training program. Data gathered on admission will be compared with performance during training to refine the admissions testing and establish standardized criteria.

Using the surgical simulator system and method of the present invention, the user can progress methodically and efficiently through the learning process, experiencing a spectrum of surgical challenges and variations in a relatively short period of time and without ever endangering a patient. Upon successful completion of the simulator-based training, the trainee returns to surgical facilities in their own countries to begin live surgical training under the supervision of a mentoring surgeon. Normally live surgical training may take years to complete and often leaves gaps in the experience. But because of the advanced simulation experience, the users of the present invention can progress rapidly to independent, high quality work. The approximate transition to independent surgical care should take between one to six months.

The surgical simulator and method gradually introduces through simulation a comprehensive array of realistic patient factors and surgical complications associated with each step so that the trainee experiences the live feel of surgery including the many variables and errors that can occur. Simulator assisted training provides a safe and efficient means to prepare for live surgical experience. The training experience presents a wide variety of surgical situations to develop confidence and surgical intuition for live surgery without risk to patients. Trainee performance on assignments on the simulator is monitored and evaluated for feedback, remedial instruction, scoring and progression. Each trainee will practice and learn until desired level of proficiency is demonstrated on the simulator.

For MSICS to be successful, the following five tasks must be completed in sequence during simulation: (1) complete patient preparation, (2) create a self-sealing tunnel into the eye, (3) remove the cataract, (4) insert the intraocular lens ("IOL"), (5) restore conditions that optimize the healing process.

The MSICS simulator comprises the hardware and software to display an eye, both visually and haptically. The simulator provides visual, haptic, and audio cues to the user to simulate a realistic MSIC surgery. The haptic and visual rendering are both derived from live surgical force data, MSICS expert subjective evaluation and objective validation of these models. The haptic rendering will provide the trainee with virtually the same forces on the instruments as experienced during live surgery. The visual rendering will reproduce the images in a non-pixilated stereoscopic display that a surgeon would see in the binocular operating microscope with photorealistic quality. The modeling generally includes: (1) tool-tissue interaction, (2) tissue to tissue interactions, (3) connections between tissues, (4) dissection of tissues, (5) alteration of tissue properties, (6) intraocular pressure, (7) injection and aspiration of fluid, (8) spread of fluid, (9) playing sounds in response to events, and (10) model patient head movement.

The MSICS simulator consists of four major simulation elements: (a) a simulator with haptic arms, (b) a physics-based computer model, (c) a visual image generator, and (d) an instructor/student operator station.

### I. Simulator with Haptic Arms

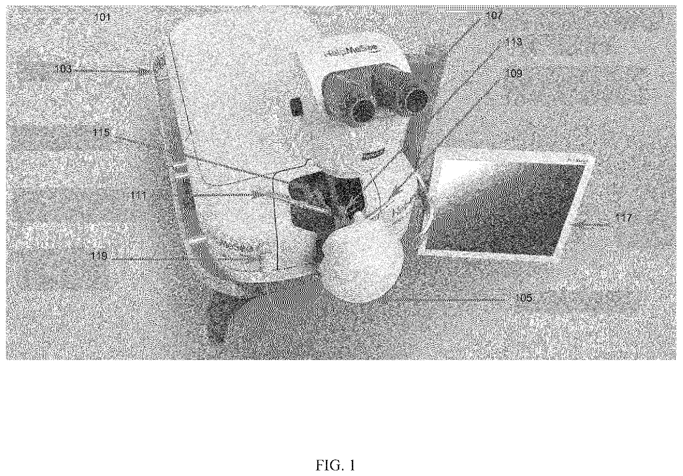

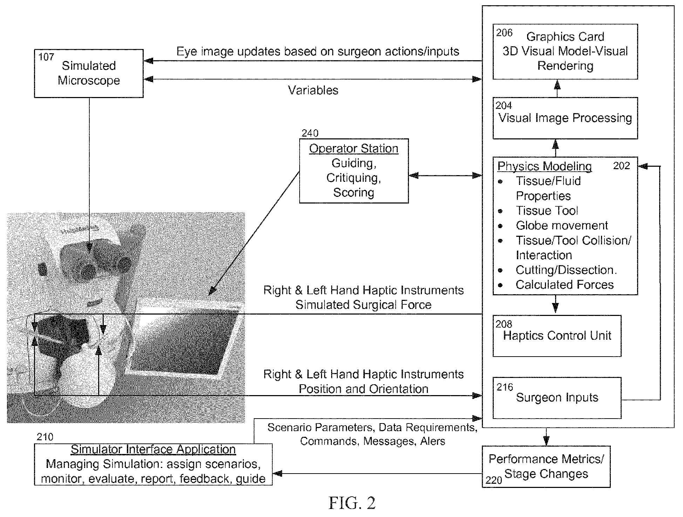

With reference to FIGs. 1 and 2, the simulator (101) may comprise a gurney body (103) from which a simulated model of a patient's head (105) extends. The simulator may further include a simulated microscope (107) through which the user may perceive a simulation. A haptic right arm (109) and a haptic left arm (111) may be connected to and controlled by a right haptic mechanism (113) and left haptic mechanism (115), respectively. The simulator (101) further comprises a touch screen (117) and a simulated aspiration syringe instrument (119).

A surgeon's hands and fingers are too large to directly manipulate the small and delicate tissues of the eye. As such, to perform MSIC surgery, a surgeon uses certain instruments. In the present invention, those instruments are simulated by the haptic right arm (109) and the haptic left arm (111). The arms are provided in the workspace and held in a user's right and left hands to simulate the surgery. The haptic arms (109, 111) are used to perform actions on a virtual eye while looking through the viewer of the microscope (107). A user may select instruments for simulation and move the instruments into the operational area of the simulator under the microscope (107). The user can use these instruments to simulate various surgical tasks.

The haptic arms (109, 111) provide tactile realism in the form of force feedback when working on the virtual eye model. The haptic arms (109, 111) are motion control devices that provide a realistic feeling of the forces to the fingers, hands and arms used to hold the instruments as they interact with the virtual eye. The virtual eye is programmed to accurately simulate a response or behavior that would be caused by interactions between an eye and selected instruments. For example, to simulate holding down an eye and increasing pressure in the eye by pressing down on the eye with Colibri forceps, a haptic arm (109, 111) would simulate the Colibri forceps and would restrict movement of the eye in the viewer of the simulated microscope (107). The resistance of a haptic mechanism (113, 115) may be increased to simulate an increase in hardness of the eye that would be felt by the Colibri forceps or another tool that interacts with the eye. Similarly, simulating interaction with a crescent blade would result in cutting the eye tissues of the virtual eye according to the simulated blade's edge, angle, force, nature of movement etc. The simulator may also simulate interactions between two or more tools.

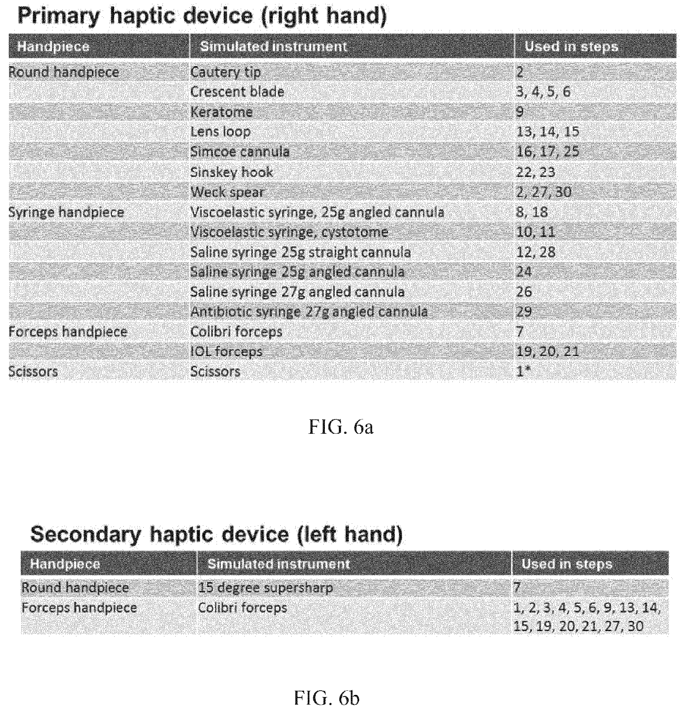

The haptic arms (109, 111) provide a simultaneous and bimanual use to represent tools used in an actual MSIC surgery. Preferably, the haptic arms (109, 111) are representative of actual surgical instruments. In one embodiment, the haptic arms (109, 111) allow the changing of handles that are representative of actual surgical instruments. In another embodiment, the haptic arms (109, 111) include permanently mounted handles that are representative but not exact replicas of actual surgical instruments. In either case, the instrument visual under the microscope (107) will change for each type of instrument. The simulator may further simulate other tools, such as for example a syringe of the Simcoe Cannula. FIGs. 6a-6b list the various tools that may be simulated by the simulator. Each tool has three translational degrees of freedom. The haptic point of interest for these translations is at the tip of the tool. Three passive rotational degrees of freedom are also provided and measured. Their rotations are centered at this same point of interest. The haptics are based on the use of admittance control, using a force sensor as an input.

As described above, the haptic rendering provides the user with virtually the same forces on the instruments as experienced during live surgery. Table 1 below lists the surgical steps that may be simulated by the simulator, the instrument that may be simulated, a direction of movement of the instrument during surgery, and maximum and minimum values of forces for each surgical step.

**Table 1**

| **Surgical step/ Maneuvers** | **Instrument** | **Direction Of Movement** | **Horizontal (Fx gms)** | **Vertical (Fy gms)** | **Anterior-posterior (Fz gms)** |
|---|---|---|---|---|---|
| Scleral tunnel pocket | Crescent blade | H+AP | Max 48.8 | Max 63.6 | Max 62.5 |
| | | | Min 21.7 | Min 35.4 | Min 24.4 |
| | | | Avg 31.9 | Avg 47.0 | Avg 45.6 |
| Scleral tunnel lateral extension | Crescent blade | H | Max 115.8 | Max 87.1 | Max 95.4 |
| | | | Min 61.9 | Min 41.0 | Min 43.5 |
| | | | Avg 91.3 | Avg 60.8 | Avg 66.2 |
| Paracentesis port | 15 degree blade | AP | Max | Max | Max 55.6 |
| | | | Min | Min | Min 13.3 |
| | | | Avg | Avg | Avg 23.4 |
| AC entry via main incision | Keratome | H+AP | Max 82.2 | Max | Max 52.0 |
| | | | Min 35.4 | Min | Min 2.1 |
| | | | Avg 55.6 | Avg | Avg 22.9 |
| Capsulotomy | Cystotome | V | Max | Max 42.0 | Max 48.0 |
| | | | Min | Min 10.9 | Min 13.6 |
| | | | Avg | Avg 22.5 | Avg 23.6 |
| Lens expression | Lens vectis | AP | Max | Max | Max 66.3 |
| | | | Min | Min | Min 7.8 |
| | | | Avg | Avg | Avg 35.1 |
| IOL repositioning | Sinskey hook | AP+H+ V | Max | Max | Max 7.4 |
| | | | negligible | negligible | Min 1.6 |
| | | | Min | Min | Avg 4.7 |
| | | | Avg | Avg | |

In Table 1, all forces are shown in grams (g) and the directions of movements are represented as follows: H- horizontal, AP- antero-posterior, V- vertical. Fx represents the forces in the x plane designating the left-to-right movement, Fy represents the forces in the y plane designating the up-and-down movements, and Fz represents the forces in the z plane designating the in-and-out movements. The primary force during the pericentesis stab is Fz inwards, whereas during the "slice" maneuver using the crescent blade, Fx (to the right or left) forces dominate.

Force values set the level of force that the simulator reproduces to give the operator a realistic feel for the procedure. A minimum force establishes the upper limit for noise in the electronics and friction in the robotic mechanism beyond which the surgeon can no longer properly experience the surgical forces. A maximum force sets the standard for the size of the motors and stiffness of the robotic mechanism. The force curve characteristics provide an objective baseline for testing the realism of simulated live tissue interaction.

The most critical step of the MSICS procedure is the creation of the scleral tunnel. It is also the most difficult step to learn. The Fx of the crescent blade during back-and-forth "wiggle" motions when creating the Scleral tunnel pocket averages 31.9 grams with a maximum (max) of 48.8 g and a minimum (min) of 21.7 g. During the same motion, the surgeon is also following the contour of the globe upwards and inwards. The Fy "wiggle" motions of the crescent blade during the Scleral tunnel pocket step (which is an upwards oriented force) is averaged at 47.0 g (max 63.6 g, min 35.4 g). Fz (which is an inwards oriented force) is averaged at 45.6 g (max 62.5 g, min 24.4 g) during this same step. All three degrees of freedom have significant forces during this maneuver. It is important that the surgeon recognize that as the blade wiggles left and right it is also advancing inwards and following the contour of the globe upwards, all up to similar average forces.

The highest force encountered during the simulation is when slicing the crescent blade to the right or left during the Scleral tunnel lateral extension step. Fx of the crescent blade during the "slice" maneuver to the right or left in Scleral tunnel lateral extension step is averaged at 91.3 g (max 115.8 g, min 61.9 g). The MSICS simulator reproduces forces from zero to at least the 115.8 g amount. The y force value for this step changes depending on whether the surgeon is slicing to the right or to the left. When slicing rightwards, Fy is downward in orientation as the left-handed surgeon follows the contour of the globe. When slicing leftwards, the Fy is upward in orientation, as the right-handed surgeon extends the tunnel leftwards while grasping the outer tunnel with Colibri forceps. The z forces encountered are outward in orientation during the crescent slice regardless of the direction of slicing. Fy max when slicing to the right, a downward oriented force, has an average of 31.3 g (max 50.2 g, min 12.0 g). Fy when slicing to the left is an upward oriented force with an average of 60.8 g (max 87.1 g, min 41.0 g). The Fz force (Fz max) during the crescent "slice" maneuver is an outwards oriented force with average of 66.2 g (max 95.4 g, min 43.5 g).

Stab incision forces are predominantly z forces during cornea entry (i.e., or Paracentesis port). Fz during stab incision, or Paracentesis port, formation, using the paracentesis 15 degree blade is inwards in orientation with an average force of 23.4 g (max 55.6 g, min 13.3 g).

Although often taught to "float" in the center of the tunnel slicing the keratome right or left, a novice surgeon would recognize that significant Fx forces can still be encountered, up to an average of 55.6 g, especially toward the far extent of the maneuver. When entering the anterior chamber using the 3.0 mm keratome (i.e., AC entry via main incision), the Fz max (inwards) averages 22.9 g (max 52.0 g, min 2.1 g). The "slicing" with the keratome to open the inner wound, the Fx (right or left) averages 55.6 g (max 82.2 g, min 35.4 g).

The cystotome can-opener forces are provided in the y and z degrees of freedom. There are no significant forces when cutting the anterior capsule during each stroke. The actual cut stroke of the cystotome has minimal forces — a surgeon cannot feel the cystotome cutting the anterior capsule. However, a significant reposition force (Fy upwards and Fz outwards) signature is provided immediately after each cutting stroke. Fy for the Capsulotomy step is an upwards oriented force which averages 22.5 g (max 42.0 g, min 10.9 g) and Fz in an outwards oriented force averages 23.6 g (max 48.0 g, min 13.6 g).

Fz (oriented outwards) during the vectis expression of the crystalline lens averages 35.1 g (max 66.3 g, min 7.8 g). Sinskey forces are minimal when dialing the IOL, highlighting that to properly dial an IOL under viscoelastic control requires minimum forces. Maximum forces in any degree of freedom when dialing the IOL with the Sinskey hook at the 9 o'clock position are negligible, with an average of 4.7 g (max 7.4 g, min 1.6 g).

### II. Physics Based Computer Model

FIG. 2 is a chart illustrating the flow of data between simulator components during a simulation. A physics modeling application (202) models the tissue and fluid properties of the simulated eye and the interaction of the tools with the eye, including the forces applied by tools to the eye. Information concerning the visual appearance of the eye and the tools may be processed by a visual image processing application (204) and delivered to a graphics card (206) for 3-D model rendering. The 3-D image of the eye is transmitted to the simulated microscope (107) and may be viewed by a user during a simulation.

A haptics control unit (208) receives simulation modeling information from the physics modeling application. The haptics control unit (208) further receives surgical input information (216) from the haptic arms (109, 111) concerning the position and orientation of the haptic arms (109, 111). The haptics control unit (208) controls the amount of force and resistance applied by the haptic mechanisms (113, 115) to the haptic arms (109, 111).

The simulator further includes a simulator interface application (210) that manages the simulation. The simulator interface application (210) allows instructors to assign surgical scenarios to users, and monitor, evaluate, provide feedback to and report on users concerning their operation of the simulator.

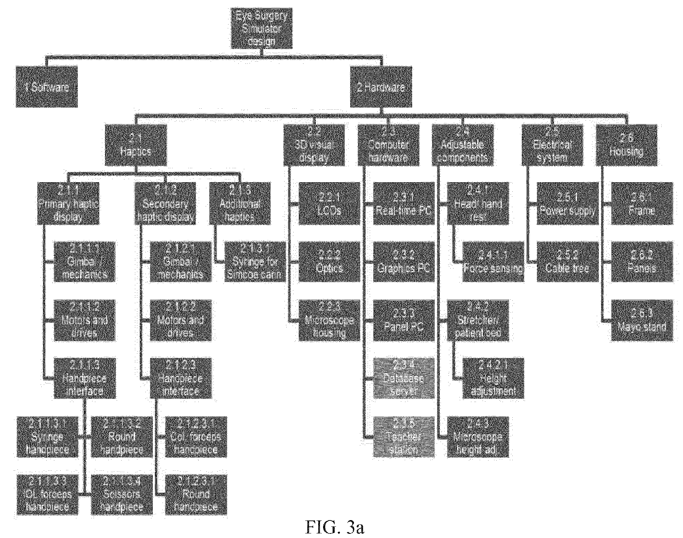

FIG. 3a is a chart illustrating the interrelation of hardware components of the simulator. The haptic components (2.1) may include Gimbal mechanisms (2.1.1.1, 2.1.2.1), motors and drives (2.1.1.2, 2.1.2.2), and hand piece interfaces (2.1.1.3, 2.1.2.3). The 3-D visual display (2.2) of the simulated microscope (107) may include LCDs (2.2.1), optics (2.2.2) and a microscope housing (2.2.3). Computer hardware (2.3) used by, sending information to, or receiving information from the simulator includes a real-time PC (2.3.1), a graphics PC (2.3.2), a panel PC (2.3.3), a database server (2.3.4) and a teaching station (2.3.5). A simulated head (2.4.1) - depicted in Figure 1 by reference element (105) - and a stretcher/patient bed (2.4.2) - depicted in Figure 1 by reference element (103) - may also be provided.

### III. Visual Image Generator

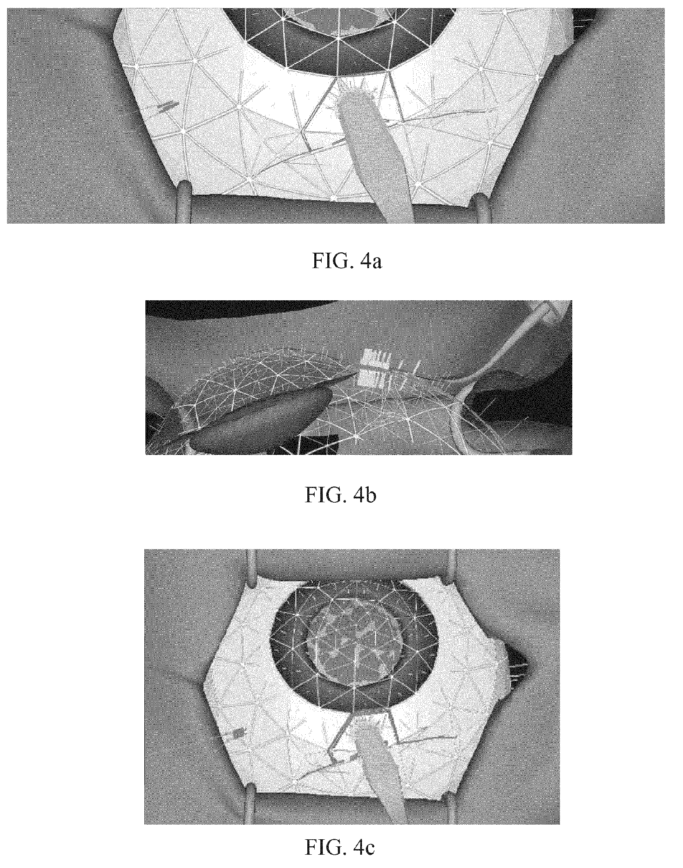

The simulator represents a visual graphical model of the eye that simulates a realistic MSIC surgery. A physics based model of the eye is programmed to simulate the eye behavior in response to surgical actions. The visual 3-D eye model will change according to these actions in real time to give the experience of working with a real eye. The virtual eye has customizable parameters that allow changing not only the way it appears (such as color of iris, skin, sclera etc.) but also other anatomical and technical parameters such as its shape, cataract type etc. in order to provide practice with wide range of patient conditions that a surgeon is expected to be exposed to. FIGs. 4a-4f illustrate the physics model depiction.

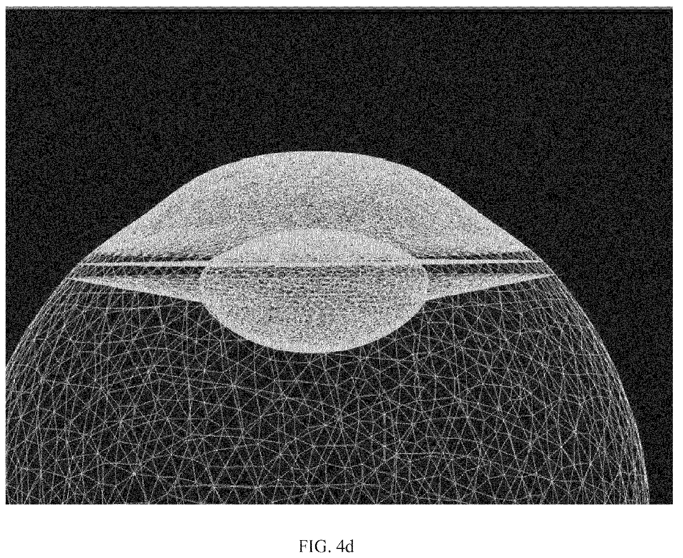

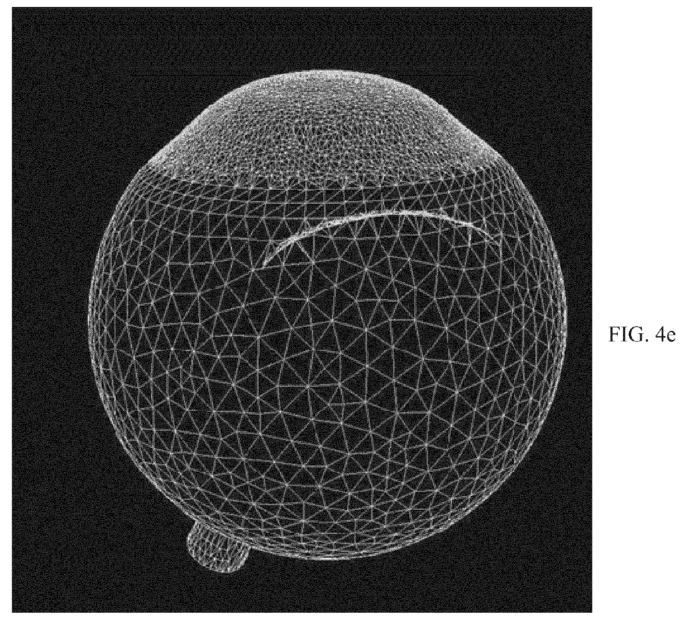

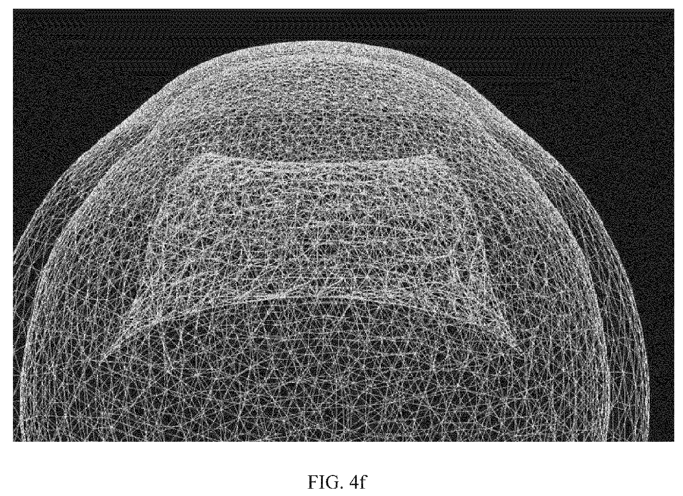

As shown in FIGs. 4a-4f, a mesh model is used to build and display the visuals using rasterization, while a physical model that influences the properties, such as collision detection and tissue deformation, runs in parallel. The eye model includes all the important structures involved in the MSICS surgery. The eye model includes high detail for the corneal and limbus. This achieves a realistic reflection from its surface. FIG. 4d shows a wireframe rendering of the eye from the side illustrating the high detail overlaps the limbus. FIG. 4e shows the main tunnel as it enters the eye, and FIG. 4f illustrates a wireframe of the main cut, as will be later described.

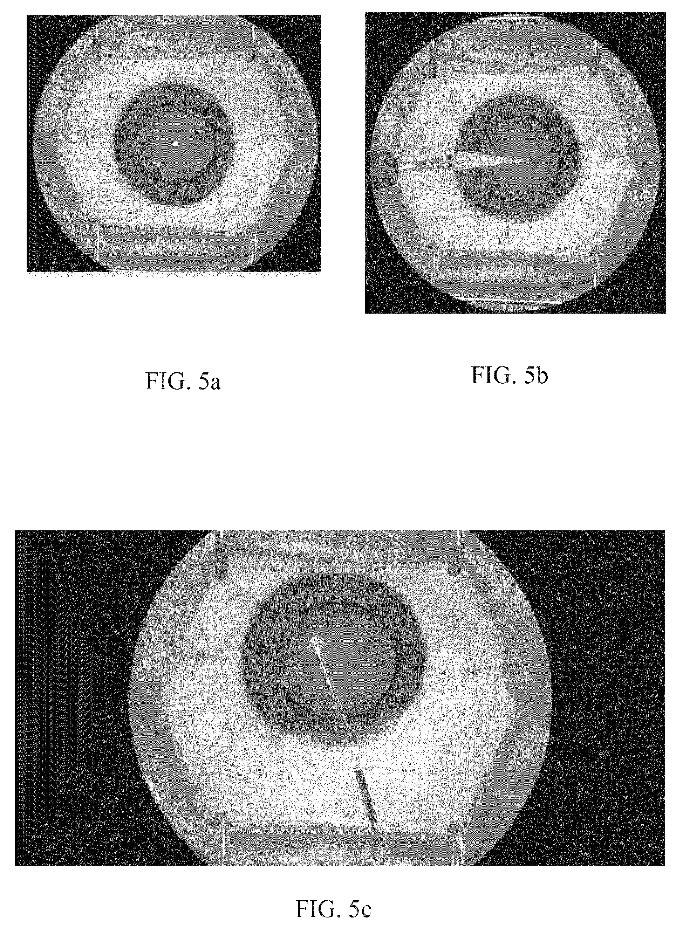

The simulator contains two visual displays that a user can work with, including the (1) microscope view, and (2) an external display screen. FIGs. 5a-5c illustrate the simulated images within the microscope view. Objects in the simulation are perceived to move and deform with physical realism. The trainee is presented with an image of the visual model through a 3-D visual display that resembles a stereoscopic microscope. Using the microscope, the trainee sees the virtual eye model and interacts with it using the haptic arms (physical forms representing surgical instruments) to perform the tasks while looking through the microscope eyepieces. The visual display depicts stereoscopic 3-D image of the eye as would be seen under an operating microscope at 5X magnification showing everything within a surgical field of 34 mm to 35 mm circular area. The image of the field is preferably surrounded by a black ring 5 mm in width, making the total visual image 4 cm in diameter. In a preferred embodiment, rasterization is used for rendering the visuals for the eye-model. In other embodiments, ray tracing may be used, or a combination of rasterization and ray tracing.

The external display allows the trainee and the instructor to communicate with the simulator. Communications include, but not limited to the following: selection of the surgical assignment to practice, review the assignment related information on screen before beginning performance, looking at feedback content (multimedia - text, audio, video, animations, etc.), and parallel display of microscope view when assignment is being performed, etc. The external display therefore contains a control panel by which the learner and/or instructor exercise what control he has over simulations.

### IV. Software and Computer Components

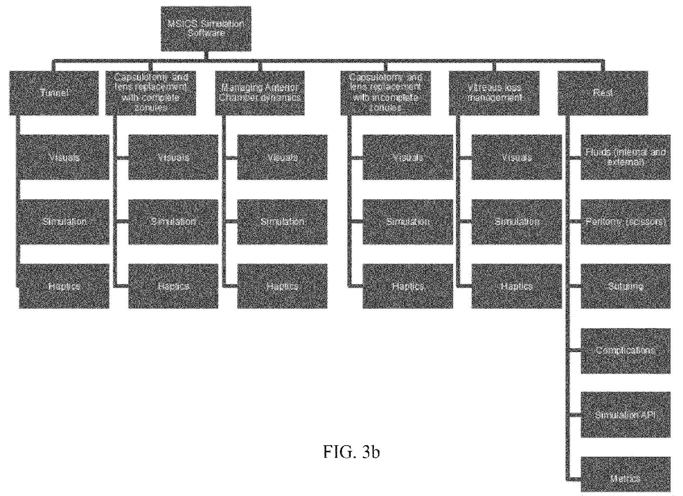

FIG. 3b illustrates software components of the simulator, including components for simulating (1) the tunnel, (2) capsulotomy and lens replacement with complete zonules, (3) managing anterior chamber dynamics, (4) capsulotomy and lens replacement with incomplete zonules, and (5) vitreous loss management. Each of those components has a visual, simulation, and haptics subcomponent.

The simulator software is programmed to support performance of the entire MSICS procedure on the MSICS simulator. The software is built around physics based models of the eye, the surgical instruments and the forces experienced during live surgery. Particularly, the software contains the graphic eye models, haptic models, and the courseware / user interface. The haptic and visual responses to interaction with the model are nearly indistinguishable from real surgery. This model reproduces precise tool-tissue contact, realistic tissue resistance, stiffness, flexibility and texture providing realistic feeling of handling the instruments with the simulator hand pieces. The simulator provides sufficient degrees of freedom for movement to allow proper simulation and force feedback.

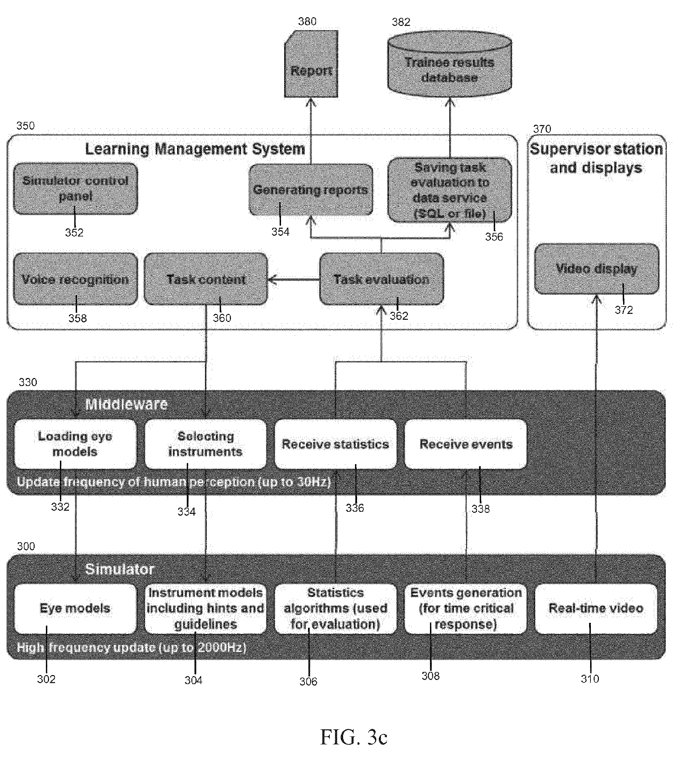

As shown in FIG. 3c, the simulator consists of a plurality of computers, including the Simulator computer, the Courseware or "Simulator Interface Application" (SIA) computer, and the Application Programming Interface (API). A simulator computer, or "graphics and/or real time PC", hosts and runs the simulator software (3-D eye and physics model) and related code. The Simulator computer (300) includes components for eye models (352), instrument models including hints and guidelines (354), statistics algorithms used for evaluations (356), events generation for time critical response (358), and real-time video (360).

A Courseware (Panel PC) computer (350) runs the "Simulator Interface Application" (SIA). The Courseware computer (350) may include components for a simulator control panel (352), generating reports (354), saving task evaluation to data service (356), voice recognition (358), task content (360), and task evaluation (362). Trainee results may be saved to a trainee results database (382). The supervisor station (370) includes a video display (372). The principal function of the (SIA) is to communicate with the simulator in order to deliver training. The SIA allows monitoring, interacting with, and managing the particular student that is currently practicing on the simulator as well as record that student's performance data. To accomplish this, the SIA interacts with the Simulator Based Learning System (SBLS), as described below, to enable the student to view active Assignments (allowed based on prerequisite conditions), and to download specific Assignment files and content / learning resources corresponding to the Assignment selected by the student. Upon completion of the Assignment performance, the SIA uploads the student's performance data at the end of each attempt and the session. When the student reaches proficiency criteria on an Assignment, the list of active Assignments are updated. The SIA may comprise similar modules described below with respect to the SBLS, including: Assignment Database, Learning Experience Logic, Assignment Information Screens, Simulator Communication, Performance Records, SBLS Interface, Video encoding, streaming, and storage, Reports, and Data/Code Update. The Simulator Communication module allows the SIA to communicate with the Simulator using the Simulator API to manage Assignment performance. Such communication is based on the standard Assignments that are received from the SBLS, as described below. In order to track each Assignment performance, trainees authenticate access in order to identify them and store their performance data to their respective records.

A Simulation Application Programming Interface (API) (Middleware layer) interfaces the simulator computer with the SIA. The Middleware layer may include components for loading eye models (332), selecting instruments (334), receiving statistics (336), and receiving events (338). API can be hosted and run either on the graphics PC or Panel PC. An interface is provided for Ethernet communication between the courseware and simulator in order to start simulations, trigger events, getting metrics and other communication that is necessary. The communication requirements between SIA and Simulator Middleware include: Assignment parameters - Eye model configuration and state, Performance Data - Event based, Performance Data - General (Ongoing), Event triggers, Audio Commands, Video Feed, Calibration and Pre-session checks, Performance Logs, and Guidelines, Orientation view and Real time feedback. The major elements of communication between SIA and simulator include:
*1. Surgical conditions:*
   a. Surgical Starting Point (a stage within the MSICS surgical procedure).
   b. Surgical Ending Point (a stage within the MSICS surgical procedure).
   c. Common Anatomical Variations (e.g. Color of iris, color of skin, anterior chamber depth etc.).
   d. Surgical Variations (right/left eye, intraocular pressure, type of cataract).
   e. Select intraoperative challenges (Pupil constricts during capsulotomy or IOP increase following removal of nucleus).
*2. Trailing conditions.*
   a. Specific Learning Objective(s) for the assignment (e.g. making the scleral groove having required shape and size).
   b. Performance parameters and metrics (e.g. Size of incision, parameters relating to shape of incision).
   c. Standards for evaluation corresponding to metrics (e.g. Range, not exceeding or lower than, event (Yes/No) etc.).
   d. Scoring logic - Points and/or weights and condition relating to each metric to calculate score(s).
   e. Selected training tools - guidelines/orientation view/real time feedback alerts/aural cues (setting to communicate if these training tools will be made available or not during performance).
*3. Assignment Performance data:*
   a. Receive data on assignment performance parameters, metrics, alerts, triggers etc.
   b. Receive video feed.
*4. Commands:*
   a. Start assignment (transfer and loading of assignment files).
   b. End / Abort Assignment (mark end of performance and ask to initiate performance data transfer).
   c. Verbal commands - for limited purpose (e.g. ask for instruments (to be loaded etc.).
   d. Trigger certain intraoperative challenges (e.g. Microscope bulb failure, patient complains of pain etc.).

The above is not an exhaustive description but fairly comprehensive view. There may be some limited additional communication requirements.

To deliver the training in the most effective manner, the SIA contains the following important features. It breaks down the procedure into smaller segments for unit practice. This allows repeated practice with segments to help focus on specific tasks or task groups. For example, assuming that the procedure is broken down into 10 tasks, one should be able to start with task 5 and stop after completing task 6. When starting at task 5, all the actions from task 1 to 4 would have already been completed and such updated state of the eye should be loaded for performance. The SIA also collects data on performance metrics (data generated on the simulator relating to the performance activity) to compare them with standards and therefore provide both formative and summative feedback. Formative feedback help improve learning. For instance, force being applied for a task was more than required resulting in poor performance or an incision made of a size much bigger than what was required. And summative feedback means assessment of performance in line with the goals. Summative feedback also involves evaluation of performance across attempts. The SIA configures scenarios same as in real life to prepare trainees suitably in managing them effectively. These could be scenarios would include intraoperative challenges such as excessive bleeding in response to an action, weak zonules that hold the capsule, and other challenges that are presented even as the task is performed accurately. Errors or suboptimal performance in earlier tasks can also lead to intraoperative challenges later in the surgery. In order to present such scenarios, without requiring a learner/trainer to create them every time they need to be practiced, the SIA saves the state of the eye such that the trainee can start practicing with that state.

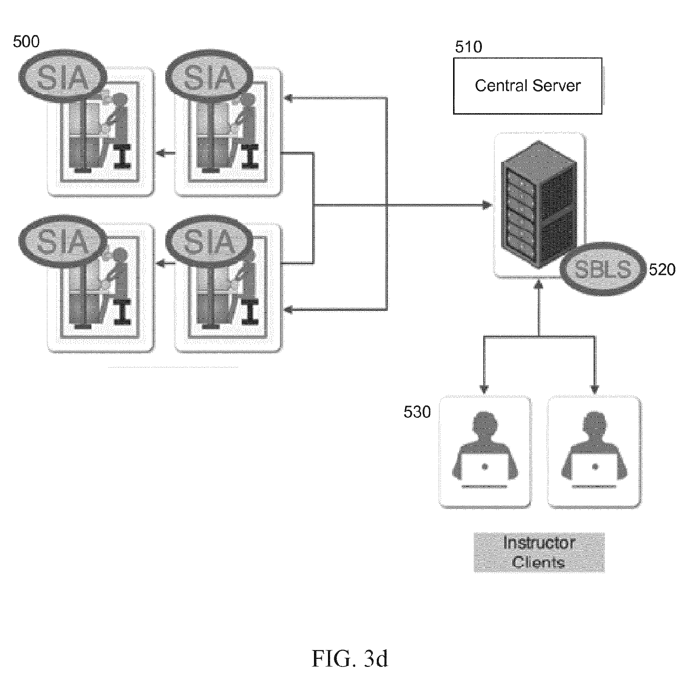

As shown in FIG. 3d, the Simulator Interface Application (SIA) (500) is part of the simulator and manages communication and data exchange with MSICS simulator for each learner and each assignment and their performance of the simulator assignments. In a preferred embodiment, the simulator is connected via an intranet to a central server (510). A server or network application, called the Simulator Based Learning System (SBLS) (520), is hosted on the central server (510) and is used to manage training delivery to a plurality of trainees. SBLS (520) manages learning management across all modes of instructional delivery simulators. It involves management of multiple learners, simulators, training delivery methods, etc. For example, the SBLS (520) may manage more than 50-100 simulators. When the simulators are operating simultaneously, a large amount of rich data in the form of video feed, etc. will be generated and exchanged between the simulators and the centralized server application (SBLS (520)). The SIA (500) communicates with the Simulation API (FIG. 3c) in order to manage simulation practice units i.e. assignments and exchange necessary information. The SBLS (520) manages delivery of the assignments for each trainee, identifies trainees, controls access to assignments, record performance data from assignment attempts, and provides other such training management features. The communication between SIA (500) and SBLS (520) includes: interface for enabling user authentication, sync up with SBLS (520) to maintain local copy of Assignment database (static files and codes relating to assignment evaluation and scoring), transfer performance data, video, etc. to SBLS (520) for updating trainee records on the SBLS (520), and instructor functionalities related communication.

SBLS (520) may comprise a plurality of modules, including the modules listed below. Preferably, a copy of selected modules or certain code from the SBLS (520) is maintained on the SIA (500). This ensures that the simulator practice can be started or continued without any dependence on the SBLS (520). As such, the SIA (500) may comprise the same modules listed below as the SBLS (520). The SIA (500) communicates with the SBLS (520) for data such as the following: user authentication, trainee status information (past records, allowable Assignments, content access relating to Assignments), data Sync with SBLS (520) (upload performance data of trainees that is stored on local system to the SBLS (520) on the network), communication with instructor client (530), transfer of video feeds, download and update latest version of SIA (500), and upload reports and issues from simulator calibration and tests.

### V. Instructor/Student Operation

### A. User Management

A module for managing various users (e.g., super user, administrator, instructor (trainer), trainee) and providing access control functionality.

### B. Assignment Development

A module to create assignments that can be assigned to the learners for practice and evaluation on the simulator. A trainee will be guided to undertake practice of a set of "Assignments" that will be designed to provide sufficient practice to the trainee on various segments of the procedure and then the entire procedure. The complexity and difficulty levels of each such assignment will increase with progress made by the trainee. The trainees are exposed to wide variety of cataract cases and complications using the assignments. The assignments are created based on selections of several conditions, training tools, performance parameters, triggers/alerts, scoring, pre-requisites, assignment meta-data etc.

Each practice unit on the MSICS simulator is called an Assignment. Each Assignment will have a starting and ending point (within the MSICS procedure) indicating the state of the eye and related parameters (e.g. color, size, type of cataract etc.). It can be a segment of the procedure wherein some tasks would have been already completed (e.g. eye with scleral groove has been made). It can also include complications to be presented etc. The SBLS supports a database of a large number of MSICS surgical Assignments using a user-friendly interface and a series of steps to capture and set all the data to define a typical assignment. Each Assignment includes all of the information that is required by the simulator to:
- Begin the simulation (using selections possible for surgical conditions, training conditions, i.e. appropriate eye model variables, patient parameters, standard/custom starting point in the surgical procedure.
- Define access to training tools - provision of guidelines, orientation view and feedback (real-time); apply other level wise settings and tolerances for errors and alerts etc., possible complications and corresponding action.
- Determine the end of the simulation based on trigger of certain conditions viz. vision threatening complications in specific assignments (level 2), abortion of Assignment by trainee, completion of Assignment performance, instructor intervention etc.
- Allow communication of alerts and performance metrics - in real-time and end of Assignment (facilitates feedback relating to errors, computation of score for the attempt, provision of detailed breakdown of scores awarded for each parameter etc.).
- Intervention by instructors to alter/trigger certain modifiable conditions.
- Validation of instruments to be used with each part of the simulation Assignment.

The Assignments also include the reference data corresponding to each performance parameter specified to enable evaluation. Corresponding to each level there is a specified value, range or setting against which the parameters will be compared to assign a score or pass/fail status.

### C. Assignment Database

The assignment database stores the assignments and corresponding data that is communicated to the simulator using XML messages/commands. The Assignment files will include following details:
- Associated performance metrics expected to be monitored and/or recorded in real-time and after the Assignment attempt has been completed.
- Guidelines, Orientation view and Real time feedback requirements (includes Aural feedback and other aural requirements)
- Scenarios for complications that can be triggered by the instructor
- Triggers for events
- Parameters (metrics) linked to feedback remedial content resources
- Reference starting point name and data files corresponding to a custom starting point (saved following a trainee or trainer performance)
- Assignment Meta data: Task Group/Task/Subtask/Movement Number according to standard proficiency definition for MSICS, Title, description, tags - level, task group etc.

A Simulator Validation Study may include a set of 30-40 Assignments, having about 8-10 unique Assignments, each with 2-3 small variations. In such variations, base assignment files can be leveraged for customization in terms of metrics to be used for scoring and alerts and triggers may change to emphasize certain specific learning objective(s) relating to the Assignment. For instance, the Assignment relating to "Making the scleral groove" might have as one of its variants an emphasis on the trainee getting the shape of the groove correct and its metrics may have higher weightage when scoring. Other metrics collected, such as length of the groove, depth of the groove etc., will carry low or no weightage. Therefore, the weightage applied to each metric for the score may vary accordingly for providing suitable emphasis corresponding to the learning objective(s).

### D. Assignment Information Screens

Assignment Information Screens are a set of screens that provide information for each Assignment, including graphics, videos, animations etc. The screen can have links that can load further details in pop-up screens. These screens will present standard information on the assignment such as Assignment Description, Steps to Perform, Surgeon Cues, and Best practice to follow, video of expert performance, video from actual surgery etc.

### E. Learning Experience Logic

The Learning Experience Logic evaluates each unique Assignment, determines completion of Assignment based on proficiency requirements, determines the flow or order in which Assignments will be made accessible to users, etc. The trainee's experience on the simulator is determined by a number of features. One of the important ones amongst them is "individualization". One of the features relating to individualization is managing trainee's progress through a list of assignments. This can be computer controlled or controlled by a trainer.

A trainee is expected to progress through the Assignments in a definite order in the computer controlled mode. While it will be possible for the trainee to view the list of Assignments, only those that he or she is qualified to access as per this predefined order are displayed as active Assignments. "Active" Assignments mean those assignments that are enabled for practice. The "Inactive" Assignments will become "Active" when the pre-requisite Assignments are completed by the trainee. The trainer can give each trainee access to any assignment (in any order) or enable/disable access to each assignment based on his individual judgment. In addition, trainers will be allowed to change select conditions (specifications provided within the Assignment) referred to as modifiable conditions. Modifiable conditions refer to a selection of conditions for the surgical simulation that any trainer can change to enhance the training experience.

An Open Assignment is available to the trainer in trainer control mode. It will be configured with default simulation conditions. Default conditions are the most basic settings for simulator practice. The trainer is able to make changes using modifiable conditions (within these default conditions and some others) and surgical start point before allowing the trainee to begin the open assignment attempt. The purpose of the open assignment is to allow trainee familiarization with the simulator setting and the assignment conditions before initiating formative or summative evaluation of the attempts. Standard parameters and performance video are recorded for each attempt. However, attempts to this assignment will not be evaluated and scored by the system. The trainer can enter attempt feedback notes into the system.

A computer control mode is also provided where preset algorithms govern the access to assignments in the assignments library and progression across assignments. However, trainer will have the ability to override the setting for a trainee, for select assignments and to permit access. In this mode, the trainees can continue practicing assignments without requiring instructors to closely monitor and determine access. However, instructors will be required to review the performance data and provide feedback either on a real time basis or later for the attempts.

A Free Play feature within computer control mode allows for a predefined number of attempts corresponding to each assignment as Free Play. The purpose of Free Play mode is to allow trainee familiarization with the simulator setting and the assignment conditions before initiating formative or summative evaluation of the attempts. However, performance data will still be recorded for monitoring of simulator use.

Evaluation "of an Attempt": Every Assignment needs both formative and summative evaluation. For evaluation, several parameters are monitored and stored during and after the Assignment performance. Summative evaluation requires the use of select metrics and comparing them with set standards of performance to assign a score and/or classify as successful/failed attempt. A composite score is calculated from these metrics. This is required in order to benchmark performances and easy comparison with other attempts by the same user (or other users with the use of same standards). A simplified example to demonstrate this requirement:

| Performance Parameter | Standard | Actual | Range 1 - Score | Range 2 - Score | Critical Errors | Outcome | Actual Performance Score | Weight |
|---|---|---|---|---|---|---|---|---|
| Parameter A | 5.00 mm | 4.70 mm | 4.70-5.30 (40) | 4.40-4.69 or 5.31-5.50 (15) | No | ✔ | 40/40 | 40% |
| Parameter B | 5.00 mm | 4.62 mm | 4.70-5.30 (40) | 4.40-4.69 or 5.31-5.50 (15) | **Yes** | X | FAIL | 30% |
| Parameter C | 5.00 mm | 4.2 mm | 4.70-5.30 (40) | 4.40-4.69 or 5.31-5.50 (15) | No | X | 0/40 | 30% |
| Composite Score | 80 | 16 | | | | | 16 | FAIL |

An Assignment is considered completed when a desired level of proficiency is demonstrated with consistent performance recorded across a series of attempts. The completion requirement may also mandate certain amount of practice in the form of minimum number of attempts required, minimum number of successful attempts, and minimum number of consecutive successful attempts.

Free play is a pre-set number of attempts that will be allowed to each trainee for each assignment before the performance data from attempts is considered for scoring and progression. It allows them to familiarize themselves and get comfort with the task on hand before they get monitored and evaluated. Such requirement for each Assignment is set as part of progression and/or evaluation logic. A sample completion requirement for a typical assignment includes:
- Minimum Attempts Required - 80 (to ensure sufficient practice).
- Minimum Successful Attempts - 40 (to ensure certain minimum amount of success).
- Minimum Consecutive Successful Attempts - 37 (to ensure consistency in successful performance).
- Free-Play - 15 (to allow familiarization with assignment, without any pressure relating to performance review or evaluation). During Free-Play, no score is assigned and no feedback is provided. However, note that the performance data may still be recorded for monitoring purposes.

A simplified example to demonstrate application of this requirement is illustrated in the table below:

| Assignment No. 1 | | | |
|---|---|---|---|
| **Free-Play: 4 Attempts** | | | |
| **Min. Successful Attempts: > 3** | | | |
| **Min. Consecutive Successful Attempts: At least 3 out of last 5 (3/5) attempts must be successful** | | | |
| Attempt No. | Score | Outcome - (Successful Attempts) \| Consecutive Successful Attempts | Requirement Status |
| 1 | 16 | Fail (NA) | NA |
| 2 | 40 | Fail (NA) | NA |
| 3 | 55 | Fail (NA) | NA |
| 4 | 81 | Pass (NA) | NA |
| 5 | 64 | Fail (0/5) \| 0 | Continue Practice |
| 6 | 69 | Fail (0/5) \| 0 | Continue Practice |
| 7 | 87 | Pass (1/5) \| 1 | Continue Practice |
| 8 | 75 | Fail (1/5) \| 0 | Continue Practice |
| 9 | 96 | Pass (2/5) \| 1 | Continue Practice |
| 10 | 95 | Pass (3/5) \| 2 | Continue Practice |
| 11 | 97 | Pass (4/5) \| 3 | Completed |

### F. Performance Records Database

Performance Records Database contains a log of all learner attempts for each of the Assignments, storage of scores, metrics, videos, etc. Each attempt by a trainee will be recorded and stored. The data generated will be stored on the SIA/SBLS. It will include video recording, time taken for each attempt, performance parameter values, scores, count of attempts (successful, unsuccessful, aborted), count of last consecutive successful attempts, etc. These will be used for reporting, analysis and also used within the logic for progression across Assignments as shown above. The SIA transfers the records/data of Assignment attempt to the SBLS for each trainee immediately after each attempt. Additionally other data such as login time, logout time, time taken for attempts and session length will also be collected and stored for each trainee

### G. Data Import/Export

The system allows exporting performance data collected, including video recordings. Records of raw 3D model parameters are stored at predefined stages of the Assignment performance or on demand. Such raw 3D model snapshots can be exported from the SBLS for review and use by courseware design team. It will be utilized for setting up unique state of the eye model when creating assignments.

### H. Instructor Module

The Instructor Module allows the instructor to monitor and manage simulator practice sessions. The instructor can connect with the SBLS using a desktop/laptop PC and preferably a tablet. The instructor module will have the following functionalities for accessing the SBLS and managing the delivery of training.
- View a dashboard of simulator lab activity - simulators and current user activity.
- Monitor Assignment performance using video data stream, where the trainer can view a set of video streams together, or select one and toggle between views.
- Get real time and event based updates/messages.
- View reports.
- Add subjective comments to each Assignment attempted by a trainee.
- Add or reduce scores for an Assignment attempt based on subjective evaluation and observation.
- Review trainee performance history.
- Override learning experience logic to allow trainees to progress or prevent progression due to subjective evaluation or other such reasons.

Instructor can also login from the simulator Panel PC to access these features and also use it for performing Assignments, just like the trainees.

### I. Reports

Reports can be generated and viewed, including:
- Assignment Status report by the assignment for a group of trainees.
- Individual trainee progress report for each assignment and across all assignments.
- Training activity report (for a trainee or group - for a selected period of time/days).
- Learning analytics (charts and tables) - for assignments (individual, group and comparison) - some examples are given here:
   ∘ Trend - successful, failed, aborted attempts.
   ∘ Average time for successful (other status) or all attempts.
   ∘ Number of attempts to proficiency.
   ∘ Trend of performance - for selected metrics within an assignment (one or more metrics selected).
   ∘ Instructor comments for an assignment.
- Number of attempts and time spent on each attempt for each assignment.
- Number and sequence of failed attempts during an assignment (failed means assignments ends due to a vision threatening error).
- Number of failed attempts by sim level (2 or 3).
- Number and sequence of aborted attempts during an assignment.
- Number of aborted attempts by level.
- Number and sequence of successfully completed attempts during each assignment.
- List number and description of vision threatening errors for each assignment by date of attempt.
- Report of selected metrics by assignment and date (as per interface requirements metrics list above) after each 10 attempts.

The presentation of the report can be filtered, changed in views, and sorted and customized.

### J. Data/Code Update

The simulation software, which includes the core simulation and the simulator API code, can get updated periodically. This would be to fix issues and for upgrading the functionality. In addition, in order to enable use of simulators independent of SBLS, certain code and data will have to be maintained locally on the SIA. This includes data such as Assignment Library and code such as logic for progressing through assignments etc. In case there are updates, the data/code update module allows to push these to each of the simulators either periodically or on demand. A check may be run periodically to confirm latest version and update simulators as may be required.

### Typical Use Case Scenario

Following table provides a Use Case Scenario that, which connects various activities with the functions of the simulator and associated components and features, including the Simulator Based Learning System (SBLS):

| **Typical progression of training activity on the simulator** |
|---|
| Turn On the simulator: It turns on the Panel PC that runs the Simulator Interface Application (SIA) automatically on startup. |
| Login Screen displayed on adjunct screen. |
| Trainee logs in with ID and Password/Code. Instructor can also login using the same screen. |
| A list of assignments is displayed on adjunct screen. The list will also be present if the assignment is active. That is, enabled for beginning or continuing practice. The simulator can restrict access to assignments for managing the order in which they must be practiced. However, the instructor will always have unrestricted access to launch any Assignment, at any time. This data is loaded from the SBLS. |
| Trainee/Instructor select the assignment to begin practice. The instructor can disable the restriction on access and change all the assignments to be active. |
| The Assignment description screen will be displayed with a "Begin" button. The description content includes text supported by media elements such as images, videos and animations that can be viewed by the user. The content can be layered (one-level) - links made available to load pop up screens. |
| Trainee begins performance by touching the "Begin" button on screen. |
| A record of the Assignment attempt is created on the SBLS. On completion of the attempt, it will be updated with the corresponding data received from the SIA. |
| An instructor can login to the network application (SBLS) at any time to view a dashboard which lists all the simulators on the network, ongoing trainee activity, their status etc. |
| The SIA communicates to the Simulator via the Simulator API - Selected Assignment related files transferred to the simulator. The assignment files define starting point, ending point from the surgical procedure to be practiced, eye model configuration/settings, list of parameters to be tracked real-time and metrics to be provided at the end of performance. Alternatively, these files are received from SBLS on selection and passed on to the Simulation API. |
| The simulator uses the assignment files to load the eye model and the simulation activity can begin. The trainee picks up the instrument handles (haptic arms) and looks through the microscope to begin. |
| Trainee gives verbal commands asking for instruments - left and right hand. SIA has voice recognition capability for several verbal commands - these trigger sending commands (using XML files) to simulator for corresponding action. |
| Simulator gives audio alert in case incorrect instrument is selected. |
| Trainee brings the instruments in the field of view (microscope viewer) and begins the performance. |
| Real time parameters required by SIA are monitored and alerts provided in case alert conditions are met. All other metrics required are recorded during the assignment attempt. |
| Guidelines, Orientation view and Real time feedback are made available (as per the Assignment settings). Guidelines and Orientation view are artificial objects (lines, arrows etc.) displayed in microscope view for trainee performance support. Feedback is audio alert on trainee errors and short text message appearing with it for a few seconds to communicate the nature of error. These can be turned On/Off by a verbal command. |
| Any specific error or condition that is classified as "complication" may result in halting of the performance as per the assignment settings. The simulator will display a message communicating the event to the trainee. These conditions are preset in the simulator software. An alert to keep this condition active for monitoring is set or registered when Assignment files are transferred to the simulator. |
| The instructor client will also be able to view the alerts for which triggers have been configured as part of the Assignment specifications. |
| The instructor/trainee can switch between real-time video on the adjunct screen OR keep the Panel PC - SIA interface displayed. Essentially, the adjunct display can be used for connecting to one of the two display feeds: 1. Panel PC - SIA application interface or 2. HDMI video feed directly from the simulator visual display. |
| The HDMI video feed is also transferred to SIA via the Simulation API and it will be encoded and stored as part of the Assignment attempt data. |
| The instructor client will allow viewing the video stream from the simulator(s) via the SBLS - limited set of simulator videos simultaneously. |
| The trainee or instructor (standing by) can give a verbal command or use an on-screen button to abort or indicate completion of practice unit. |
| End of Assignment performance metrics are communicated to the SIA. |
| SIA receives the performance metrics and calculates scores corresponding to preset rubric for each Assignment. Or transfers the metrics to SBLS for calculations. SIA/SBLS computes a composite performance score. SIA/SBLS provides a success/fail outcome to the performance based on the evaluation rubric. |
| SIA/SBLS updates the Assignment status using a preconfigured proficiency requirement. (Not Started, In progress or Completed). Each assignment may have different completion requirement in terms of: performance scores across attempts, or trend of continuous successful performances (i.e., at least 10 successful attempts from last 15 attempts) |
| The assignment performance report is displayed on the screen for the trainee and instructor to review. Reports are represented visually. Some visual representations may be made available through links (pop-up). This will allow the instructor to use the performance data along with his/her observations for attempt evaluation and providing feedback to the trainee. |
| Closing the Assignment Attempt report will display a screen that provides summary of the user's performance history for the Assignment. The performance report for each attempt can be viewed by selecting any attempt from the list (a drill down report). |
| More options/features are made available to the users. They can go to "Reports" screen to select and view other available reports. |
| It will be possible to jump to the Assignments list to select another active Assignment to continue practice, or Log off and close the simulator session. |
| Logging off will update the SBLS with corresponding data viz. time. |
| Immediately after the Assignment attempt is completed and anytime thereafter, the instructor can review the attempt data and add (type in) a subjective evaluation comment and/or edit the score on the Assignment. Such comments and changes will be stored with the instructor identity. Two such notes/edits can be made - the second one allowing expert instructor to provide his/her comments and inputs. The trainee can also add comments or notes relating each attempt after completion. |

### VI. Sounds, Voice Commands, and Voice Recognition

Verbal commands are incorporated into the simulator to increase the realism of instrument changes and intraoperative management. That is, a verbal command asking for an instrument change can be given and the instrument visual under the microscope will change accordingly. In case handles are not the same, the trainee will need to manually change the handles that will be designed to feel like the actual instrument that is chosen for use.

Voice recognition is also provided as part of the courseware to support the selection of instruments by voice command and other commands by the trainee.

In some scenarios there are interactions with scrub nurse requiring the surgeon to communicate with other persons. The simulator restricted to what you see in the microscope and what you feel with your hands. Any other interactions or higher level communication will be handled by courseware and the simulator will provide an interface to the courseware to make any changes to the simulated scene based on whatever interaction is performed on a higher level. For example, the simulator may indicate that the microscope bulb has burned out. In this scenario the microscope bulb burns out and the microscope view dims. The response of the surgeon is to ask the nurse to change the microscope bulb, and when this is done the normal light is restored. The sequence between simulator and courseware is as follows: (1) courseware sends command to simulator that the bulb should burn out, (2) simulator responds by making the view appropriately dark, (3) courseware identifies whatever sequence of events that are required for the bulb to be changed, e.g. voice command to tell the nurse to change bulb, (4) courseware sends command to simulator that the bulb should work properly again, and (5) simulator restores the light in the microscope view.

Heart monitor sounds are also provided. Heart rate is connected to events in the simulator and also be settable from courseware side. In addition, patient sounds, requests, and other sounds are incorporated where the courseware can set sounds to be played at certain events or directly.

### VII. Simulation

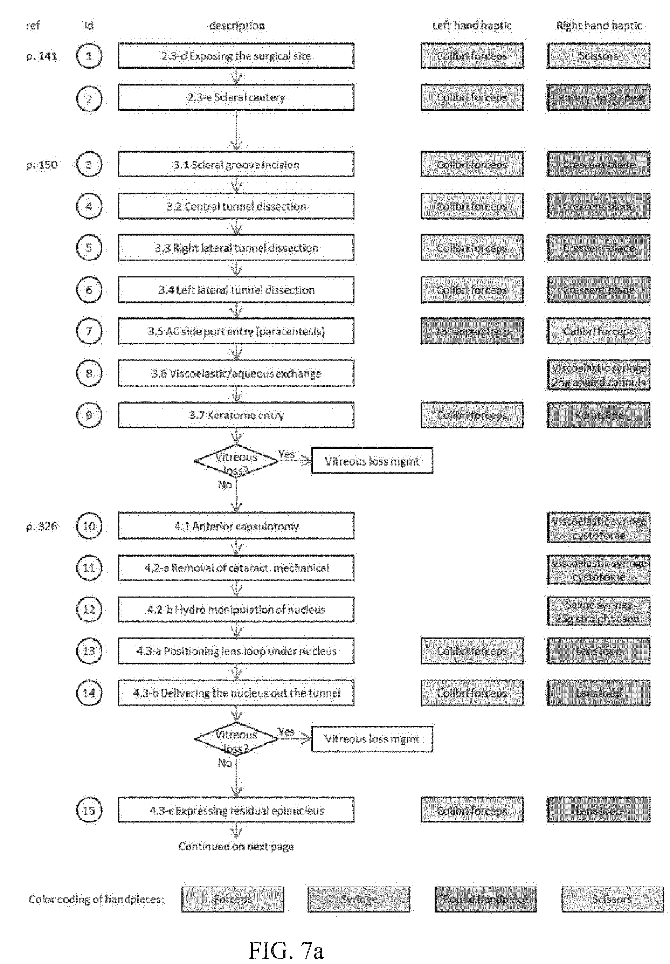

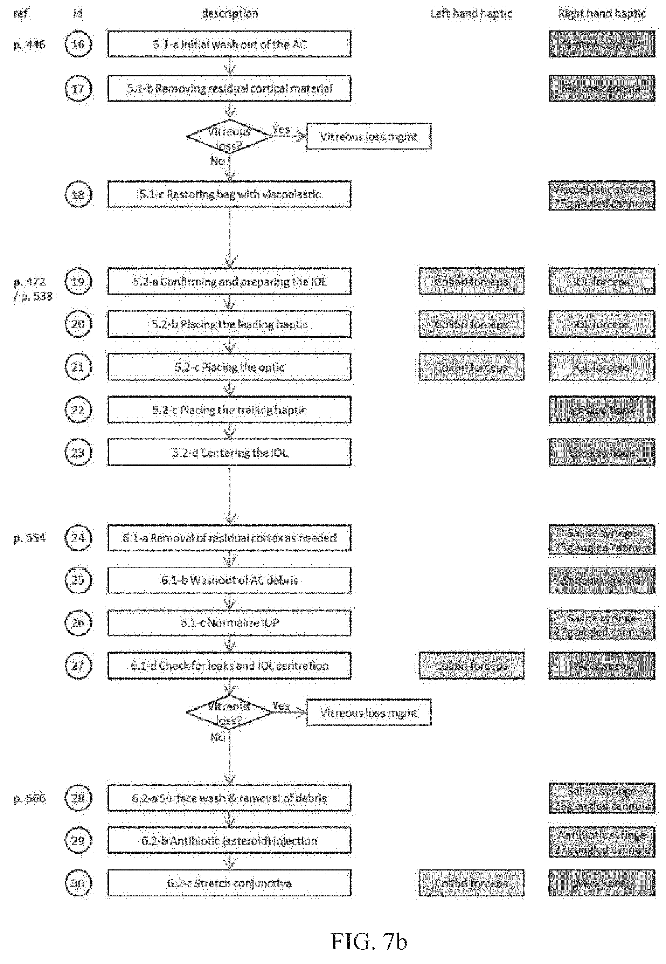

To achieve high fidelity realism, the sections below include the description of the steps of the MSIC surgery replicated by the simulator. The entire MSICS simulation consists of a plurality of surgical sub steps plus variations, complications, and specific remediating techniques. The steps and the corresponding left hand and right hand haptic tools are illustrated in FIGs. 7a-b. The simulated steps incorporate the forces listed in Section IV.

### A. General Overview

This section gives a general overview of the steps. The simulation starts assuming the patient and the equipment have been prepared for surgery and the patient has been successfully blocked and is comfortably lying on a surgical stretcher. The simulation begins after all preparations have been done up to, and including, inserting the lid speculum and the surgeon is positioned at the surgical microscope. The following steps are described with reference to FIGs. 7a-b.

In an embodiment, the following metrics are common to all the steps of the simulator: globe rotation from rest position, tool positions/orientations, contact info/forces with different anatomies of the eye, colibri positions on eye, intraocular pressure, timer for task and subtask, erratic or wasted movements (algorithm for calculating this given by courseware).

### Step 2.3d-e - Preparing the Surgical Field

The surgeon will expose the surgical site with conjunctival peritomy. This is done with scissors by cutting the conjunctiva to expose the sclera. The exposed sclera is then cauterized to minimize bleeding.

### Haptics:

- Friction from scissors when closing. There is no difference in cutting force when closing scissors without cutting tissue compared to when tissue is cut.
- Appropriate resistance when interacting with the eye depending on what part of conjunctiva is grabbed by forceps.

### Visuals

- Week spear interaction. Fluid shifts to the Week spear (clear or red depending on if it is blood or fluid)
- Blood. Pooling, not spurting.

### Simulation

- Check IOP by touching cornea.
- Cutting of membrane tissue using scissors.
- Scissor interaction with conjunctiva, sclera and Tenons. Initial snip to create opening and then one scissor jaw inserted between sclera and conjunctiva. Possibly inserting both jaws between sclera and conjunctiva and open jaws to create a tissue plane for cutting.
- Forceps interaction with conjunctiva. Grabbing conjunctiva and manipulating eye globe with it.
- Bleeding is simulated and pools in the eye. Blood does not spurt.
- Week spear interaction. Dries up surface on contact. Fluid shifts to the Week spear.
- Cautery of exposed sclera.

### Hardware

- Left hand colibri forceps, right hand scissors for the peritomy.
- Left hand colibri forceps, right hand cautery tip for cautery or weck spear for drying.

### Metrics

- Cut positions in conjunctiva including limbal start and stop positions.
- Angle between scissor jaws and sclera.
- Exposed sclera shape/size.
- Percent of exposed sclera that has been cauterized.
- All bleeding has stopped

### Step 3.1 - Scleral Groove Incision

Create a first groove (at 75 degrees angle to the scleral surface) to establish the proper orientation for the tunnel in relationship to the cornea and the correct length for the external tunnel opening. The depth of the groove is also important.

### Haptics

- Proper cutting forces. Slight resistance when cutting. If misaligned resistance increases.
- Hard surface with minimal deformation if left hand is pushing to get sufficient IOP, otherwise spongy and easily deformed.

### Visuals

- Color in groove cut base depends on depth - white to bluish to black. Black is too deep, bluish good, white too shallow.
- Stereopsis important to determine cut depth.
- Slightly lifted upper edge of groove is important cue.

### Simulation

- Bi-manual manipulation of eye. Cut could be done by holding the eye still with the forceps and cutting by moving the crescent blade (normal case) but could also be done by holding the blade still and moving the eye with the forceps. Forceps interaction with conjunctiva. Grabbing conjunctiva and manipulating eye globe with it.
- Pressing Colibri at limbal insertion stabilizes eye. Grabbing conjunctiva away from the insertion will not stabilize eye (it is too loose).
- Bleeding as in previous steps.
- IOP based on pressure from tools.
- Cutting a groove in sclera. Cutting is more difficult if IOP is low.
- Visualization is more difficult if the surface is wet (it must be dried before starting groove)

### Hardware

- Left hand colibri forceps, right hand crescent blade.

### Metrics

- Groove shape (length, depth, position).
- Groove shape in relation to limbus.
- Sclera/crescent blade angle.
- Force applied during groove dissection
- Count of redundant movements (multiple passes)

### Step 3.2 - Central Tunnel Dissection

Establish the correct tissue dissection plane and the proper limit of penetration of the tunnel into the cornea. This tunnel is created within the sclera into the cornea going through three distinct tissue types: sclera, limbus and cornea.

### Haptics

- Scleral tissue has higher resistance to cutting than the limbus or cornea.
- If tool is misdirected in the wrong plane or misaligned resistance to cutting increases.
- Resistance decreases by 60 % when entering cornea if the blade is oriented properly.

### Visuals

- Proper visibility of the tool depending on depth of blade and the tissue type it is going through.
- Sclera is more opaque than limbus. Cornea is transparent.
- Slight clouding of cornea tunnel parts, either in white or red due to some minor blood leaking in from the outside.

### Simulation

- Tunneling by swiveling the heel of the crescent blade back and forth. Cutting resistance as in Haptics section.
- Forceps interaction with the globe or groove and conjunctiva. Manipulating eye globe with forceps from various positions.
- IOP based on pressure from tools.
- Proper deformations of cornea and sclera depending on pressure of tool on different parts while cutting.

### Hardware

- Left hand colibri forceps, right hand crescent blade.

### Metrics

- Tunnel shape, position and length/depth.
- Sclera/crescent blade angle including perpendicular to tangent of the globe curvature in two directions (towards the corneal apex and around the limbus).
- Force applied during dissection
- Count number of times blade slips out of groove.
- Count deviations from correct orientation (correct orientation being given by courseware).

### Step 3.3-3.4 - Lateral Tunnel Dissection (Left/Right)

Extension of the central tunnel to the sides.

### Haptics

- Cutting through sclera, limbus and cornea simultaneously. More resistance at the end points where the ratio of sclera is higher.
- If tool is misdirected in the wrong plane or wrong motion is used (chop instead of slice) resistance to cutting increases.

### Visuals

- Proper visibility of the tool depending on depth of blade and the tissue type it is going through.

### Simulation

- Forceps interaction with groove. Cut edge of groove is grasped with forceps.
- IOP based on pressure from tools.
- Proper deformations of cornea and sclera depending on pressure of tool on different parts while cutting.
- Cutting:
   ∘ Forces not directed at the cutting edge will move the eye or deform the sclera instead of cutting.
   ∘ Slicing motion will cut the tissues. If no slicing motion a significant amount of force is required to cut.

### Hardware

- Left hand colibri forceps, right hand crescent blade.

### Metrics

- Tunnel shape, position and length/depth.
- Sclera/crescent blade angle including perpendicular to tangent of the globe curvature in two directions (towards the corneal apex and around the limbus)..
- Force applied during slicing.
- Count premature entry and button hole.
- Count deviations from correct orientation (correct orientation being given by courseware).

### Step 3.5 - Anterior Chamber Side Port Entry (Paracentesis)

Creation of a side port for secondary access to the anterior chamber. This is usually used for viscoelastic/aqueous exchange and is done by a self-sealing stab incision through the cornea.

### Haptics

- High initial resistance to entry that drops significantly after tip entering the outer third of the cornea.

### Visuals

- Incision leaves a faint mark through the cornea.

### Simulation

- Stab entry:
   ∘ Very much bi-manual task - forceps grabbing on opposite side of entry.
   ∘ If the angle is wrong the resistance does not drop after the outer third of the cornea has been entered.
   ∘ Initial dimpling of cornea that disappears on entry.

### Hardware

- Left hand 15 supersharp, right hand colibri forceps.

### Metrics

- Tool/cornea angle.
- Entry point.
- Entry angle.
- Tool insertion depth.
- Force applied during stab entry.
- Count iris contact, anterior capsule contact with stab blade.

### Step 3.6 - Viscoelastic/aaueous exchange

Stabilization of anterior chamber by filling it with viscoelastic without raising the intraocular pressure.

### Haptics

- No resistance to cannula sweep or withdrawal (will be resistance if misaligned).
- Injecting through a syringe

### Visuals

- Leading edge of bolus of viscoelastic as it is injected.
- Possible air bubbles.

### Simulation

- Lens-iris diaphragm.
   ∘ Moving together up/down depending on pressure.
- Exchange of viscoelastic/aqueous. Viscoelastic flows from cannula, aqueous flows out of entry point.
- IOP simulation based on amount of fluid in AC.
- Entry into AC
   ∘ Tool must be aligned properly to enter. Cannula will bend and move eyeball if not correct.
- Bending of cannula.

### Hardware

- Left hand stabilizes cannula, right hand holds syringe with cannula attached. Metrics
- Amount of viscoelastic injected.
- IOP.
- Amount of aqueous left.
- Position if lens/iris diaphragm (too deep, too shallow).

### Step 3.7 - Keratome Anterior Chamber Entry

Fully open the tunnel into the anterior chamber without touching any other intraocular structures.

### Haptics

- No resistance as tip is passed to the inner limit of the tunnel.
- At entry, significant resistance from lifting the tool handle which continues unchanged as blade enters AC.
- No resistance felt when realigning tool for slicing after stab entry.
- 50 % lower resistance to slicing motion than stab entry.
- 15-20% increase in resistance at the lateral limits of the tunnel.

### Visuals

- Distinct dimple in light reflex as cornea bulges inward as pressure is put on tip of keratome.
- Keratome is slightly dull looking when in cornea but bright and shiny when in AC.

### Simulation

- Stab entry for initial entry.
- Cutting left/right to fully open tunnel.

### Hardware

- Left hand colibri forceps, right hand keratome.

### Metrics

- Tool/tunnel angles
- Entry angle and entry point
- Force to enter
- Count number of snags on the inner tunnel wall and premature entries
- Count deviations from following corneal vault.

### Complications (Section 3)

### Full thickness perforation of scleral wall

Simulation will stop if the scleral wall has been fully perforated and the student has to start over and try again. Below is an explanation what the surgeon would need to do. Rare, but if it happens it usually happens during the scleral groove creation. Uveal prolapse possible, higher probability the larger the perforation.
- If no prolapse, continue as normal and avoid the area.
- If prolapse,
   ∘ Suture with help of assistant(extra weck spear).
   ∘ Continue by making new grove.

### Perforations and lacerations of inner or outer tunnel walls

Misdirection of crescent blade or keratome can easily lead to damage of inner or outer wall. Perforation refers to penetration of the wall resulting in a hole, laceration to cutting an edge. Laceration only happens for the novice surgeon and the simulator does not need to address the recovery of laceration. If that happens the student will have to start over.
- Likely to happen if withdrawing blade with pressure still at cutting edge.
- Outer wall (button holes):
   ∘ If defect needs repair, suturing is required before proceeding.
- Inner wall (premature entry):
   ∘ Experienced as a sudden decrease in resistance and a small gush of aqueous out of the tunnel.
   ∘ Addressed by creating a secondary tunnel slightly shallower than the primary tunnel.

### Oversized tunnel

If tunnel or stab paracentesis is too large, it will leak after procedure. Leaking stab paracentesis is addressed with injection of saline solution. Leaking tunnel needs to be sutured.

### Undersized tunnel

Tunnel is too small to get the nucleus out. This is addressed by enlargement of the tunnel after attempt to deliver nucleus.

### Failure to control the anterior chamber

- Low IOP, shallow AC
   ∘ Cornea folds/dimples
   ∘ Addressed by injecting viscoelastic.
- Low IOP, deep AC
   ∘ Usually damaged zonules that cannot hold the viscoelastic up. Allows for chamber to be excessively filled without pressure building.
   ∘ Addressed by changing the angle of injections and allow slight shallowing of AC prior to injection of viscoelastic.
- High IOP, shallow AC
   ∘ Check speculum or drapes by lifting or removing speculum (verbal command checked in courseware).
   ∘ If globe is bulging forward
      ▪ Cut at lateral canthus made with scissors and abort procedure(verbal command checked in courseware).
   ∘ If globe not bulging forward
      ▪ Attempt to deepen AC with viscoelastic
      ▪ If unsuccessful, abort procedure
   ∘ If eye is rock hard
      ▪ Do vitreous tap by inserting needle into eye 2.5 mm behind limbus, 10 to 12 mm deep, and gentle aspiration should drop the pressure.
      ▪ If IOP normal the procedure can continue, otherwise it is aborted.
- High IOP, deep AC
   ∘ Addressed by pressing lower edge of paracentesis or tunnel to allow aqueous and viscoelastic to flow out.
- Descemet's detachment
   ∘ Small, nothing needed
   ∘ Medium, visoelastic/air exchange
   ∘ Large, leave a large bubble of air.

### Step 4.1 - Anterior Capsulotomy

Create an opening in capsular bag in order to access the cataract. Can be done with one of two variants, multi-cut or interrupted tear capsulotomy. Cystotome tool is used to enter the tunnel and perform the capsulotomy. Pressure in AC needs to be maintained during the step using injection of viscoelastic into AC.

### Haptics

- No perceivable force from cataract during proper capsular tearing or cutting.
- Contact with cataract will be felt and hardness will vary depending on cataract type and variations.
- Appropriate resistance will be felt when moving cystotome in or out of tunnel.
- Cystotome will snag in tunnel if not entered properly with the right angle and orientation.
- Tunnel texture including scleral ridges (from crescent dissection) and fibrous feel

### Visuals

- Red reflex as appropriate for cataract type.
- Cataract color as appropriate for cataract type.
- Bulging of cornea above cutting edge if endothelial touch(inside of cornea).
- Bulging of tunnel if cannula lifted.
- Possible bubbles from viscoelastic.
- Feathering/irregularities where cystotome has touched cataract.
- Cortical tracks from passing the cystotome.
- Anterior capsule mobile and able to interact with it separately once capsulotomy complete.

### Simulation

- Appropriate tunnel deformations when moving tool inside tunnel.
- Cutting/tearing of anterior capsule to open properly sized circular hole(topology change in triangle mesh depending on tearing forces).
- Mobile anterior capsule.
- Viscoelastic management to keep appropriate pressure in anterior chamber. Viscoelastic can be inserted through tool and will leak out especially if pressing on walls of the tunnel.
- Movement/damage of iris if touched.
- Pupillary movement from cystotome pressure.
- Endothelial touch.

### Hardware

- Step performed using two-hand grip on tool with left index finger stabilizing cystotome and right hand holding the syringe with cystotome attached.

### Metrics

- Amount viscoelastic used.
- Amount viscoelastic leaked.
- Forces in tunnel.
- Cannula-tunnel plane angle.
- Lift distances and force in the tunnel.
- Average radius of opening in anterior capsule.
- Cut position and tool movement for each cutting motion in multi-cut capsulotomy.
- Count number of attempted cuts
- Count number of times cannula depth exceeds safe limit below anterior capsule
- Count endothelial touch

### Step 4.2 Removal of cataract from capsular has

Removal of cataract through the opening. Cataract is larger than opening. Cataract nucleus is removed without damaging the surrounding tissues. The surgeon must loosen the attachments of the nucleus to cortical material and pass the nucleus up through the capsular opening and into the tunnel while maintaining control of the anterior chamber. Step ends with equator of nucleus in tunnel.

### Haptics

- As in step 10.
- Typically forces are between 10-20 grams during nucleus manipulation.
- 15-20 grams shear forces when cystotome tip is embedded in cataract to manipulate it and the forces decrease once the nucleus is free and rotates.
- Up to 20 grams or initial cleavage plane creation between nucleus and cortical layers.
- Minimal resistance to downward movement.
- Resistance to rotation highest at beginning of rotation and decreases when rotated.
- Minimal rotation resistance once equator is over the edge of the capsular bag.

### Visuals

- As in step 10.
- Stirring up of cortex.
- Possible peripheral red reflex when excessive lateral force is used.
- Full size of cataract seen in AC (mostly hidden behind iris prior to this step)

### Simulation

- Appropriate tunnel deformations when moving tool inside tunnel.
- Viscoelastic management to keep appropriate pressure in anterior chamber. Viscoelastic can be inserted through tool and will leak out especially if pressing on inner wall of the tunnel.
- Movement of nucleus in capsular bag including restrictions of attachments to it that will be released in the process.
- No anatomically distinct line between cortex and nucleus, so important to simulate their behavior differently. Nucleus moves as a mass, cortex produces tufts of tissue that move with the fluids in AC.
- Hydromanipulation with cannula to get nucleus fully exposed in AC.
- Cataract acts as a guide for cannula.

### Hardware

- During first part same as in 4.1, two-hand grip on cystotome and syringe.
- Two-hand interaction with cannula and syringe for injection of BSS (balanced saline solution)

### Metrics

- Amount of viscoelastic used.
- Amount of viscoelastic leaked.
- Forces in tunnel.
- Rotational force applied to nucleus and radial force applied to zonules
- Cystotome and Cannula-tunnel plane angle.
- Lift distances of tunnel.
- Count number of times the cystotome tip passes the mid point of lens thickness
- Count number of times the cannula loses contact with the cataract once it has contacted the cataract (cataract acts as guide for tip of cannula)
- Count number of times the nucleus equator crosses them midline

### Step 4.3 - Removal of cataract from anterior chamber

Removal of nucleus through tunnel using lens loop technique with the nucleus in one piece. Position lens loop under cataract using 4 distinct lens loop positions and then withdraw the lens loop through the tunnel bringing the nucleus out.

### Haptics

- 10-15 grams of force while moving lens loop into place under nucleus.
- Up to 80 grams of force downward on inner tunnel wall when moving nucleus through tunnel.

### Visuals

- Lens loop barely visible through nucleus.
- Nucleus appearance changes as passes through tunnel

### Simulation

- Movement of nucleus while moving lens loop into place.
- Proper interaction between nucleus, lens loop and tunnel.
- Removal of epinucleus.

### Hardware

- Left hand colibri forceps (no grasping until lens loop in position under nucleus), right hand lens loop, when withdrawing lens loop.
- Fingers steadied on patients forehead.

### Metrics

- Forces in tunnel.
- Lens loop-tunnel plane angle.
- Movement/orientation of lens loop.
- Angle of withdrawal.
- Count contact of nucleus with corneal endothelium
- Count iris entrapment between lens loop and nucleus

**Cataract Variations -** Below are the example cataract cases that are included in the simulator:
- Advanced nuclear sclerotic (HMS standard case) (70%)
   ∘ Golden brown to dark brown
   ∘ Red reflex dim to absent
   ∘ Nucleus size: 7 - 8 mm
   ∘ Cushion of dense cortical material between nucleus and capsular bag.
- Mature cortical cataract (20%)
   ∘ Complete opacification of the cortex surrounding a dense nucleus.
   ∘ White color
   ∘ No red reflex
   ∘ Hard nucleus that moves more than normal.
   ∘ No dense supporting cortical material.
   ∘ Could be difficult to pierce capsular bag (reduced back force from cataract).
- Hypermature cortical cataract (5%)
   ∘ Liquefication of cortex
   ∘ Nucleus can be partially or completely resorbed.
   ∘ No red reflex
   ∘ Milky white appearance
   ∘ Rapid collapse of capsular bag since cortex is liquid.
   ∘ Gentle pressure on anterior capsule feels spongy and surface may wrinkle like a soft grape.
   ∘ Easy to remove since it can essentially be washed out.
   ∘ Anterior capsulotomy can be harder because of rapid collapse of capsular bag on first cut.
- Hypermature black cataract (2%)
   ∘ Cortex is compressed to one large rock hard nucleus
   ∘ Dark black appearance
   ∘ Larger cataract
   ∘ Rock hard
   ∘ Requires wider tunnel than normal.
   ∘ Fragile anterior capsule

### Complications (Section 4)

### Enlarging anterior capsulotomy

If capsulotomy was too small it needs to be enlarged. This is done by:
- Westcot scissors used to make a relaxing cut on cut edge of capsule.
- Cut edge is grasped with forceps and torn off after IOL insertion.

This can just as well be done by cystotome.

### Posterior capsule rupture

If the posterior capsule is penetrated vitreous loss might occur and in extreme cases the lens can fall down into the posterior segment.
- If lens falls into the posterior segment, vitreous loss is managed.

### Wooden pupil

Pupil is stiff from scarring. Handled by micro scissors making three 2 mm cuts at 3, 6, and 9 o'clock positions in iris.

### Secondary technique for nucleus removal

If nucleus will not rotate after two attempts of the standard technique a secondary technique can be used (listed in order of increasing patient risk):
- Blunt cannula hydromanipulation. Sweep around nucleus, inject fluid and lift the nucleus.
- Press cannula downwards on capsular edge until the equator of nucleus is seen, rotate and lift.

### Rescue of falling nucleus

- Alert scrub nurse **(verbal command checked in courseware)**
- Check lid speculum not applying any pressure to eye **(verbal command checked in**

### courseware)

- Iris sweep tool (or Sinsky hook) entered through paracentesis
- Viscoelastic cannula through tunnel
- Put a cushion of viscoelastic under nucleus.
- Then use lens loop and remove as usual
- Collapse of eye with corneal dome turning inwards possible with the valve effect of the tunnel lost. Usually when removing black cataract.
   ∘ Correct by grasping posterior lip of scleral groove with Colibri forceps in left hand while viscoelastic is injected into the AC in right hand.

### Intracapsular cataract extraction

- Lens hinged in one quadrant (listed as a contraindication for surgery).
- Blunt cannula with viscoelastic used on hinge side to move other side up.
- Cannula swept around lens to get the loose part of the lens up.
- After this lens loop can be used as normal.

### Considerations regarding nucleus removal in standard case

- If iris is trapped between lens loop and nucleus when lens loop is inserted and then lifted iris prolapse can occur (this can occur as a metric since there is no treatment to provide-the experienced surgeon will not cause this).
- If lens loop pushes and wedges nucleus between cornea and iris endothelial damage will occur (this needs to be a metric but there is no visual damage until postoperatively).
- Inaccurate orientation of lens loop or inadequate tunnel size while delivering nucleus might result in splitting the nucleus in two or more pieces.

### Step 5.1 - Preparing for IOL insertion

After removal of nucleus, the intraocular pressure is reduced from 5 mm Hg to zero. There are residual nucleus and epinucleus material in AC what needs to be washed out and the AC reformed and maintained.

### Haptics

- Feel the syringe plunger in the left hand plugged vs not plugged.
- Tunnel forces as before.
- Tunnel textures as before

### Visuals

- Cortex material, feathery white. Like feathery plant in water.
- Capsular bag, cellophane-like.
- Light ring reflection off posterior capsule on Simcoe contact.
- Red reflex as cortex is removed becomes brighter where the cortex is removed.

### Simulation

- Appropriate tunnel deformations when moving tool inside tunnel.
- Irrigation fluid washing out debris.
- Pulling cortical material out using aspiration to hold it to Simcoe tip.
- Cortical movement, stickiness, and stretch.
   ∘ Aspiration of material into cannula.
- Fluid management to keep appropriate pressure in anterior chamber. Fluid is inserted I&A port and pressure can also be adjusted by height of irrigation bottle (height of irrigation bottle can be set by courseware). Fluid will leak out when pressing on inner wall of the tunnel or the posterior lip of scleral groove.
- Pupil dilation when inserting viscoelastic to restore capsular bag.

### Hardware

- Left hand I&A (irrigation/aspiration) syringe, right hand Simcoe cannula.
- Left hand stablizes cannula, right hand plunger when restoring capsular bag.

### Metrics

- Amount of viscoelastic used.
- Amount of viscoelastic leaked.
- Forces in tunnel.
- Simcoe and Cannula-tunnel plane angle.
- Lift distances of tunnel.
- Irrigation fluid used.
- Particles removed/remaining.
- Count number of times the posterior capsule is aspirated

### Step 5.2A - Implanting a posterior chamber IOL (intra-ocular lens)

The implantation of a new IOL into the posterior chamber. The surgeon chooses an appropriate IOL and grasps it with the IOL forceps from its container. The IOL is then inserted through the tunnel into the capsular bag. In the simulation the lens will already be grasped correctly when forceps is introduced to the simulation. The part of grasping it from its' container will not be included.

### Haptics

- Tunnel forces as before.
- Once inside the eye, minimal torsional forces.
- Minimal forces while inside the eye when performed correctly, mostly guided by visual cues.
- 5-10 grams when tapping on IOL (can feel the tapping of metal tip on rigid plastic)

### Visuals

- Cut edge of anterior capsular bag visible when filled with viscoelastic.
- IOL light reflex (larger and dimmer than corneal light reflex)

### Simulation

- Appropriate tunnel deformations when moving tool and IOL inside tunnel.
   ∘ Manipulation of IOL with forceps and Sinksey hook.
- IOL is rigid so will follow tool when grasped firmly.
- Simulation of leading and trailing haptic and their interaction with the different parts of the eye, e.g., IOL can be only rotated in one direction, tire tool effect of the cut capsular edge.

### Hardware

- Left hand colibri forceps, right hand IOL forceps when inserting IOL.
- Two-handed Sinskey hook when handling haptic and final placement of IOL.

### Metrics

- Amount of viscoelastic used.
- Amount of viscoelastic leaked.
- Forces in tunnel.
- IOL-tunnel plane angle.
- Lift distances of tunnel.
- Movement of IOL.
- Movement of haptic.
- Count contact with corneal endothelium
- Report if IOL haptic are in the capsular bag or out of capsular bag
- Count iris contacts

### Step 5.2B - Implanting an anterior chamber IOL

The implantation of a new IOL into the anterior chamber instead of the posterior chamber. This is required in 5 % of cases when there is no capsular bag, a broken posterior capsule or insufficient support from zonules. It requires a different lens type with different haptics that is inserted in the AC angle instead of the capsular bag.

### Haptics

- Similar to 5.2A
- Resistance to moving the optic is significantly more than rotating the PC IOL(est. 10-15 grams)

### Visuals

- Similar to 5.2A.

### Simulation

- Similar to 5.2A with the difference that the haptics are different shapes, the positioning of the haptics is in AC angle instead of capsular bag and the rotation into place is a bit different.
- Iris interaction with IOL

### Hardware

- Left hand colibri forceps, right hand IOL forceps when inserting IOL.
- Two-handed Sinskey hook when handling haptic and final placement of IOL. Metrics
- Amount of viscoelastic used.
- Amount of viscoelastic leaked.
- Forces in tunnel.
- IOL-tunnel plane angle.
- Lift distances of tunnel.
- Movement of IOL in AC space.
- Movement of haptic into the AC angle.
- Count contacts with endothelium.
- Report iris trapped by IOL

### Complications (Section 5)

### Zonular weakness

### Signs for zonular weakness

- Tapping gently on scleral wall while observing for phacodonesis at beginning of case (vibration of lens).
- Linear folds of posterior capsule and/or cortex.
- Peripheral bright red reflex.
- Rolled up edge of capsular bag.

Zonules have limited elastic recoil properties and excessive tension results in stretching and breaking and no return to normal length. If zonules have insufficient tension to stretch the posterior capsule, the removal of the bulk of material results in laxity of the capsular bag. This can result in:
- Posterior capsule may suddenly shift upwards during I&A.
- More difficult to peel cortex off capsular bag.
- Capsular bag may roll up like a scroll.

Need to be able to distinguish between cortex and vitreous.
- Cortex keeps its shape and moves more like a solid while vitreous move more like a gel.
- Cortex tends to collapse capsular bag inwards in a wedge shape, vitreous more broad deformation.
- Vitreous blocks the aspiration of cortical material and causes a sudden increase in resistance to aspiration from blocked aspiration (same as cortex plugging the aspiration portand a peripheral movement of the capsular bag from where the vitreous strand enters the posterior segment.

### Considerations

- < 3 clock hours of zonular damage - IOL can be rotated in usual fashion.
- 3-6 clock hours of zonular damage - IOL inserted either into the ciliary sulcus or the capsular bag depending on location and amount of zonular damage. If > 4 clock hours sulcus fixation is mandatory.
- >9 clock hours of zonular damage - alternative technique must be used. IOL haptic placed directly into capsular bag or sulcus without rotation the IOL more than 5 to 10 degrees. Viscoelastic is used to open the rolled up capsular bag and leading haptic is then directed into a portion of the capsular bag where zonular support is present. The trailing haptic is then placed into the capsular bag without rotation using Sinskey hook or long angled forceps to compress the trailing haptic over the optic, push the haptic and optic down towards posterior capsule and release the haptic into capsular bag or sulcus.
- If IOL is not centered and cannot be centered the IOL must be removed with forceps and replaced with an AC IOL.

### Posterior capsule rupture

Errors during removal of residual cortex with Simcoe I&A are the most common cause of tears or holes in the posterior capsule. Holes in PC are rounded. Can be caused by:
- Unnoticed aspiration of the posterior capsule followed by movement can tear a hole.
   ∘ Identified by seeing striae (fine lines) from the PC to I&A port.
- Direct puncture with Simcoe cannula.
- Extension of an anterior capsular tear during I&A. An anterior capsule edge tag is aspirated and tension applied it can tear it and in some cases extend all the way to posterior capsule.
   ∘ Identified by movement of the capsular bag with the I&A port and direct visualization of the anterior capsular tag entering I&A port.

### Rupture identified by:

- A slowly expanding rounded line at the plane of the PC.
- If cortical remnants are still attached to the PC, these remnants may highlight the edge of the capsular hole.
- Any free iris pigment sticks to vitreous

### Iris damage

- If iris is aspirated into the I&A port and dragged towards the center point, iris shredding may occur. In most cases the iris will shred before it pulls loose from the AC angle.
- Any contact with iris is likely to result in localized loss of iris pigment free floating in AC.
- Bleeding may occur from torn iris blood vessels (slow leak not pulsing).

### Anterior chamber blood

- Normal iris vessel when torn produces bright red blood that fills the AC within 1 to 2 seconds.
- Normal response is irrigation and AC washout with Simcoe cannula as in step 5.1 for cortical washout.

### AC loss

If AC collapses during I&A and cannot be maintained at least 2.5 mm centrally Simcoe should be repositioned through the paracentesis
- Enlarge parencentesis to 2.0 mm with stab blade (1 mm originally)
- Harder to remove residual cortex because it is controlled by left hand and the tighter fit of the cornea reduces the ease of movement.

### Vitreous loss

There are a number of different scenarios which can lead to vitreous loss. Vitreous loss is when the vitreous gel is no longer contained within the posterior segment of the eye. Vitreous behaves as egg white and is anchored in the vitreous body. Hence movement of vitreous can damage retina or posterior capsule.

### Week vitrectomy

### If vitreous loss is suspected

- Week sponge is used in left hand to touch outer tunnel opening and lifted just enough to check for a clear strand of vitreous coming out of the tunnel.
- Pupil moving debris looking "trapped in jello" are signs of vitreous loss.
- Wescott scissors in right hand cut the strand flush with the tunnel opening while holding slight tension on the strand with the weck sponge in the left hand.
- Repeat from 1-3 until no vitreous at tunnel opening. Only dry parts of Weck sponge can be used since vitreous will not stick to wet parts.

### Scissors vitrectomy

- Low flow I&A
- Viscoelastic is used to stabilize the vitreous and move it close to tunnel by beginning the injection in the 6 o'clock AC angle.
- Wescott scissors are used to cut the vitreous at the level of the iris.
   ∘ Entered full opened scissor blades, positioned just past edge of pupil opposite tunnel closed and then withdrawn without being opened.
- Vitreous will stick to scissor blades as it is withdrawn.
- Sometimes two more cuts are needed.
- Clean tunnel with Week vitrectomy.
- Sweep vitreous off iris with viscoelastic.

### Step 6.1 - Establishing Optimal Postop Conditions for the Anterior Chamber

Establish optimal conditions for healing to begin and avoid postoperative complications. Possible residual cortex is removed with aspiration, AC is washed clear of debris, IOP is normalized and wound checked for leaks.

### Haptics

- Tunnel forces as before.

### Visuals

- Variable red reflex that indicates posterior segment pathology (old vitreous hemorrhage for example).
- Various debris (as in previous sections)
- Reflex off IOL surface

### Simulation

- Appropriate tunnel deformations when moving tool inside tunnel.
- Aspiration of residual cortex (similar to 5.1 except with 25 gauge cannula instead of simcoe)
- Washing out debris and viscoelastic (similar to 5.1)
- Leak detection (sponge swelling) and globe tension

### Hardware

- Right hand syringe and plunger stabilized by left hand finger when removing residual cortex and normalizing IOP.
- Left hand syringe, right hand Simcoe cannula when removing debris and washing out viscoelastic.
- Right hand colibri forceps, left hand Week spear when checking for leaks.

### Metrics

- Amount of viscoelastic used.
- Amount of viscoelastic leaked.
- Forces in tunnel.
- Cannula-tunnel plane angle.
- Lift distances of tunnel.
- Amount saline used.
- Particles removed/remaining.
- Cortex material removed/remaining.
- Leaking tunnel
- Position of IOL

### Step 6.2 Restoring the Surgical Field to Normal Conditions

Surface of eye is washed, antibiotic injected and conjunctiva is stretched over the tunnel to cover the sclera. There are more steps following this but they are not covered by the simulator.

### Haptics

- Tunnel forces as before.

### Visuals

- Same as 6.1

### Simulation

- Washing and removal of debris on outer surface of eye.
- Injection of antibiotics, cefuroxime into AC directly through the paracentesis
- Stretching of conjunctiva over scleral wound site.

### Hardware

- Left hand colibri forceps, right hand syringe during surface wash.
- Syringe 5 when injecting antibiotic.

### Metrics

- Cannula-paracentesis plane angle.
- Coverage of scleral field

### Variations (included in the simulator)

This section outlines the different anatomical variations that the simulator supports in terms of color, shape, cataracts and other pre-decided variations.

### Visual variations

- Skin color - the hues of skin color will be included, including Caucasian, Black, and Asian.

### Conjunctiva

- Two variants of blood vessel "layouts"
   ∘ Many blood vessels
   ∘ Not so many blood vessels
- Two hues of white in the conjunctiva for each such variant
- One variant of pinguecula (yellow white deposit on the conjunctiva near the limbus)
- One variant of pterygium (skin-like growth extending from conjunctiva into cornea) extending 2 mm onto cornea (only affects visuals, no change to simulation)

**Sclera -** Two variations with perforating vessels in random locations.
- Stark white
- Yellowish white

**Limbus** -Four variations with differences in color, texture, arcus and brown pigmentation. (Arcus is a hazy white opacity of the peripheral cornea. It looks very scarring but does only affect visibility and not resistance to cutting)
- Normal
- Brown pigmentation
- Normal with arcus
- Brown pigmentation with arcus

**Cornea -** Three levels of transparency from completely clear to slightly opaque **Iris -** Five variations
- Light brown
- Medium brown
- Dark brown
- Blue
- Blue/brown with white streaks (areas where pigment has been knocked off)

### Pupil size: 3, 5 and 8 mm

### Shape variations

**Prominent brow/deep set eye -** Two different variations are be included, including normal and deep-set. Will affect how easy it is to access the eye with the different tools.

**Anterior chamber depth** (related to axial length of globe) - Three variations of axial length will be included. Axial length affects anterior chamber depth. Three variations of AC depth will be included, including, normal, shallow, and deep.

### Effects to simulation

- >26 mm. Deep AC. Thin sclera. Color adjustments to sclera and grove
- <22 mm. Shallow AC.

### Simulations variations

### Scleral stiffness

- Difference in resistance to cutting
- Difference in tunnel wall bending

### Anterior capsule

- Two variations of anterior capsule fibrosis (areas of fibrosis will not tear)
   ∘ Centered (normal capsule visible 360°
   ∘ Extends to the side in one quadrant

### Zonular weakness

- Two variations of Zonules weakness will be provided
   ∘ Normal
   ∘ 3 clock hours of damage
   ∘ 6 clock hours of damage
   ∘ 9 clock hours of damage

**Scarring -** Scarring in sclera that affects the visibility of the tools as well as the resistance to cutting.

**Cataracts -** Same type as in Visuals section.

### Iris prolapse

### Iris damage

- Wooden pupil (pupil that does not move)
   ∘ Normal
   ∘ Adhesion to AC
- Iris holes (surgical or traumatic)

### B. Simulator Task List

Preferably, the following list of tasks is simulated by the simulator.

| Task Group 1: Prepare the surgical site to make the tunnel | |
|---|---|
| **1.6 Check the IOP and the block** | |
| **1.7 Perform conjunctival peritomy** | 1.7.1 Localize site and stablize conjunctiva with Colibri |
| | 1.7.2 Cut peritomy |
| **1.8 Cauterize and dry sclera** | 1.8.1 Perform scleral cautery |
| | 1.8.2 Dry the sclera |
| Task Group 2: Make the tunnel | |
| **2.1 Perform scleral groove dissection** | 2.1.1 Localize site and stabilize globe with Colibri |
| | 2.1.2 Cut initial groove |
| | 2.1.3 Finalize groove depth |
| **2.2 Perform tunnel dissection** | 2.2.1 Create central tunnel |
| | 2.2.2 Extend central tunnel to the right |
| | 2.2.3 Extend central tunnel to the left |
| | 2.2.4 Check and finalize tunnel size and shape |
| **2.3 Produce a paracentesis** | 2.3.1 Align stab blade for paracentesis |
| | 2.3.2 Create paracentesis entry |
| **2.4 Perform a visco-aqueous exchange** | 2.4.1 Insert viscoelastic cannula |
| | 2.4.2 Exchange viscoelastic for aqueous |
| **2.5 Perform keratome entry into AC** | 2.5.1 Insert keratome into the tunnel |
| | 2.5.2 Enter the AC |
| | 2.5.3 Extend entry the full length of the tunnel |

| Task Group 3: Remove the cataract | |
|---|---|
| **3.1 Perform a multicut anterior capsulotomy** | 3.1.1 Insert and orient the cystotome to start capsulotomy |
| | 3.1.2 Cut anterior capsule 360° |
| | 3.1.3 Sweep cut edge |
| **3.2 Perform nucleus dislocation into the tunnel** | 3.2.1 Rotate nucleus to dislocate |
| | 3.2.2 Float nucleus into tunnel |
| **3.3 Perform nucleus delivery** | 3.3.1 Position lens loop |
| | 3.3.2 Deliver cataract |
| | 3.3.3 Remove epinucleus |
| **3.4 Perform a multitear capsulotomy*** | 3.4.1 Insert and orient cystotome to start capsulotomy |
| | 3.4.2 Perform initial cut |
| | 3.4.3 Extend initial cut with multiple tears 360° |
| **3.5 Dissect a secondary tunnel*** | 3.5.1 Create a secondary tunnel plane |
| | 3.5.2 Extend the secondary plane to the left and/or right |
| **3.6 Enlarge the tunnel opening*** | 3.6.1 Stabilize the nucleus and inject viscoelastic |
| | 3.6.2 Slice the lateral tunnel wall(s) |
| | 3.6.3 Reinsert lens loop and deliver nucleus |
| **3.7 Perform a sphincterotomy*** | 3.7.1 Inject viscoelastic |
| | 3.7.2 Make two cuts opposite the tunnel |
| | 3.7.3 Make two cuts towards the tunnel |
| **3.8 Perform an intracapsular cataract extraction*** | 3.8.1 Stabilize the cataract |
| | 3.8.2 Insert lens loop |
| | 3.8.3 Deliver the cataract |
| | 3.8.3 Constrict the pupil |
| **3.9 Perform a scissors vitrectomy*** | 3.9.1 Perform Weck vitreous test at the scleral groove |
| | 3.9.2 Constrict the pupil |
| | 3.9.3 Displace the vitreous towards the tunnel with viscoelastic |
| | 3.9.4 Sweep vitreous trapped in the tunnel into the AC |
| | 3.9.5 Cut and remove the vitreous in the AC |
| | 3.9.6 Trap the vitreous behind the pupil with viscoelastic |
| | 3.9.7 Perform weck vitrectomy at the groove |
| **3.10 Remove hypermature cortical cataract*** | 3.10.1 Perform a linear capsulotomy |
| | 3.10.2 Irrigate liquified cortex and debris out of capsular bag |
| | 3.10.3 Inject viscoelastic into capsular bag |
| | 3.10.4 Insert PC IOL |
| | 3.10.5 Remove anterior capsular flap |
| **3.11 Remove Posterior subcapsular cataract*** | 3.11.1 Perform multitear capsulotomy |
| | 3.11.2 Perform hydrodissection to dislocate nucleus |
| | 3.11.3 Float nucleus into tunnel for removal |
| Task Group 4: Remove residual cortex | |
| **4.1 Perform cortical clean up** | 4.1.1 Prepare the Simcoe cannula |
| | 4.1.2 Form the AC and wash out loose debris |
| | 4.1.3 Remove capsular cortex in all quadrants but sub incisional |
| | 4.1.4 Remove sub incisional cortex |
| **4.2 Remove residual subincisional cortex*** | 4.2.1 Insert 27 gauge cannula through paracentesis |
| | 4.2.2 Aspirate and drag sub incisional cortex into AC |
| | 4.2.3 Wash out cortical debris with Simcoe |

| Task Group 5: Insert and center the Intraocular lens (IOL) | |
|---|---|
| **5.1 Reform capsular bag with viscoelastic** | |
| **5.2 Insert the leading PC IOL haptic into the capsular bag** | 5.2.1 Pass leading haptic through tunnel |
| | 5.2.2 Reorient haptic and enter capsular bag opposite the tunnel |
| **5.3 Insert the trailing PC IOL haptic into the capsular bag** | 5.3.1 Drag trailing haptic into AC |
| | 5.3.2 Rotate trailing haptic into the capsular bag |
| **5.4 Remove anterior or posterior capsule fibrosis*** | 5.4.1 Inject viscoelastic |
| | 5.4.2 Cut across fibrotic capsule |
| | 5.4.3 Tear and remove residual anterior capsule |
| **5.5 Manage iris prolapse*** | 5.5.1 Reduce AC flow and IOP |
| | 5.5.2 Sweep iris back into AC |
| | 5.5.3 Normalize AC depth and pressure |
| **5.6 Insert the leading AC IOL haptic*** | 5.6.1 Pass leading haptic through tunnel |
| | 5.6.2 Reorient haptic and enter the AC angle opposite tunnel |
| **5.7 Insert the trailing AC IOL haptic*** | 5.7.1 Drop the trailing haptic into the AC angle under the tunnel |
| | 5.7.2 Walk the haptics into the proper position |

| Task Group 6: Close the tunnel and restore the eve to physiologic conditions | |
|---|---|
| **6.1 Perform viscoelastic washout** | 6.1.1 Prepare the Simcoe cannula |
| | 6.1.2 Washout viscoelastic |
| **6.2 Normalize AC and check for leaks** | 6.2.1 Insert 27 gauge cannula through paracentesis |
| | 6.2.2 Normalize IOP |
| | 6.2.3 Tap and center IOL |
| | 6.2.4 Check tunnel for leaks |
| **6.3 Inject antibiotic** | |
| **6.4 Reposition conjunctiva** | |
| **6.5 Insert a scleral suture*** | 6.5.1 Stabilize the tissue with the Colibri |
| | 6.5.1 Drive the needle |
| | 6.5.2 Tie the suture |

| | |
|---|---|
| * These tasks may be performed as supplementary, while the remainder of tasks are standard. | |

### C. Detailed Simulation Specifications

### Section 1: Global features

The following global features define the set of condition for all simulation scenarios.
1.1 Global ocular conditions
   1.1.1 Basic anterior segment anatomy showing proper shapes, tissue textures, colors, and relationships
   1.1.2 Selected anatomical variations
      1.1.2.1 Types of cataracts each with capsular bag, cortex, and nucleus: NS cataract, Hypermature NS, Cortical cataract, Hypermature cortical, Posterior sub capsular, NS cataract dislocated to the left or right
      1.1.2.2 Limbus: lightly pigmented, and heavily pigmented
      1.1.2.3 Corneal clarity: Normal clarity, Moderate haze, Corneal arcus
      1.1.2.4 Eyelid/lashes/drape: Dark skin, Indian skin, and Caucasian skin. Shows skin and lashes covered by standard drape with speculum in position. Feels contact with eyelid through the drape/speculum but no deformation required
      1.1.2.5 Conjunctiva: Normal vascularity, Increased vascularity, Pinguecula, Pterygium, Limbal pigmentation.
      1.1.2.6 Iris: Dark brown, Medium brown, Blue, Atrophic (white streaks).
      1.1.2.7 Sclera: Yellowish and White. Shows the realistic look of cauterized sclera as cautery is applied.
      1.1.2.8 Capsular bag: Shows realistic look and behavior of anterior capsule including tearing and cutting. Shows realistic look and behavior of posterior capsule including tearing and puncturing. Shows generalized central anterior capsular fibrosis. Shows localized anterior capsular fibrosis extending under pupil.
      1.1.2.9 Zonules: Shows realistic behavior of loose zonules in 1 or 2 quadrants. This is shown as easy movement of the lens away from the loose zonules during capsulotomy but without dislocation. Highlighted by appearance of the red reflex in area of loose zonules. Shows realistic behavior of broken zonules in 1 or 2 quadrants. This is shown as dislocation in the direction of the broken zonules.
      1.1.2.10 Preselects anatomical variations
   1.1.3 Tissue textures: Corneal tissue has smooth silicone-like feel with slight friction. Scleral tissue has slightly rough fibrous feel with moderate friction. Iris tissue has no feel but visual texture is an irregular web of fibrous material with radial orientation.
   1.1.4 Conjunctival movement: Realistic movement constrained by attachment to limbus and lid speculum. Realistic stretching constrained by elasticity of the tissue and loose attachment to Tenon's
   1.1.5 Tear film and debris: Wet cornea as primary condition. Dry cornea with fuzzy specular reflex. Transitions realistically from wet to dry cornea. Turns off corneal drying. Realistically irrigates cornea on command. Particulate debris on cornea shows realistic random dispersal and behavior.
   1.1.6 IOP: Normal IOP 15 to 25 mm Hg, Low IOP 5 to 10 mm Hg, High IOP 40 to 50 mm Hg. Zero IOP - only intrinsic tissue stiffness is felt. Varies IOP continuously from 0 to high IOP. Preselects IOP.
   1.1.7 Eye movement: Eye movement of blocked eye with realistic snap back and stiffness. Eye movement of poor block with eye rolling up requiring force to center. Preselects eye movement.
   1.1.8 Bimanual interactions: Realistic ballottement with left or right instrument with dependency on IOP. Realistic ballottement from any surface to any other surface of the globe. Realistic two instrument collision look and feel.
   1.1.9 Pupil movement: Realistic response to viscoelastic. Realistic response to surgical conditions including vitreous loss, iris contact, posterior synechia, or injection of acetylcholine. Realistic response to all instruments and IOLs. Varies pupil size continuously from minimum to maximum diameter. Preselects pupil dilation size.
   1.1.10 AC depth (lens-iris diaphragm movement): Shows normal depth AC with volume approximately 0.25 cc. Show shallow AC with volume approximately 0.15 cc. Shows excessively deep AC with volume approximately 0.35 cc. Varies smoothly from shallow to deep AC with dependency on posterior pressure, fluid flow in AC, and area of the inner tunnel opening.
   1.1.11 Position of globe relative to head rest: Realistic relationship between orbital rim and limbus in all axes. Show deep set eye with z axis 5 mm lower than normal. Show realistic displacement of the eye approximating pressure on head rest in the X, Y, and Z axes. Show left or right eye with proper relationship to orbital rim.
   1.1.12 Hand pieces
      1.1.12.1 Proper feel and functionality of right hand piece suitable for: Blade handles, Scissor handle and action, Simcoe cannula, Sinskey handle, Lens loop, IOL forceps, Needle holder, 27 gauge cannula on saline syringe, 25 gauge cannula on viscoelastic syringe, Colibri forceps, Cautery, and Weck sponge.
      1.1.12.2 Proper feel and function of left hand piece suitable for: Stab blade, Colibri forceps, Weck sponge, Sinskey hook.
      1.1.12.3 Proper feel and function of Simcoe aspiration syringe suitable for aspiration and irrigation.
   1.1.13 Microscope display
      1.1.13.1 Realistic through the microscope field of view including entrance pupil size, round 40° viewing field angle, and black rim around view.
      1.1.13.2 Realistic color and color saturations of display.
      1.1.13.3 Realistic through the microscope brightness: Show dim lighting to simulate microscope light failure. Hi intensity illumination approximately the same as setting 5 on Zeiss.
      1.1.13.4 Show realistic white light.
      1.1.13.5 Show even illumination of all tissues in surgical field.
      1.1.13.6 Accurate 6 X magnification shows round surgical field of 35 mm.
      1.1.13.7 Show addition of corneal magnification approximately 10% for all elements in the AC including instruments and ocular structures.
      1.1.13.8 Realistic through the microscope stereopsis.
      1.1.13.9 Realistic defocus.
   1.1.14 Realistic, single light source specular reflections
1.2 Realistic instrument appearance, mechanics, and function: (1) Speculum- static image only, but shows interaction with other instruments. (2) Colibri forceps. (3) Westcott scissor. (4) Weck spear sponge, wet and dry. (5) Wet field cautery. (6) Crescent blade. (7) Stab blade. (8) 25 gauge cannula, (9) Keratome, (10) Cystotome, (11) 27 gauge cannula, (12) Lens loop, (13) Simcoe cannula, including a Left hand piece I&A syringe and Right hand piece Simcoe and tubing, (14) PC IOL out of packaging, (15) AC IOL out of packaging, (16) Sinskey hook, (17) Needle holder, (18) 1/2 circle spatula needle for 10-0 nylon, (19) Voice commands to activate each instrument for each hand piece for example: "Colibri, left hand please".
1.3 Realistic fluid appearance, hydrodynamics, and interactions with tissue: (1) Saline/aqueous same as 4.2.6, (2) Viscoelastic same as 3.6.3 - 3.6.4, 3.7.4 - 3.7.5, (3) Vitreous same as 4.1.12, (4) Bubbles in AC - show single bubble and groups of bubbles, (5) Bubbles on surface of conjunctiva, (6) Suspended debris/blood, (7) Voice commands to initiate or stop flow: Irrigate cornea, Irrigation on (Simcoe), and Irrigation off (Simcoe).

### Section 2: Conjunctival Peritomy and Scleral Cautery Training Features Milestone Definition and Evaluation Criteria.

The simulator is judged as ready for training (RFT) based on the realism and training features of the conjunctival peritomy and the application of cautery in an anatomically correct model. RFT criteria also include evaluation of the following variations and complications at designated milestones: 1. Conjunctival bleeding, 2. Conjunctival tearing, 3. Excessive cautery causing charring of scleral tissue, 4. Bleeding of perforating scleral vessel, and 5. Tenon's remains attached to sclera. The simulator is judged as RFT based on realism and training features of the surgical steps in an anatomically correct model as viewed through an operating microscope.

### Step 2.3 Conjunctival peritomy and scleral cautery

2.3.1 Wet field cautery tip features: Interaction with speculum produces metal on metal feel. Interaction with lid margin produces metal on lid feel. Interaction with other tools produces metal on metal feel. Triggers cautery using the right hand piece wings. Emits realistic sound of activated cautery device. Cautery is applied to the sclera with dependency on: activation of hand piece, orientation of the flat side of the tip against the scleral wall (shows that if the flat side is not against the sclera, no cautery effect is produced), and speed of tip movement across sclera (shows that slow movement produces more cautery effect than fast movement).
2.3.2 Colibri features same as 3.1.1. Scissor blade can be passed under Colibri.
2.3.3 Westcott scissor features: Interaction with speculum produces metal on metal feel. Interaction with lid margin produces metal on lid feel. Interaction with other tools produces metal on metal feel. Shows that cutting of conjunctiva with dependency on: points of contact with the scissor blade as it closes, and stretching of the conjunctiva across the scissor blade as it closes. Shows that scissor blade is constrained by attachment of conjunctiva to limbus. Able to place one or both scissor blades under the conjunctiva.
2.3.4 Weck sponge features same as 4.1.14.1 - 4.1.14.5. Able to push conjunctiva away from limbus with dry sponge. Able to dry scleral wall at any angle of contact with the sponge. Shows stiffness of sponge is dependent on the amount of fluid absorbed by the sponge. Shows that a wet sponge is less stiff than a dry sponge.
2.3.5 Conjunctival features same as 3.1.4, 3.5.4.2, and 4.3.3. Shows realistic deformation of conjunctiva over one or both scissor blades. Shows conjunctival cutting controlled by stretch of conjunctiva over the scissor blade as it closes. Shows that residual conjunctiva at the limbus is dependent on: (1) the pressure applied by the blade under the conjunctival against the conjunctival insertion (this insertion constrains the blade movement (2.3.3.5); shows that residual conjunctiva is minimized when gentle pressure is applied against the insertion; (2) the angle of the blade at the conjunctival insertion (shows that residual conjunctiva is minimized when the plane of the scissor blade is parallel to the scleral wall). Shows that the conjunctiva contracts locally when in contact with activated cautery tip. Shows residual Tenon's capsule attached to scleral wall with dependency on: (1) Insertion of the scissor blade into the subconjunctival pocket, (2) Maintenance of gentle force with the scissor blade downward against the scleral wall while cutting.
2.3.6 Scleral features: Shows realistic wet sclera. Shows realistic dry sclera with dependency on contact with the dry weck sponge. Shows realistic response of sclera to cautery with dependency on the amount of energy applied (2.3.1.5). Shows that proper energy produces darkening of the color. Shows that excessive energy produces charring. Shows that inadequate energy produces no effect. Shows that proper or excessive energy prevents bleeding.
2.3.7 Moves seamlessly through all of Step 2.3 changing only the instruments.
2.3.8 Moves seamlessly from Step 2.3 to Step 3.1 changing only the instruments.

### Section 3: Tunnel Dissection Training Feature Milestone Definition and Evaluation Criteria

The simulator is judged ready for training (RFT) based on the realism and training features of all the steps in making the MSICS tunnel (detailed below) in an anatomically correct model. RFT criteria also includes evaluation of the following various variations and complications:
1. Show of normal scleral groove variations in length, orientation, depth, and appearance.
2. Show of variations in limbal landmarks, corneal landmarks, and corneal clarity.
3. Show of variations in skin color, iris color, scleral color, and conjunctival vascularization.
4. Show of normal variations in tunnel dissection limits including lateral limits and inner tunnel limits.
5. Show of normal variations of the inner tunnel opening length.
6. Show of normal variations in keratome stab entry site and inner tunnel opening orientation.
7. Show of inaccuracies in use of the crescent blade including lateral tunnel lacerations, shredding of scleral groove, scleral laceration, premature entry, and button holes;
8. Show of inaccuracies in use of keratome including snagging in tunnel, deviations of inner tunnel opening from inner tunnel limit, premature entry, button holes, lateral wall lacerations, inner limit extension into cornea, iris contact, anterior capsule rupture, and Descemet's scroll.
9. Show of normal variations in paracentesis including location, orientation, length, and width.
10. Show of inaccuracies in paracentesis including excessive stabbing force, failure to align with Colibri, conjunctival stabbing or laceration, and inaccurate angle of entry with or without iris contact.
11. Show of variations in delivery of viscoelastic including patterns for filling of AC and volume of viscoelastic delivered.
12. Show of inaccuracies in delivery of viscoelastic including failure to attach cannula to syringe properly (as represented by failure of surgeon to give a verbal command to check cannula connection), iris contact, corneal contact, misdirection of cannula, overfilling of AC, and underfilling of AC.
13. Management of iris prolapse.
14. Management of premature entry and button hole by recutting new tunnel at a new plane.
15. Show of the effect of a small tunnel.
16. Show of various causes of the leaking tunnel.
17. Ability to enlarge a small tunnel with keratome.
18. Management of leaking tunnel with suturing.
19. Ability to repair button holes and lateral tunnel lacerations with suturing.

The steps of Section 3 will be integrated into a continuous scenario. Complications may be represented in some cases as an error message or as a visual representation of the complication. Alternatively, there is an ability to repair or correct complications.

### Step 3.1 Scleral groove incision (right and left hand)

3.1.1 Colibri features (left hand):
   3.1.1.1 Interaction with crescent blade and speculum shows metal on metal feel.
   3.1.1.2 Interaction with lid margin shows stiffness of lid margin
   3.1.1.3 Able to feel first touch and show synchronization of touch with visual
   3.1.1.4 Stabilizing globe with closed Colibri shows dependency on the force against globe of at least 30 mm Hg as measured by IOP.
   3.1.1.5 Successfully grasping intact conjunctiva, cut edge of conjunctiva away from the limbus, or cut edge of conjunctiva at the limbus shows dependency on contact with both teeth of the Colibri either simultaneously or one before the other.
   3.1.1.6 Maintaining successful grasp on intact conjunctiva, cut edge of conjunctiva away from the limbus, or cut edge of conjunctiva at the limbus shows dependency on the amount of tissue grasped. Shows if the teeth do not grasp the conjunctiva at least 0.2 mm past the cut edge, the Colibri will not hold.
   3.1.1.7 Maintaining successful grasp on intact conjunctiva, cut edge of conjunctiva away from the limbus, or cut edge of conjunctiva at the limbus shows dependency on the amount of tension applied to the tissue. Show that once the conjunctiva starts to stretch, only slightly more force will cause the edge to tear.
   3.1.1.8 Successfully grasping the groove shows dependency on placement of lower Colibri tooth in the groove before closing Colibri 3.1.1.9 Able to press against the eye with the Colibri open or the Colibri closed.
   3.1.1.10 Shows that pressure against the globe is required with the teeth open prior to grasping limbus or groove. Pressure on the eye is not required to pick up the intact or cut edge of conjunctiva.
   3.1.1.11 Change in IOP by pressing on limbus, cornea, or sclera with the Colibri open or closed shows limits of deformation at an IOP of about 80 mm Hg.
3.1.2 Crescent blade features (right hand):
   3.1.2.1. Interaction with Colibri and speculum shows metal on metal feel
   3.1.2.2 Interaction with lid margin shows stiffness of lid margin
   3.1.2.3 Able to feel first touch and show synchronization of touch with visual
   3.1.2.4 Bimanual interaction with Colibri shows that the IOP is dependent on the force of each instrument
   3.1.2.5 Bimanual interaction with Colibri shows equivalence across all tissue models
   3.1.2.6 Bimanual interaction with Colibri shows proper correlation with IOP in the living eye
   3.1.2.7 Dragging the cutting edge backwards (away from the limbus) over the sclera produces a squeegee effect. This effect is needed for tip and right side of blade only.
   3.1.2.8 Dragging the cutting edge of the blade backwards over the groove produces a speed bump effect (used to localize the groove haptically for deepening the groove)
   3.1.2.9 The cutting track shows exact dependency on the movement and position of the crescent edge in contact with the sclera
   3.1.2.10 The depth of cut shows dependency on the force being applied. Shows a deeper cut results from more force.
   3.1.2.11 The depth of cut shows dependency on the IOP. Shows a deeper cut results when IOP is higher.
   3.1.2.12 The depth of cut shows dependency on the alignment of the short axis with the cutting direction. Shows 20% shallowing if off axis up to 5° and dragging over sclera when >5°.
   3.1.2.13 The cutting track shows dependency on alignment with the short axis of blade with the cutting direction. Shows widening of the groove if the short axis is off the direction of the cutting by up to 5°.
   3.1.2.14 The resistance while cutting shows dependency on alignment of the short axis with the cutting direction up to approximately 5° of axis. Beyond this, the blade will stop cutting and will drag across the sclera without cutting.
   3.1.2.15 The resistance and texture while cutting shows dependency on the friction of the scleral fibers against the blade edge
   3.1.2.16 Shows realistic combined effect of speed, blade force, blade angles (long and short axis), and IOP based on comparison with Sierra Leone data
   3.1.2.17 Able to use the blade right side up or upside down to make the groove
   3.1.2.18 Shows that a right side up cut produces a bevel down edge on the side of the groove nearest the limbus and a bevel up on the opposite side.
   3.1.2.19 Shows that upside down cut produces no bevel.
   3.1.2.20 Shows the crescent blade edge snags the beveled groove by sliding up into it from below.
   3.1.2.21 Able to use Colibri to direct the cut by moving the globe while holding the crescent against the sclera.
3.1.3 Scleral features:
   3.1.3.1 Realistic look and feel to scleral interaction with crescent and Colibri
   3.1.3.2 No cuts from contact with crescent blade edges if no movement on the sclera
   3.1.3.3 Wet feel to scleral texture
   3.1.3.4 Accurate specular reflections and surface deformation during cutting
   3.1.3.5 Visually realistic scleral groove showing variable depth of cut up to and including scleral perforation (may not show iris prolapse at M3)
   3.1.3.6 Visually realistic scleral groove showing dependency on alignment of short axis with direction of cut. A 'V' type beveled groove results when the short axis is aligned. A 'U' type non-beveled groove results when short axis not aligned up to 5°. No cut occurs when alignment of short axis is off by more than 5°.
   3.1.3.7 Haptically realistic cutting of the scleral groove with dependency on force against the sclera and IOP. Shows there is more resistance when cutting with more force against sclera. Shows that pressure on the limbus with the Colibri to raise the IOP reduces the resistance to cutting
   3.1.3.8 Haptically realistic cutting of the scleral groove with dependency on alignment of short axis with existing cut. Shows more resistance when direction of force applied is off axis with crescent short axis up to 5°, and slippage if >5°.
   3.1.3.9 Haptically realistic groove feels like speed bump when wiping down across it with back side of crescent.
   3.1.3.10 Haptically realistic groove snags cutting edge of crescent blade when sliding up towards it. This effect is maximized when a beveled groove is produced on first pass by using the crescent blade in upright position.
   3.1.3.11 Haptically realistic second pass with dependency on alignment and direction of movement relative to existing cut. Shows that getting out of the existing track is like crossing speed bumps not like falling in a hole.
      *(3.1.3.9 - 11 is used to position the crescent tip in the groove for the second pass and to stay in the groove for the second pass).*
   3.1.3.12 Sclera doesn't cut unless there is movement of crescent on the scleral wall, enough force from the crescent, and cutting angle of at least 30°
   3.1.3.13 Sliding across the sclera perpendicular to the cutting edge of crescent produces a squeegee effect.
   3.1.3.14 Shows the effect of IOP on scleral wall stiffness and resistance to cutting.
   3.1.3.15 Accurate feel and look to cutting groove without gaps in the graphics, or dragging or hesitation of the blade movement.
3.1.4 Conjunctival features
   3.1.4.1 Limbal conjunctiva stretches where grasped by Colibri with dependency on the proximity to its insertion at the limbus. Shows that failure to apply pressure against the limbus with the open Colibri arms before grasping results in a poor grasp and tearing of the conjunctiva.
   3.1.4.2 Conjunctiva falls back from the scleral groove with dependency on the rotation of the globe. Shows the conjunctiva sliding away from the groove if you rotate the globe down and covering the groove if you roll the eye up.
   3.1.4.3 Shows conjunctival tears at the point of grasping with the Colibri with dependency on the amount of stretch. Shows that once the conjunctiva starts to stretch, only slightly more force will cause the edge to tear (3.1.1.7).

### Step 3.2 Central tunnel dissection (right and left hand)

3.2.1 Colibri features same as 3.1.1 features for grasping limbal conjunctiva
3.2.2 Crescent features (right hand):
   3.2.2.1 Basic features same as 3.1.2.1- 3.1.2.6
   3.2.2.2 Shows cutting by pivoting the tip (circumferential cutting like a circular saw blade) and slicing with either edge (linear cutting like a knife).
   3.2.2.3 The depth of dissection during central tunneling shows dependency on the angle of the long axis relative to the curve of the sclera and limbus. Shows correct, deep, or shallow dissection.
   3.2.2.4 The depth of dissection during central tunneling at the limbus shows dependency on the "straightening" of the limbal curve. Shows that failure to lift the tip during limbal dissection results in premature entry.
   3.2.2.5 The depth of dissection during central tunneling shows dependency on the starting depth in the scleral groove
   3.2.2.6 The shape of the dissected space shows exact dependency on the travel of the cutting edge of the crescent blade
   3.2.2.7 The resistance while dissecting shows dependency on the orientation of the short axis of the blade relative to direction of cutting vector. Shows that if the short axis is tilted more than 5° off the plane of the dissection, resistance increases by 20 to 30%.
   3.2.2.8 Shows that progression in cutting is proportional to the movement of the cutting edge (slicing vs chopping effect).
   3.2.2.9 The resistance while dissecting shows dependency on the amount of force on the non-cutting surfaces of the blade (friction effect). Shows that pressing down on the sclera during dissection increases resistance. Lifting against outer tunnel wall only deforms the wall with no significant change in resistance.
   3.2.2.10 Shows use of the crescent blade to make the central tunnel centered at any position between 9 and 3 o'clock.
   3.2.2.11 Shows realistic combined effect of speed, blade force, blade angles (long and short axis), and IOP based on comparison with Sierra Leone data
   3.2.2.12 Shows button holing effect at any location on the outer tunnel wall.
   3.2.2.13 Shows premature entry effect (lower IOP, fluid leakage, +/- iris prolapse) at any location on the inner tunnel wall.
3.2.3 Tunnel features:
   3.2.3.1 Interaction with the groove to start the dissection same as 3.1.3.10 - 3.1.3.12
   3.2.3.2 Visually realistic tunnel dissection showing dependency on the exact position of the Colibri. Shows realistic appearance of crescent in the tunnel and the appearance of the tip and corneal haze corresponding to the cutting track in the cornea.
   3.2.3.3 Visually realistic outer wall movement during dissection showing dependency on the orientation of the long and short axis of the crescent. Shows visual effect of tilting the blade in any axis.
   3.2.3.4 Visually realistic tunnel dissection showing dependency on force of the non-cutting surfaces of the crescent blade against the tunnel walls. Shows the effect of pressing down or lifting the crescent blade while dissecting on the appearance of the scleral wall.
   3.2.3.5 Visually realistic tunnel dissection showing dependency on the depth of the dissection. Shows the effect of a thin (<0.2 mm) or thick (>0.4 mm) outer wall on the appearance of the crescent blade deformation of the outer wall. (draping effect).
   3.2.3.6 Haptically realistic tunnel dissection with dependency on the type of tissue (corneal or scleral). Able to feel reduced resistance when entering corneal tissue.
   3.2.3.7 Haptically realistic tunnel dissection with dependency on the depth of the dissection in the sclera. Able to feel reduced resistance when inner wall < 0.2 mm. Able to feel increased resistance when outer wall < 0.2 mm
   3.2.3.8 Haptically realistic tunnel dissection with dependency on the amount of tissue in contact with cutting edge at any given time. Shows increased resistance proportional to amount of tissue being sliced at any given moment.
   3.2.3.9 Haptically realistic tunnel dissection with dependency on the friction between the non-cutting surfaces of the crescent and the inner tunnel wall. Shows that pressing down increases resistance to dissection.
   3.2.3.10 Shows that the dissection plane starts from the bottom of groove with dependency on the starting angle of long axis of crescent
3.2.4 Conjunctival features same as 3.1.4
3.2.5 Moves seamlessly from Step 3.1 to Step 3.2 without removing crescent blade.

### Step 3.3 right lateral tunnel dissection (right and left hand)

3.3.1 Colibri features (left hand):
   3.3.1.1 Same basic features as described in 3.1.1.1 - 3.1.1.11
   3.3.1.2 Successfully grasping the outer tunnel wall edge shows dependency on getting lower blade of Colibri into the tunnel at least 0.2 mm before closing second blade. Shows tearing of grasped edge with minimal force if one tooth of the Colibri is not in contact with inside of outer wall.
   3.3.1.3 Realistic look and feel as Colibri lifts the edge of the outer tunnel wall with dependency on length and width of tunnel (more tenting as you get more central)
   3.3.1.4 Maintaining successful grasp of Colibri on the edge of outer tunnel wall with dependency on force applied by Colibri to lift edge. Shows that excessive force from the Colibri will tear the groove edge even if adequate tissue was grasped.
   3.3.1.5 Maintaining successful grasp of Colibri on the edge of outer tunnel wall with dependency on force applied by the blade. Shows that excessive force by the crescent during dissection will tear the groove edge where grasped.
   3.3.1.6 Shows realistic look and feel of blade dissection forces on the point grasped by the Colibri during dissection.
   3.3.1.7 Accurate interaction with crescent blade. Able to see and feel metal on metal contact and pass the blade under the Colibri.
3.3.2 Crescent features (right hand):
   3.3.2.1 Basic dissection behavior same as 3.2.2.5 - 3.2.2.14
   3.3.2.2 The depth of dissection during lateral tunneling shows dependency on following the curve of the sclera with the short axis of the blade. Shows thinning of outer tunnel wall as dissection progresses laterally if leading edge is tilted up off the scleral curve.
   3.3.2.3 The depth of dissection during lateral tunneling shows dependency on maintaining the long axis of the blade in the central tunnel plane. Shows that if the limbal curve is not consistently lifted as lateral dissection progresses, premature entry will occur
   3.3.2.4 The depth of dissection during lateral tunneling shows dependency on keeping the cutting edge of the crescent blade in the scleral groove. Shows development of shelved outer wall entry (long, thin beveled entry rather than a distinct edge) in front of the scleral groove if the heal of crescent is lifted during dissection.
   3.3.2.5 The resistance while dissecting shows dependency on the amount of tissue in contact with the cutting edge at the moment of cutting. Shows that resistance to slicing decreases when heal of blade is angled in the direction of slicing. Resistance from sweeping motions of the tip will not be felt due to the lack of a rotational force in the haptronics system.
   3.3.2.6 Shows use of the crescent blade to exactly define the inner and lateral limit of the tunnel centered at any position between 9 and 3 o'clock.
   3.3.2.7 Able to slide the crescent blade freely within the dissected space constrained only by the limits of the existing dissection.
   3.3.2.8 Shows laceration of the outer wall edge if sliced with the crescent
3.3.3 Tunnel features
   3.3.3.1 Basic features same as 3.2.3.2 - 3.2.3.10
   3.3.3.2 Able to slide the crescent blade back and forth from right lateral limit to left lateral limit without enlarging the tunnel. Used to test the size of the tunnel haptically since it cannot be seen visually.
   3.3.3.3 Able to feel a dry, high frequency, low amplitude texture while sliding crescent in the tunnel
   3.3.3.4 Realistic bleeding into the tunnel space, pools at the inner tunnel limit
3.3.4 Conjunctival features same as 3.1.4
3.3.5 Able to move seamlessly from Step 3.2 to Step 3.3 without removing the crescent blade

### Step 3.4 left lateral tunnel dissection (right and left hand)

3.4.1. Colibri features same as 3.3.1.1 - 3.3.1.7
3.4.2 Crescent features same as 3.3.2.1 - 3.3.2.8
3.4.3 Tunnel features same as 3.3.3.1 - 3.3.3.4
3.4.4 Conjunctival features same as 3.1.4
3.4.5 Able to move seamlessly from Step 3.3 to Step 3.4 without removing the crescent blade
3.4.6 Able to move seamlessly from Step 3.1 to Step 3.4 without removing the crescent blade

### Step 3.5 AC side port entry (paracentesis right and left hand)

3.5.1 Colibri features- right hand
   3.5.1.1 Basic features same as 3.1.1.1 - 3.1.1.11 except for right hand. (note that the articulation of the right hand piece will be less realistic for the Colibri)
   3.5.1.2 Ability to stabilize the globe during paracentesis shows dependency on the alignment of the Colibri with the stab blade. Shows that if the direction of force applied by the stab blade is off by more than 15° in any direction from intersecting with the point of fixation of the Colibri, the Colibri will not be able to keep the eye from rotating and paracentesis will not be possible.
   3.5.1.3 Shows realistic look and feel of counter forces equal and opposite to stab blade force vector during stab
3.5.2 Stab blade features- left hand
   3.5.2.1 Shows realistic corneal stab with dependency on the angle of contact with cornea. If >15° off perpendicular in any direction, it will cause rotation of the globe rather than stab. Note that rotation will be around the point of contact of the Colibri.
   3.5.2.2 Realistic look at point of initial contact with tip. Note that the sharpness of the tip reduces the feeling of first contact. Shows first contact without haptic feel.
   3.5.2.3 Shows that the tip snags conjunctiva and cornea.
   3.5.2.4 Realistic look when trying to rotate the blade while in the cornea. Will not be able to feel that the cornea prevents the blade from rotating while in the cornea. Shows the deformation of the corneal wound.
   3.5.2.5 The size of the path through the cornea shows exact dependency on the edges of the paracentesis blade.
   3.5.2.6 The orientation of the path through the cornea shows exact dependency on the orientation of the edges of the stab blade.
   3.5.2.7 The non-cutting edge of the stab blade follows the path of the tip of the blade but does not cut tissue
   3.5.2.8 Shows slicing at the cutting edge as the blade is withdrawn from the cornea with dependency on the force against the cutting edge. This feature is used to enlarge the paracentesis after stab incision.
   3.5.2.9 Able to perform a stab at any point within 1 mm of the limbus between 12 and 4 o'clock
   3.5.2.10 Shows realistic combined effect of blade force, blade angle relative to the corneal surface, and IOP based on comparison with Sierra Leone data
3.5.3 Corneal features:
   3.5.3.1 Shows dimple in cornea at first contact with size dependency on IOP and stab blade force
   3.5.3.2 Shows that once any part of the blade is in the cornea, forces against the cornea from all non-cutting surfaces cause movement of the globe and minimal deformation of cornea (due to stiffness of cornea)
   3.5.3.3 Visually realistic cutting path with dependency on shape and orientation of the blade
   3.5.3.4 Shows one way valve effect of paracentesis site with dependency on the angle through the cornea, the length of the corneal opening, and the IOP. Shows fluid leakage from the paracentesis if entry angle is within 15° of perpendicular to the surface of the cornea and the IOP is >20 mm Hg. Shows fluid leakage from the paracentesis if the outer opening is > 2.0 mm and the IOP is >30 mm Hg. Shows fluid leakage from the paracentesis if the outer opening is > 3.0 mm and the IOP is >10 mm Hg. Shows fluid leakage from the paracentesis if the outer entry angle is within 15° of perpendicular to the cornea, the opening is > 1.5 mm and the IOP is >10 mm Hg.
   3.5.3.5 Able to cause fluid leakage from the paracentesis by pressing down on the bottom lip or rotating the stab blade if the IOP is > 10.
3.5.4 Conjunctival features
   3.5.4.1 Basic features same as 3.1.4
   3.5.4.2 Shows bleeding from point where Colibri tears conjunctiva
   3.5.4.3 Shows bleeding from the point where the stab blade tip snags the conjunctiva

### Step 3.6 Viscoelastic / aqueous exchange (right hand only)

3.6.1 25 gauge cannula features - right hand
   3.6.1.1 Realistic look and feel as cannula contacts tissue. Cannula shows slight flexibility in all directions (unlike cystotome which is flexible only in one axis) and some recoil (but not as much as 27 gauge cannula which is more flexible 4.2.2.5)
   3.6.1.2 Ability to slide cannula tip into paracentesis shows dependency on the angle of approach. Shows that cannula must be lined up with the orientation of the stab incision to enter.
   3.6.1.3 Ability to slide cannula tip into paracentesis shows dependency on the force against outer lower corneal lip. Shows that slight downward pressure on bottom lip allows cannula tip to enter paracentesis.
   3.6.1.4 Shows the cannula is constrained by the paracentesis size and orientation.
   3.6.1.5 Ability to enter the AC shows dependency on the force on the lower lip of the paracentesis at the opening into the AC. Shows the tip of the cannula snags on the upper lip.
3.6.2 Corneal features
   3.6.2.1 Shows that upper lip of cornea snags the tip of cannula at the entrance to the paracentesis if entry angle is not the same as the paracentesis.
   3.6.2.2 Shows that upper inner lip of cornea snags tip of cannula. This can only be avoided by slight downward pressure against the inner lip.
   3.6.2.3 Ability to enter paracentesis shows dependency on IOP. Shows that IOP >30 mm Hg makes the entry wound more difficult to enter. Shows that IOP <10 causes the cornea to deform and makes entry more difficult.
   3.6.2.4 Same basic fluid leakage features as 3.5.3.4 - 3.5.3.5
3.6.3 Viscoelastic features
   3.6.3.1 Viscoelastic displaces the aqueous volume one to one
   3.6.3.2 Viscoelastic flow shows dependency on the force on the plunger. Shows significant force needed to move the viscoelastic (much more than saline).
   3.6.3.3 Viscoelastic flow shows dependency on the size of the cannula. Shows flow is contrained by the size of the cannula no matter how much force is applied to the plunger.
   3.6.3.4 Viscoelastic flow shows dependency on the direction the cannula is aimed
   3.6.3.5 Viscoelastic flow shows dependency on the proximity to solid structures in the AC. Shows viscoelastic fills first available space, forms bolus and then expands.
   3.6.3.6 Viscoelastic flow shows dependency on the viscosity of the viscoelastic (snake-like)
   3.6.3.7 Viscoelastic loss through tunnel shows dependency on pressure on the inner tunnel wall with the non-cutting surface of the keratome in Step 3.7
   3.6.3.8 Shows that viscoelastic appears to trap and move particulate debris
   3.6.3.9 Change in IOP shows dependency on relative outflow of aqueous compared to inflow of viscoelastic. Shows that inflow of viscoelastic without outflow of aqueous raises the IOP. IOP increases dramatically with only 0.1 cc of viscoelastic injected if there is a 2.5 cc of aqueous in the AC and it does not flow out.
3.6.4 Anterior chamber features
   3.6.4.1 Viscoelastic force against the pupil causes pupil dilation up to 8.0 mm
   3.6.4.2 Viscoelastic viscosity causes dispersion of bubbles across the corneal dome towards the AC angle.
   3.6.4.3 Viscoelastic force against the anterior capsule causes flattening with dependency on the type of cortex (see 4.1.10.2). Shows soft and liquid cortex deforms readily while the dense cortex does not.
   3.6.4.4 Viscoelastic causes deepening of the AC with dependency on the amount of viscoelastic in the AC (see 4.1.7.4).

### Step 3.7 Keratome entry into AC (right and left hand)

3.7.1 Colibri features:
   3.7.1.1 Basic features same as 3.3.1.1 - 3.3.1.6
   3.7.1.2 Able to pass the keratome blade under the tip of the Colibri while it grasps the tunnel
   3.7.1.3 Able to feel metal on metal contact between Colibri and keratome blade
3.7.2 Keratome features:
   3.7.2.1 Shows stabbing (both edges cutting at the same time) and slicing (one edge cutting)
   3.7.2.2 Ability to successfully stab into the AC shows dependency on the size of the ring of light around the keratome tip (the dimple). Shows that a 3 mm ring of light is needed for successful entry. Shows that < 3 mm dimple results in tracking into cornea rather than into AC.
   3.7.2.3 The resistance to stabbing into the AC shows dependency on the orientation of the short axis. Shows that resistance increases if keratome is tilted along short axis.
   3.7.2.4 The resistance to stabbing into the AC shows dependency on the alignment with the long axis. Shows that resistance to stab entry increases if alignment is off by >5°.
   3.7.2.5 Shows dimple size dependency on the downward force of the tip against the inner tunnel wall (note- not be able to feel the torque)
   3.7.2.6 Shows dimple size dependency on the long axis orientation in the tunnel. Shows that tilting the blade down works better to produce a dimple than pressing down on the inner tunnel wall with non-cutting surface of keratome
   3.7.2.7 Slicing to left and right shows dependency on the relationship of the keratome to the inner tunnel limit: (1) Shows that by approximately following the curve of the corneal dome with the short axis of the keratome, the keratome follows the path of the inner tunnel limit with the least resistance; (2) Shows that by slicing out towards the tunnel, the keratome follows the path of the inner tunnel limit with the least resistance; (3) Shows that keeping the orientation of the long axis of the keratome approximately parallel to the iris, the keratome follows the path of the inner tunnel limit with the least resistance; (4) Shows that by avoiding downward pressure on the inner tunnel wall, the keratome follows the path of the inner tunnel limit with the least resistance.
3.7.3 Tunnel features:
   3.7.3.1 Shows snagging of keratome tip if blade not parallel to plane of tunnel
   3.7.3.2 Shows keratome extending into cornea past inner limit without entering AC with dependency on size of dimple
   3.7.3.3 Shows premature entry in any location on the inner tunnel wall resulting from snagging of keratome tip on inner tunnel wall
   3.7.3.4 Shows Descemet's detachment with dependency on a shelved entry (shallow angle with essentially no edge) to the AC.
   3.7.3.5 Uplift of outer tunnel wall shows dependency on the angle of the long axis of the blade in the tunnel
   3.7.3.6 Uplift of outer tunnel wall shows dependency on the angle of the short axis of the blade in the tunnel
   3.7.3.7 Uplift of outer tunnel wall shows dependency on the location of the keratome in the tunnel
   3.7.3.8 Breakthrough of the tip into the AC suddenly reduces resistance to stabbing
3.7.4 Viscoelastic features:
   3.7.4.1 Viscoelastic loss through keratome AC entry site shows dependency on the IOP
   3.7.4.2 Viscoelastic loss through keratome AC entry site shows dependency on pressure on the inner tunnel wall with the non-cutting surface of the keratome
   3.7.4.3 Viscoelastic loss through keratome AC entry site shows dependency on the resistance to slicing the inner opening (Shows that excessive force during slicing causes loss of visco)
   3.7.4.4 Viscoelastic loss through keratome AC entry site shows dependency on the size of the inner tunnel opening. Shows that the risk of visco loss increases as the inner opening gets longer.
3.7.5 AC features:
   3.7.5.1 Shows AC depth dependency on the amount of viscoelastic + aqueous in the AC. For normal chamber depth should be a total of 0.25 cc. (see 4.1.9)
   3.7.5.2 Shows sudden shallowing of AC from excessive downward pressure on inner tunnel wall during stab entry.
   3.7.5.3 Shows contact with iris or anterior capsule if AC suddenly shallows
3.7.6 Moves seamlessly through all the actions of the keratome without removing the blade
3.7.7 Shows realistic combined effect of speed, blade force, blade angles (long and short axis), and IOP based on comparison with the Sierra Leone data
3.7.8 Able to perform Steps 3.1 - 3.7 changing only the instruments

**Supplementary Step 3.8 Dissecting a secondary tunnel:** The features below are specific for addressing the non-suturing repair of a button hole or premature entry.
3.8.1 Restarts central tunnel dissection features (3.2) from any point in the groove.
3.8.2 Restart right and left tunnel dissection features (3.3 - 3.4) from a new central tunnel.
3.8.3 Adjusts depth of starting point based on the appearance of the thickness of the groove edge. Can start at a point nearer or further from the surface of the sclera in the groove.
3.8.4 Adjusts depth of starting point based on the appearance of tip of crescent blade in the groove. Amount of tip covered by the groove corresponds to depth in the groove.
3.8.5 Shows that the crescent dissection creates a new internal tunnel wall (secondary tunnel) with dependency on the depth of the dissection. If the new plane is above the premature entry, the new tunnel will behave as if there is no premature entry. If the new plane is below the plane of the buttonhole, the new tunnel will behave as if there is no button hole (though it still remains visible).
3.8.6 Produces a second button hole or premature entry during attempted repair of existing button hole or premature entry.
3.8.7 Shows fluid leakage from premature entry site during dissection if the IOP is >15 mm Hg. Shows that reduced force must be used during recutting of tunnel to avoid shallowing of AC.
3.8.8 Refills AC with viscoelastic 25 gauge cannula through the premature entry site if AC shallows.
3.8.9 Shows that the keratome, Simcoe, cannula, IOL, and Sinskey can enter the AC through the secondary tunnel.
3.8.10 Shows that the primary tunnel prior to premature entry or button hole retains its boundaries.
3.8.11 Show that the primary and secondary tunnels constrain the passing of keratome, Simcoe, cannula, IOL, and Sinskey based on their boundaries.

**Supplementary Step 3.9 Enlarging the tunnel opening:** The features below are specific for addressing the problem of a nucleus that will not deliver.
3.9.1 Able to push the nucleus out of the tunnel back into the AC with 25 gauge viscoelastic cannula while injecting.
3.9.2 Able to inject viscoelastic through the tunnel entrance above the nucleus to create a space between nucleus and endothelium.
3.9.3 Able to reposition the keratome in the existing tunnel opening with dependency on the angle of entry. Shows that when the keratome enters parallel to the existing tunnel, there is no resistance to movement or snagging; the keratome should feel like its floating in the tunnel space and passes easily to the inner tunnel limit. Able to feel the keratome tip snag the approximately 0.2 mm lip at the inner tunnel opening. This lip results from normal keratome entry when the keratome handle is lifted and the dimple is produced before entry. Shows that if keratome angle is increased by at least 20° when the tip reaches the inner opening, it will not snag the lip of the inner tunnel opening. Shows that slight downward pressure at the inner tunnel opening with the tip of the keratome is needed to enter the existing opening. This does not produce a dimple since there is no resistance from the inner tunnel wall.
3.9.4 Able to feel existing boundaries of the tunnel or bands of uncut tissue with the edge of the keratome.
3.9.5 Able to extend existing tunnel boundaries to the left or right with the same features as keratome entry (3.7).
3.9.6 Shows realistic interaction between nucleus, tunnel, and keratome at the inner tunnel opening.

**Supplementary Step 3.10 Scleral suturing:** The features below are specific for addressing the problem of wound leak due to tunnel wall problems including outer tunnel wall laceration, button hole, and premature entry.
3.10.1 Colibri features (left hand):
   3.10.1.1 Interaction with needle holder shows metal on metal feel.
   3.10.1.2 Interaction with needle shows metal on metal feel but only when needle is held by the needle holder.
   3.10.1.3 Able to grasp any cut edge of sclera with the Colibri. This includes edges of button hole or outer tunnel wall lacerations.
   3.10.1.4 Able to grasp the surface of the outer tunnel wall at any point, to stabilize it for passing the needle through the wall.
   3.10.1.5 Ability to realistically grasp the suture needle with dependency on the cross sectional shape of the needle. Shows the needle twists when the tying platform of the Colibri is not aligned with the flat cross sectional shape of the needle.
   3.10.1.6 Able to grasp the 10-0 nylon suture anywhere on the tying platform of the Colibri. It is assumed that once grasped, the suture will not slip.
   3.10.1.7 Shows that by grasping at the edge of the visible surgical field in the microscope, a properly sized suture loop (for wrapping around the needle holder tip) can be created and tangling of the suture can be avoided.
   3.10.1.8 Shows realistic interaction of the Colibri with the needle and tissue as the needle is passed. Able to release the grasp on the tissue when the needle is partially passed. Shows that applying pressure with the closed Colibri on the tissue ahead of the needle path influences the path of the needle by deforming the tissue. This pressure is in proportion to the force applied by the needle holder/needle.
   3.10.1.9 Shows realistic interaction of the Colibri with the suture and tissue as the suture is tied. Able to complete the entire knot tying process without releasing the suture grasp. Able to manipulate the curve of the suture loop by changing the angle of the Colibri in any direction.
3.10.2 3/8 curve cutting suture needle features.
   3.10.2.1 Shows that the needle passing through the tissue creates a track through which the suture passes.
   3.10.2.2 Shows that the needle track follows the curve of the needle.
   3.10.2.3 Shows realistic needle characteristics. The tip of the needle cuts like a bevel down keratome and creates the track. The shaft of the needle does not cut but follows exactly in the track of the tip.
   3.10.2.4 Shows that the depth of the track in the tissue is dependent on the angle of entry and the curve of the needle. The depth of the track cannot be more than the radius of curvature of the needle. Increasing the angle of entry results in a deeper track.
   3.10.2.5 Shows that the exit point of the needle (the length of the track) is dependent on the angle of entry, the curve of the needle, and the curvature of the tissue it is passing through. The length of the track cannot be more than the length of the needle. Increasing the angle of entry results in a longer track. Increasing the curvature of the tissue that the needle passes through will shorten the track. Increasing the curvature of the tissue just ahead of the tip of the needle by applying pressure with the Colibri, forces the needle to exit where the tissue is pressed inward.
   3.10.2.6 Shows that the needle bends if the rotation of the needle holder does not follow the arc of the needle track. Shows that the needle cannot be forced to change directions once it has entered the tissue. Attempts to do so will bend the needle.
3.10.3 Needle holder features (right hand)
   3.10.3.1 Shows the stability of the needle is dependent on proper positioning of the needle in the needle holder. Grasping on the round part of the needle allows the needle to rotate in the needle holder when force is applied to pass the needle. Grasping on the flat part of the needle secures the needle against rotation while the needle is being passed. Needle is shown to snap into position when grasping across the flat part of the needle. The needle holder must be perpendicular to the plane formed by the arc of the needle to avoid increased resistance when passing the needle. The needle holder must grasp the needle just past the transition to the flat part of the needle, about halfway around the curve of the needle. This allows adequate length for the needle to be passed in and out of the tunnel and good stability when force is applied.
   3.10.3.2 Shows rotation of the needle holder tip must follow the arc of the needle as it is advanced. This is facilitated by grasping the needle approximately half way along its curve.
   3.10.3.3 Shows the needle holder can grasp the suture at any point. It is assumed that it does not slip once grasped.
3.10.4 10-0 nylon features
   3.10.4.1 Shows realistic color, diameter, and flexibility of the nylon suture. A 7 mm to 8 mm piece of suture will support its own weight when held at one end. A longer piece of suture will bend under its own weight. Even though a longer piece of suture will bend under its own weight, it appears to almost float in the air and settles slowly downward if undisturbed. Suture sticks to surface such as the corneal especially when it is relatively dry. Shows that wetting the cornea allows the suture to float and makes it easy to grasp.
   3.10.4.2 Shows knot tying guided by the movement of both ends of the suture. This includes wrapping around the needle holder (one, two, or three wraps), grasping the free end of the suture with the needle holder, and pulling it through.
   3.10.4.3 Show knot tying with dependency on the force on both ends of the suture.
   3.10.4.4 Show the first throw lying down flat on the tunnel between the entry and exit site of the suture from the tunnel.
   3.10.4.5 Shows a triple throw knot.
   3.10.4.6 Shows a double throw knot.
   3.10.4.7 Shows single throw knot.
   3.10.4.8 Show a single throw knot compacting a triple throw knot as it tightens over it.
   3.10.4.9 Shows tension on suture loop in the tissue with dependency on how the knot is laid down on the tissue before tightening. When a triple throw is laid down straight and flat across the surface of the tunnel with no tension, it tightens without distorting the out tunnel wall when the knot is compacted. When a triple throw is laid down on the surface with tension, it distorts the tunnel wall when it is compacted. This distortion appears as puckering of the tunnel which causes gape at the groove in proportion to the tension. When a triple throw is laid down on the surface with slack in it, it remains loose when compacted.
   3.10.4.10 Shows that the triple throw can be locked by sliding the throws to one side.
   3.10.4.11 Shows realistic breaking point of suture from excessive force from either the needle holder or Colibri.
   3.10.4.12 Shows suture cutting with the Westcott scissors with realistic suture ends left behind at the knot.
3.10.5 Tunnel features.
   3.10.5.1 Shows realistic distortion of the outer tunnel wall as the needle is passed with the following dependencies: (1) Forces applied against the back or top of the needle, distort the tissue but do not puncture or cut, (2) Forces aligned with the tip of the needle, produce a small dimple before puncturing (similar to what happens with paracentesis blade), (3) The force against the tissue is transmitted through the needle from the needle holder. The needle should act as an extension of the needle holder once grasped.
   3.10.5.2 Shows realistic distortion of the tunnel based on tension of the suture.
   3.10.5.3 Shows that the one way valve effect of tunnel is restored when needle is passed at the proper depth, proper length, in the proper position, and with proper tension.
   3.10.5.4 Shows that the needle should enter approximately 1.0 mm behind and exit 1.0 mm in front of a cut scleral edge.
   3.10.5.5 Shows that apposition of sutured cut scleral edges is achieved with dependency on the point of needle entry and exit and the amount of tension placed on the suture. Shows that tissue distortion is minimized by symmetrically placed needle exit and entry sites approximately 1.0 mm from the cut edges. Show that suture tension can be adjusted to produce apposition of tissue edges without distortion if the needle entry and exit sites are approximately 1.0 mm from the edges.

### Section 4: Cataract Removal Training Feature Milestone Definition and Evaluation

### Criteria

The simulator is judged ready for training (RFT) based on the realism and training features of all the steps of the MSICS cataract removal (detailed below) in an anatomically correct model. RFT criteria is also include evaluation of the following variations and complications:
1. Variations of cortex and nucleus for the 5 standard types of cataracts
2. Variations in AC depth including shallow, normal, and deep AC
3. Inaccuracies in use of cystotome for can opener capsulotomy including tunnel snagging, inaccurate capsulotomy size and shape, burying cutting edge in cataract, inaccurate spacing of cuts, and excessive force on zonules
4. Inaccuracies in use of cystotome for multitear capsulotomy including tunnel snagging, inaccurate capsulotomy size and shape, burying cutting edge in cataract, inaccurate spacing of tears, inaccurate tearing angle resulting in run out or inadequate size opening, and excessive force on zonules
5. Inaccuracies in use of cystotome for nucleus dislocation including failure to engage nucleus properly (location, depth, or force), excessive or inadequate rotational force, radial force greater than tangential force, excessive speed of rotational movement, failure to maintain force against outer tunnel wall
6. Inaccuracies in use of 27 gauge cannula for nucleus dislocation including excessive or inadequate amount of fluid injected, failure to sweep the cannula around the equator to position tip under the cataract, angle of contact of the tip with the posterior capsule >20° and force against posterior capsule with the tip (results in PC rupture), excessive or inadequate force of the cannula or tip against the nucleus, and failure to apply force against the inner tunnel wall with the cannula while injecting saline
7. Inaccuracies in use of lens loop for nucleus delivery including excessive force on the tunnel while inserting, failure to slide under the nucleus equator while inserting, inadequate positioning under nucleus for delivery, capture of iris between lens loop and nucleus, misalignment of lens loop during delivery (relative to tunnel), excessive force against nucleus, excessive or inadequate force against tunnel during delivery (cataract size and lens loop combined force), excessive speed of delivery, inaccurate orientation of the lens loop relative to the tunnel opening
8. Inaccuracies in use of Colibri forceps including inaccurate positioning relative to tunnel and lens loop long axis, inaccurate positioning relative to limbus, conjunctival bleeding from tearing of conjunctiva, and excessive or inadequate counter force during delivery relative to resistance of delivery
9. Shows posterior capsule rupture following nucleus delivery with and without vitreous loss
10. Shows wooden pupil and small tunnel effect on nucleus delivery
11. Operating on deep set eye, left and right eye, and moving head
12. Shows zonular weakness from 1 up to 3 quadrants
13. Shows capsular rupture including iris prolapse (with vitreous loss), management of iris prolapse, vitreous management (Weck and scissor vitrectomy)
14. Able to manage wooden pupil and small tunnel including sphincterotomy and tunnel enlargement
15. Shows and manage anterior capsule variations including fibrosis and posterior synechia
16. Able to manage zonular weakness or rupture during capsulotomy and nucleus rotation
17. Shows and manage iris prolapse with premature entry and one or more of the following: IOP >40 (any cause), injection of saline under iris instead of into capsular bag, excessive force on nucleus during nucleus delivery
18. Shows and manage iris prolapse without premature entry and one or more of the following: IOP >60 (any cause), excessive force on tunnel with cystotome, cannula, or lens loop, excessive flow of saline while holding tunnel open, excessive flow of saline under iris instead of into capsular bag, snagging iris with cannula or cystotome causes pigment loss and prolapse (in some cases), excessive force with lens loop during nucleus delivery, entrapped iris with lens loop during nucleus delivery
19. Shows and manage iridodialysis with iris prolapse resulting from entrapped iris during nucleus delivery including iridectomy
20. Shows and manage dislocated nucleus with 1 or 2 quadrant hinge including preexisting dislocation and dislocation occurring during capsulotomy or nucleus dislocation
21. Shows dropped nucleus with standard management of vitreous (no management of nucleus possible)

The steps of Section 4 are integrated into a continuous scenario. Complications may be represented in some cases as an error message or as a visual representation of the complication. Alternatively, there is an ability to repair or correct complications.

### Step 4.1 Anterior capsulotomy (using right haptic alone except for McPherson)

4.1.1 Cystotome features
   4.1.1.1 Interaction with speculum produces metal on metal feel
   4.1.1.2 Interaction with lid margin produces metal on lid feel
   4.1.1.3 Interaction with Colibri produces metal on metal feel and accurate visual and haptic first contact
   4.1.1.4 Insertion through the tunnel shows dependency on orientation of tip relative to tunnel walls
   4.1.1.5 Bending of cystotome shaft shows dependency on the vector of force applied
   4.1.1.6 Positioning of the cystotome shaft is accurately constrained by the shape of the tunnel
   4.1.1.7 Ability to perforate and cut the capsule shows dependency on the orientation of the cystotome tip relative to the capsular surface
   4.1.1.8 Shows exact correlation between the position of the tip and the perforation of the capsule
   4.1.1.9 Able to connect the perforations of the capsule to produce a continuous tear (postage stamp effect)
   4.1.1.10 Shows exact correlation between side to side movement of the tip and the cut of the capsule (blade effect)
   4.1.1.11 Ability to tear the capsule shows dependency on contact of the capsule with the flat side of the tip (spade effect) or the cannula (friction effect).
   4.1.1.12 Shows that tearing will not occur unless cystotome is on top of the capsule.
   4.1.1.13 Shows that as the capsulotomy progresses, the capsular flap must be over the remaining capsule to be manipulated.
   4.1.1.14 Shows combined blade and spade tearing effect with dependency on the direction of movement of the tip.
   4.1.1.15 Shows that capsule tearing propagates to the right and the left of a perforation point with dependency on the direction of the force that is applied. Shows that radial centripetal tears result in a smooth capsulotomy using the multicut technique.
   4.1.1.16 Shape of the capsular tear shows dependency on the direction of tip travel. Centripetal movement tears an inward scallop shape (tips of the scallop on the outer fixed edge point inward), centrifugal movement tears an outward scallop shape (tips of the scallop on the outer fixed edge point outward), non-radial movement produces an asymmetric tear.
   4.1.1.17 The length of the tear shows exact dependency on the tearing of the inner free edge away from the outer fixed rim of capsule based on the movement of the cystotome.
   4.1.1.18 The ability to adjust the direction of a tear with the spade or friction effect of the cystotome shows dependency on the vector of force applied to the free capsular edge. Tearing a circle requires a continuous small centripetal vector component in addition to the linear component.
   4.1.1.19 Control of the forces that direct the tear is easiest near the point of tearing. Shows that small changes in the linear or centripetal forces near the point of tearing cause a change in direction of tear.
   4.1.1.20 Control of the forces that direct the tear is easiest near the point of tearing. Shows that as the point of application of the tearing forces gets farther away from the point of tearing, the centripetal angle gradually increases in a spiral pattern and eventually tearing is not possible.
   4.1.1.21 The ability to adjust the direction of the tear, with the spade or friction effect of the cystotome, shows dependency on the zonular support providing counter force. Shows that lack of support increases the centripetal travel to achieve the usual tearing force.
   4.1.1.22 The resistance while cutting or tearing shows dependency on the force of contact with the nucleus while cutting (chopping block effect) and the type of nucleus. The hypermature nuclear cataract produces the most resistance and the hypermature cortical cataract produces no resistance since the small nuclear chip is freely mobile.
   4.1.1.23 Shows realistic combined effect of speed of movement in the tunnel (tunnel friction and texture) and force against the cataract based on comparison with Sierra Leone data.
4.1.2 Long angled McPherson forceps features
   4.1.2.1 Interaction with speculum produces metal on metal feel.
   4.1.2.2 Interaction with lid margin produces metal on lid feel.
   4.1.2.3 Interaction with other tools produces metal on metal feel.
   4.1.2.4 Able to feel first touch at the scleral groove and show accurate synchronization of touch with visual
   4.1.2.5 Insertion through the tunnel shows dependency on orientation of forceps arms relative to the tunnel. Shows advantage of entering AC with forceps closed.
   4.1.2.6 Ability to grasp the cut free edge of anterior capsule shows dependency on forceps contact on both sides of the capsular flap either simultaneously or one before the other. Note that use of the McPherson will always require a cystotome cut to create a free edge to be grasped by the McPherson.
   4.1.2.7 Ability to maintain the grasp on the free edge of the capsule shows dependency on keeping the arms of the forceps closed.
   4.1.2.8 Ability to maintain the grasp on the free edge of the capsule shows dependency on force applied to the capsule. Shows inaccuracies that result in incomplete capsulotomy and capsular tags.
   4.1.2.9 The movement of the free capsular edge is controlled by the movement of the tip of the McPherson at the exact point where it grasps the capsule.
   4.1.2.10 Ability to tear the free capsular edge shows same dependencies as in 4.1.1.9, and 4.1.1.16 - 4.1.1.21
   4.1.2.11 As the point of grasping moves farther from the point of tearing, the linear component of the tear (in line with the direction of tearing) becomes more difficult to maintain due to space constraints in the AC. Regrasping is required to keep the direction of movement of the point of grasping lined up with the desired direction of tearing.
   4.1.2.12 Shows run out of tear from misdirection of forceps. 4.1.2.13 Able to correct run out by adjusting vector of force on the free capsular edge at the grasping point. Produces the feeling of being able to "lead" the tear back on track.
   4.1.2.14 Able to convert to a multicut capsulotomy using the cystotome if run out occurs.
4.1.3 Viscoelastic cannula (25 gauge) features see 3.6.1
4.1.4 Viscoelastic features see 3.6.3 - 3.6.4, 3.7.4 - 3.7.5
4.1.5 Tunnel features
   4.1.5.1 Uplift of outer tunnel wall shows dependency on the tilting angle of the cystotome in the tunnel.
   4.1.5.2 Outer tunnel wall constrains tilting angle of cystotome with dependency on the proximity to the lateral tunnel limits. 15° tilt possible in central tunnel; 5° tilt possible at lateral limits
   4.1.5.3 Uplift of outer tunnel wall shows dependency on the location of the cystotome in the tunnel.
   4.1.5.4 The inner tunnel opening is controlled by the force applied against the inner tunnel wall; the greater the force, the greater the gap in the inner tunnel opening.
   4.1.5.5 Inward gaping of the inner tunnel wall is constrained by contact with the lens iris diaphragm
   4.1.5.6 Snagging of cystotome tip on inner tunnel wall shows dependency on a tip angle of >30° downward relative to the plane of the tunnel.
   4.1.5.7 Basic tunnel features for McPherson same as 4.1.5.1 - 4.1.5.3
   4.1.5.8 Basic tunnel features for Westcott scissors same as 4.1.5.1 - 4.1.5.3
   4.1.5.9 The tunnel walls constrain the opening of the forceps or scissors when opened perpendicular to the tunnel plane (visual effect only since hand piece will not have active simulated feel).
4.1.6 Anterior capsule features
   4.1.6.1 Realistic interactions with the anterior capsule are based on three primary properties:
      4.1.6.1.1 The anterior capsule is stretched and supported by the zonules like the springs of a trampoline. The rest of the capsular bag hangs below it and is supported primarily by the anterior capsule. For example, even if there is a hole in the posterior capsule, if the anterior capsule fixed ring is intact, an IOL can be placed on top of the anterior capsule.
      4.1.6.1.2 The capsule is inelastic and has significant tensile strength when intact.
      4.1.6.1.3 The capsule has no stiffness. Its shape depends on the contents filling the bag and on the supporting zonules. For example, if the cortex leaks out of the bag during capsulotomy, the bag will always collapse upwards to a flat plate since it is stretched by the zonules. If a section of zonules are weak or broken and the cortex leaks, the capsule will sag or fold over in this area.
   4.1.6.2 Curvilinear tears of the capsule produce a convex edge and a concave edge. Shows the convex edge is free due to lack of stiffness and the concave edge is fixed due to inelasticity.
   4.1.6.3 Shows the convexly curved free edge will fold back over itself from the two points where the edge ends.
   4.1.6.4 Shows that the free edge of the capsule folds upwards or downwards without resistance.
   4.1.6.5 Shows that the concave, fixed edge cannot fold back on itself unless the zonules are broken.
   4.1.6.6 Shows that the fixed edge resists the cystotome due to inelasticity of the capsule. Blunt force against the fixed outer edge of capsule produces a highly localized 'v' like deformation in the edge.
   4.1.6.7 Able to produce a "nick" in the fixed capsular edge with cystotome or scissors.
   4.1.6.8 Shows that the nick produces a hinged capsular flap on the fixed rim that effectively enlarges the capsular opening.
   4.1.6.9 Shows that excessive force on the hinged capsular flap on the fixed rim can cause the nick to run out
   4.1.6.10 Shows that a radial curvilinear tear or cut from the center outward produces a free edge that can be folded over on itself as the starting point for the circumferential curvilinear tear (J stroke effect).
   4.1.6.11 Shows that a tear will not propagate through a curve. It will propagate only at the point of a cut.
   4.1.6.12 Shows the smooth propagation of a circular tear with dependency on the curve of the surface being torn. Shows increased risk for run out when the anterior capsule surface is convex and reduced risk when it is concave. Shows this curvature with various cataract types.
   4.1.6.13 Shows the smooth propagation of a circular tear with dependency on the centripetal vector inwards and away from the fixed edge. Shows that centripetal cutting and tearing produces a smooth capsular tear.
   4.1.6.14 Shows that a linear cut in the capsule produces two free edges.
   4.1.6.15 Shows that tearing will only occur at each end of a cut and not along the cut edge.
   4.1.6.16 Shows 3 variations in capsule strength: (1) normal with behavior as described above, (2) thin, friable capsule found in hypermature cataracts with reduced resistance to puncture and tearing, and (3) thick stiff capsular plaques found in some hypermature cortical cataracts with intrinsic stiffness and increased resistance to puncture and tearing
   4.1.6.17 Able to vary the surface curvature of the capsule with dependency on the amount of viscoelastic in the AC and the density of the nucleus.
   4.1.6.18 Shows collapse of capsular bag if liquefied cortex leaks out.
   4.1.6.19 Shows a realistic hinged anterior capsular flap with folding at the limits of the capsular tears.
4.1.7 Zonular support features
   4.1.7.1 Graphically and haptically realistic movement of lens with dependency on downward force applied at any point on the surface. Shows that when zonules are intact, the lens displaces downward without tilting.
   4.1.7.2 Zonular weakness shows graphically and haptically realistic tilting of lens (space between iris and surface of lens) with dependency on the amount of force is applied in zone of zonular weakness.
   4.1.7.3 Zonular weakness shows graphically and haptically realistic decentration of the lens with dependency on the amount of radial force applied.
   4.1.7.4 Able to realistically show that excessive filling with viscoelastic increases distance of the capsular surface and iris from the cornea. Note that this has a visual (stereoscopic) and haptic component. The haptic component is experienced as an increase in the distance of the capsule from the inner tunnel opening. Contact with the capsule requires more of an angle of the cystotome at the inner tunnel opening. This increases the gaping of the outer tunnel wall which results in flattening of the AC.
   4.1.7.5 Shows sudden release of support with dependency on excessive force on the lens or capsule AND preset risk factors (age and hypermaturity of the lens).
   4.1.7.6 Shows release of support in 1 or 2 quadrants results in dislocation (tilting) of the lens proportionate to loss of support.
4.1.8 Iris features
   4.1.8.1 Interaction with instruments shows graphically realistic movements of iris
   4.1.8.2 Shows distinction between tilting lens (with gap between lens and iris) and over-filling with viscoelastic (iris and lens move together away from the cornea)
   4.1.8.3 Shows realistic V shaped deformation of the pupil edge caused by contact with tip of cystotome or McPherson
   4.1.8.4 Shows realistic V shaped deformation of the pupil edge caused by vitreous strand passing over the edge of the pupil and out the tunnel or paracentesis
   4.1.8.5 Shows realistic constriction of pupil with dependency on number of instrument contacts with iris or as preset condition
   4.1.8.6 Shows 2 to 3 clock hours of adhesions between capsule and iris with the following characteristics: (1) Iris is immobile over the adhesion; (2) Cystotome will cut the adhesion when passed under the iris with the tip parallel to the anterior capsule (same orientation as at entry); (3) Injection of viscoelastic under the iris will cause the surrounding iris to lift off the capsule like a parachute
   4.1.8.7 Shows stiff (wooden) pupil
   4.1.8.8 Able to cut through the pupil into the iris with Westcott scissors (sphincterotomy)
   4.1.8.9 Cutting the pupil with scissors shows realistic retraction of edges effectively enlarging the pupil
   4.1.8.10 Shows normal iris bleeding (relatively rapid, red)
   4.1.8.11 Shows iris bleeding related to rubeosis (slower ooze of slightly darker colored blood)
4.1.9 Anterior chamber features
   4.1.9.1 Shows realistic AC space of 0.25 ccs in normal eye.
   4.1.9.2 Ability to change the volume of the AC space with dependency on the position of the lens-iris diaphragm. Shows a deep AC with a volume of 0.35 ccs and a shallow AC with a volume of 0.15 ccs
   4.1.9.3 Position of the lens-iris diaphragm shows dependency on the condition of the zonules and the vitreous pressure
4.1.10 Cortex features
   4.1.10.1 Graphically realistic interaction with cystotome shows dependency on type of cortex.
   4.1.10.2 Shows 3 distinct cortical behaviors with dependency on type of cataract: (1) Mature cortical cataract shows white cortical material with strands that stand up and sometime break off when manipulated by cystotome; (2) NS and PSC cataract show clear to opaque sticky cortical material that forms persistent grooves as the cystotome tip drags through the cortex; (3) Hypermature cortical cataract shows liquefied cortex that stirs into the viscoelastic as the cystotome moves in the AC.
   4.1.10.3 Interaction with cystotome shows dependency on depth of tip in cortex. Shows the tip covered by cortex
   4.1.10.4 Shows realistic leakage of liquefied cortex into AC through capsular perforation
4.1.11 Nucleus features
   4.1.11.1 Graphically and haptically realistic interaction with cystotome shows dependency on type of cataract. Shows maximum chopping block effect in hypermature nuclear cataract. Shows movement of nuclear chip away from cystotome tip in hypermature cortical cataract (no chopping block effect). Shows standard chopping block effect in nuclear sclerotic and PSC cataracts.
   4.1.11.2 Interaction with nucleus shows dependency on where the cystotome tip contacts the nucleus. Nucleus density greatest in the center (where thickest) with less density (minimal chopping block effect) within 1 mm of the equator. Shows cystotome tips slips rather than engaging when within 1 mm of the equator
4.1.12 Vitreous features
   4.1.12.1 Shows realistic behavior of a strand of vitreous in the AC extending from under the pupil to the tunnel or to the paracentesis including realistic notch in pupil and movement of debris in vitreous thread
   4.1.12.2 Shows realistic effect of a vitreous blob ("knuckle") in the AC including pupil dilation, pupil decentration towards the tunnel, deepening of AC, and movement of debris in vitreous
   4.1.12.3 Shows vitreous adhesion to a weck sponge with dependency on contact with a dry edge of the sponge.
   4.1.12.4 Shows realistic behavior of vitreous when stretched away from the tunnel. Note that viscoelastic does not stretch,
   4.1.12.5 Shows puppet-like effect on iris when manipulating vitreous at the edge of the tunnel
   4.1.12.6 Shows retraction of cut vitreous towards sponge. Not able to see this effect on the eye side of the cut vitreous. This is used to distinguish vitreous from viscoelastic which also sticks to sponge but does not stretch out and does not retract when cut.
   4.1.12.7 Ability to increase vitreous pressure with dependency on scleral wall pressure. Shows that pressure on the scleral wall will move the lens-iris diaphragm up and down if the AC pressure is low.
   4.1.12.8 Increases vitreous pressure as a surgical condition preset. Shows that positive vitreous pressure will collapse the AC.
4.1.13 Westcott scissors features (for vitrectomy)
   4.1.13.1 Interaction between scissor and vitreous shows realistic cutting with dependency on immobilizing the vitreous by capture in the pupil
   4.1.13.2 Interaction between scissor and vitreous shows realistic cutting with dependency on immobilizing the vitreous by stabilizing it with a weck sponge
   4.1.13.3 Interaction between the scissors and the iris shows realistic cutting. The iris should not require any immobilization to produce a successful cut (iris stiffness should be sufficient hold iris in the scissor for cutting)
   4.1.13.4 Interaction between scissors and capsule shows realistic cutting with dependency on the capsule being immobilized by zonular support (such as the outer fixed rim) or by point of attachment to the outer fixed ring. Shows that the scissor will not cut a free floating edge of capsule; instead it slides out of the scissor.
   4.1.13.5 Cutting should occur at the exact point the two sides of the scissor come together.
   4.1.13.6 Realistic interaction of the scissors with the tunnel with dependency on the spread of the blades. Shows that with the scissors open, the scissor will enter the tunnel only if parallel to plane of tunnel; with blades closed can enter the tunnel with the scissor rotated.
4.1.14 Weck sponge features
   4.1.14.1 Interaction with saline and aqueous show gradual wicking and swelling of fibers
   4.1.14.2 Swelling of sponge occurs only at the edge (triangular shaped part does not change shape as water is absorbed)
   4.1.14.3 Dry parts of the sponge are stiff enough to manipulate conjunctiva and iris
   4.1.14.4 Able to feel first touch of the dry sponge on the ocular tissues and show accurate synchronization of touch with visual
   4.1.14.5 Wet parts of the sponge are flexible but still able to manipulate iris. No feeling of first touch needed.
   4.1.14.6 Dry parts of sponge will stick to vitreous but will not absorb vitreous
4.1.15 Right hand piece syringe features
   4.1.15.1 Shows Injection of viscoelastic with moderate force on plunger
   4.1.15.2 Shows injection of saline with gentle force on plunger
   4.1.15.3 Able to move the scissor blades precisely with approximately realistic spring resistance
   4.1.15.4 Able to move the McPherson forceps arms precisely with approximately realistic spring resistance (used by the right hand for IOL insertion)
4.1.16 Left hand piece grasping features
   4.1.16.1 Able to move the Colibri arms precisely with approximately realistic spring resistance
   4.1.16.2 Able to move the McPherson forceps arms precisely with approximately realistic spring resistance (used by the left hand for capsulotomy if possible)

### Step 4.2 Removal of cataract from capsular bag (using right haptic only)

4.2.1 Cystotome features
   4.2.1.1 Interactions with speculum, other tools, lid, and tunnel same as 4.1.1.1 - 4.1.1.6
   4.2.1.2 Graphically and haptically realistic interaction with the nucleus shows dependency on: the density of the nucleus; the proximity to the equator of the nucleus; and the stiffness of the zonules
   4.2.1.2 Ability to snag the nucleus shows dependency on the density of the nucleus and the force of the tip against the nucleus
   4.2.1.3 Ability to maintain the snag of the nucleus without slipping shows dependency on the density of the nucleus, the force of the tip against the nucleus, and the cortical forces preventing the nucleus from rotating (the cortical cleavage force)
4.2.2 Saline cannula (27 gauge) features
   4.2.2.1 Interaction with speculum produces metal on metal feel
   4.2.2.2 Interaction with lid margin produces metal on lid feel
   4.2.2.3 Able to feel first touch on ocular tissues and show accurate synchronization of touch with visual
   4.2.2.3 Interaction with conjunctiva, cornea, and sclera have a realistic look and feel. Shows flexibility like cystotome).
   4.2.2.4 Bending of the shaft shows dependency on the vector of force applied
   4.2.2.5 Shows recoil of shaft when bent and suddenly released. This applies to dragging over ocular tissues and interaction with other instruments including speculum. It is an especially important interaction of the 27 g cannula with the paracentesis (not the 25 g).
   4.2.2.6 Interaction of the shaft with the cornea shows dependency on the IOP. Shows realistic speculars, and accurate haptics for low, normal, and high IOP
   4.2.2.7 Positioning of cannula is accurately constrained by the shape of the tunnel
   4.2.2.8 Uplift of outer tunnel wall shows dependency on the angle of the cannula in the tunnel
   4.2.2.9 Uplift of outer tunnel wall shows dependency on the location of the cannula in the tunnel (more uplift possible at the center than at the lateral limits)
   4.2.2.10 Ability to enter tunnel shows dependency on slight downward pressure at the groove with the tip
   4.2.2.11 Ability to slide cannula through tunnel shows dependency on angle of cannula relative to inner and outer tunnel wall. Shows increasing drag in tunnel if the cannula is >20° off plane of tunnel (not the same as snagging which is a dead stop).
   4.2.2.12 Realistic interaction of the shaft with the nucleus with dependency on the shape of the nucleus. Shows graphically and haptically that the shaft follows the curve of the nucleus.
   4.2.2.13 Realistic interaction of the tip with the nucleus with dependency on the density of the nucleus. Able to bury the tip in a NS cataract nucleus with some resistance, easily bury tip in PSC nucleus, but not able to stab a hypermature nuclear cataract.
   4.2.2.14 Shows that saline flow originates exactly from the position of the tip.
   4.2.2.15 Shows that the direction of saline flow is controlled by orientation of cannula shaft (swirling debris pattern consistent with direction of stream).
   4.2.2.16 Shows that the speed of saline flow is constrained by size of the cannula opening and the pressure on plunger
4.2.3 Tunnel features
   4.2.3.1 Cystotome interaction same as 4.1.5.1 - 4.1.5.6
   4.2.3.2 27 gauge cannula interaction same as 4.1.5.1 - 4.1.5.5
   4.2.3.3 Shows leakage of fluid with dependency on type of fluid, IOP, and size of gap at inner tunnel opening. Shows that viscoelastic requires either higher IOP or larger gap to leak out.
   4.2.3.4 Shows the inner tunnel opening conforming to the shape of the nucleus with dependency on the force pushing the nucleus into the tunnel
   4.2.3.5 Shows leakage of fluid with dependency on the amount of nucleus plugging the tunnel. Shows leakage decreases by 80% when tunnel completely plugged by nucleus but leakage increases when partially plugging the tunnel.
4.2.4 Viscoelastic cannula (25 gauge) features see 3.6.1
4.2.5 Viscoelastic features see 3.6.3 - 3.6.4, 3.7.4 - 3.7.5
4.2.6 Saline features
   4.2.6.1 Saline delivery at the cannula tip shows dependency on pressure on the plunger. Shows dripping saline with slight pressure on the plunger or high velocity stream with maximum pressure on the plunger.
   4.2.6.2 Shows that the volume of saline delivery in cc's (cubic centimeters) evenly fills AC space to a total of approximately 0.25 ccs
   4.2.6.3 Shows normal liquid behavior of saline in the AC.
   4.2.6.4 Shows realistic interaction of saline with viscoelastics including swirling and bulk displacement of viscoelastic.
   4.2.6.5 Shows saline displacement of the nucleus out of the capsular bag and into the tunnel with dependency on the fluid pressure of the saline behind the nucleus
   4.2.6.6 Shows saline cleavage of the cortex from the nucleus (golden ring sign) with dependency on the proper placement of the cannula tip adjacent to the nucleus.
4.2.7 Capsular features
   4.2.7.1 Anterior capsular features same as 4.1.6.1
   4.2.7.2 Realistic interaction of capsular bag with the nucleus shows dependency on size and orientation of the nucleus
   4.2.7.3 Ability to laterally displace the capsular bag with dependency on the zonular support. Able to show semilunar deformation of equatorial capsule (which shows red reflex) in 1 or 2 quadrants of weak zonules.
   4.2.7.4 Ability to laterally displace the whole capsular bag with dependency on the radial force applied against the zonules. Able to expose the equatorial lens capsule (which shows the red reflex) with sufficient radial force against the zonules.
   4.2.7.5 Realistic interaction of the fixed capsular rim with the nucleus with dependency on which surface of the lens contacts the rim. Shows that the top surface of the nucleus cannot pass through the capsulotomy (diameter of the capsulotomy opening is smaller than the diameter of the nucleus). Shows that contact with the equator of the nucleus causes upward folding of the rim (like a taco) by the force of the nucleus equator against the rim from below. Shows that folding of the rim in one axis allows the diameter to expand only in the axis of the fold. The resulting increase in diameter along this axis allows the nucleus to pass out of the bag even though it is a larger diameter than the anterior capsular opening.
   4.2.7.6 Realistic interaction of the fixed capsular rim with the cannula with dependency on force of the cannula against the rim and the position of the nucleus equator. Shows that downward folding of the rim behind the equator has the same effect as upward folding of the rim (4.2.7.5).
   4.2.7.7 Shows the limit of the increase in diameter of the rim along the axis of folding is constrained by the size of the nucleus. Shows that the hypermature nucleus requires a larger capsulotomy not just proper execution of the tire tool maneuver. Diameter of the capsulotomy must be within 1.5 mm mm or less of the diameter of the nucleus.
   4.2.7.8 Shows that relaxing cuts in the rim increase the effective diameter equal to the length of the cuts.
   4.2.7.9 Realistic interaction of posterior capsule with instruments
      4.2.7.9.1 Blunt pressure downward against clear capsule produces dimple with ring of light (27 gauge cannula)
      4.2.7.9.2 Blunt pressure tangentially against the posterior capsule produces a wrinkle (25 and 27 gauge cannulas only)
      4.2.7.9.3 Sharp contact (cystotome tip) with minimal force produces a round hole in the posterior capsule
      4.2.7.9.4 Excessive blunt force against the posterior capsule produces a round hole in posterior capsule
      4.2.7.9.5 Excessive force against a radial tear in the fixed capsular rim produces a linear tear that extends to the posterior capsule
   4.2.7.10 Shows a posterior sub capsular cataract can be aspirated off the posterior capsule
   4.2.7.11 Shows posterior capsule fibrosis which cannot be aspirated off the posterior capsule
4.2.8 Zonular support features
   4.2.8.1 Graphically and haptically realistic interactions of zonules with lens same as 4.1.7.1 - 4.1.7.5
   4.2.8.2 Changes the volume of the AC with dependency on the position of the lens-iris diaphragm. Shows that loose zonules allow the lens-iris diaphragm to fall back, increasing the AC volume to approximately 0.35 ccs.
4.2.9 Iris features
   4.2.9.1 Interaction with cannula, vitreous, and viscoelastic same as 4.1.8.1 - 4.1.8.5
   4.2.9.2 Graphically realistic interaction of iris with nucleus including expansion of pupil as nucleus passes through
   4.2.9.3 Shows iris prolapse with dependency on IOP, premature tunnel entry, or excessive pressure on inner tunnel wall.
   4.2.9.4 Constrains the passage of the nucleus into the AC with dependency on the stiffness of the pupil. Shows a stiff pupil will not allow a nucleus to pass into AC.
   4.2.9.5 Shows a sphincterotomy same as 4.1.8.8 - 4.1.8.9
   4.2.9.6 Iris bleeding same as 4.1.8.10 - 4.1.8.11
4.2.10 AC features see 4.1.9
4.2.11 Cortex features
   4.2.11.1 Interaction with cystotome same as 4.1.10.1
   4.2.11.2 Interaction with 27 gauge cannula shows dependency on depth of tip in cortex. Shows the cannula tip buried in the cortex.
   4.2.11.3 Three types of cortical features same as 4.1.10.2
   4.2.11.4 Shows realistic interactions with nucleus with dependency on the type of cortex
   4.2.11.5 Shows shearing between selected layers of cortex.
   4.2.11.6 Shows fluid wave separating the cortical layers during hydrodissection with dependency on position of cannula during saline injection. Cannula tip must be peripheral enough, under the fixed anterior capsular edge, and directed towards the equator for fluid wave to occur.
   4.2.11.7 Shows that fluid wave can be stopped from separating cortical layers if there is downward force on the nucleus by the cannula. This force compresses the nucleus against the posterior layers of cortex and prevents fluid wave spread.
   4.2.11.8 Shows cortical debris as layers of highly viscous debris
   4.2.11.9 Shows cortical debris as discrete, non-viscous clumps
4.2.12 Nucleus features
   4.2.12.1 Interaction with cystotome same as 4.1.11.1 - 4.1.11.2
   4.2.12.2 Resistance to rotation within the cortical shell shows dependency on the type of cataract. In the hypermature cortical cataract there is no resistance to nucleus rotation. In the PSC cataract the density of the nucleus is insufficient to engage the cystotome tip to rotate the nucleus (requires hydrodissection). In the nuclear sclerotic (standard) cataract the resistance to rotation is the highest but the density of the nucleus is sufficient to engage the nucleus to overcome the resistance. In the mature cortical cataract the resistance to rotation is in between the hypermature cortical and the NS cataract
4.2.13 Vitreous features see 4.1.12.1 - 4.1.12.8
4.2.14 Right hand piece features
   4.2.14.1 Movement of fluid out the tip shows dependency on fluid type. Viscoelastic requires more force on plunger than saline to start flow.
   4.2.14.2 Shows that the speed of saline delivery is controlled by amount of pressure on the plunger with dependency on size of opening
4.2.15 Moves seamlessly from Step 4.1 to Step 4.2 without removing the cystotome
4.2.16 Moves seamlessly through all the actions of the cystotome
4.2.17 Moves seamlessly through all the actions of the 27 g. cannula
4.2.18 Shows realistic combined effect of speed, force, and direction of movement of the cystotome based on comparison with the Sierra Leone data

### Step 4.3 Removal of cataract from the anterior chamber (using left and right haptic)

4.3.1 Lens loop features (right hand)
   4.3.1.1 Interaction with speculum and Colibri produces metal on metal feel
   4.3.1.2 Interaction with lid margin produces metal to lid feel
   4.3.1.3 Able to feel first touch at the scleral groove and show accurate synchronization of touch with visual
   4.3.1.4 Bimanual interaction with Colibri shows that the IOP is dependent on the force of each instrument
   4.3.1.5 Realistic interaction with tunnel shows dependency on the curved shape of the long axis of the lens loop profile. Shows that the angle of insertion in the tunnel requires following the curve of the lens loop to avoid leakage.
   4.3.1.6 Realistic interaction with tunnel shows dependency on the short axis of the lens loop profile. Shows that orientation of the short axis of the lens loop is constrained by plane of the tunnel.
   4.3.1.7 Realistic interaction with the nucleus shows dependency on the shape of the lens loop profile and the shape of the nucleus. Shows that to slide under the nucleus, the lens loop must follow the curve of the nucleus.
   4.3.1.8 Shows that optimal alignment of long axis of lens loop for nucleus delivery is perpendicular to the inner opening of the tunnel. This orientation produces the least resistance.
   4.3.1.9 Realistic interaction with the cortex or epinucleus. Shows that non-nucleus portions of cataract can be moved but not dragged by the loop.
   4.3.1.10 Shows realistic combined effect of speed, force, orientation (long and short axis), and IOP during nucleus delivery based on comparison with the Sierra Leone data
4.3.2 Colibri features (left hand)
   4.3.2.1 Basic features same as 3.1.1
   4.3.2.2 Interaction with conjunctiva same as 3.5.1 but with left hand
   4.3.2.3 Shows that increasing pressure against limbus during nucleus delivery increases the force driving the nucleus through the tunnel.
   4.3.2.4 Shows optimal orientation for counterforce during delivery is in alignment with long axis of lens loop
4.3.3 Conjunctival features
   4.3.3.1 Shows leash effect of conjunctiva to the limbus used to stabilize the globe
   4.3.3.2 Shows tearing of conjunctiva when stretching force exceeds ability of tissue to stretch.
4.3.4 Tunnel features
   4.3.4.1 Uplift of outer tunnel wall shows dependency on the angle of the long axis of the lens loop in the tunnel
   4.3.4.2 Uplift of outer tunnel wall shows dependency on the orientation of the short axis of the lens loop in the tunnel
   4.3.4.3 Uplift of outer tunnel wall shows dependency on the location of the lens loop in the tunnel
   4.3.4.4 Shows outer tunnel wall conforms to the shape of the nucleus as it passes through.
   4.3.4.5 Ability of the nucleus to pass through the tunnel shows dependency on the lens loop pressure against the inner tunnel wall and the IOP. Intentionally deforming the inner tunnel wall with the lens loop creates the space for the passage of the nucleus; IOP drives it through.
   4.3.4.6 Ability of the nucleus to pass through the tunnel shows dependency on alignment of the long axis of lens loop with the inner opening of the tunnel. Shows that the most efficient orientation of lens loop is perpendicular to inner tunnel opening and in the center of the tunnel.
   4.3.4.7 Shows that the tunnel constrains the passing of the nucleus if the circumference of the inner opening boundary (approximately twice the length) is the same or longer than the transverse circumference of the lens at its thickest point (the fish mouth effect of the tunnel). Shows that a thick nucleus or a large diameter nucleus both require a large inner tunnel opening.
   4.3.4.8 Shows that spreading of the tunnel walls creates potential energy in the inner and outer walls as the lateral limits are pulled together (the squeeze purse effect). This energy slowly pushes the nucleus out once the midline of the nucleus is at the scleral groove and the lateral limits are returning to normal positions.
   4.3.4.9 Basic functionality of tunnel same as 4.1.5.4 - 4.1.5.5 and 4.2.3.3 - 4.2.3.5
4.3.5 Cortex features
   4.3.5.1 Shows three types of cortex same as 4.1.10.2
   4.3.5.2 Cortex behavior same as 4.2.10.4 and 4.2.10.7 - 4.2.10.8. Shows these behaviors as: (1) Cortex left behind attached to the capsular bag in sheets, (2) Cortex left behind floating in AC, (3) Cortex still attached to the nucleus
   4.3.5.3 Realistic interaction of the cortex with the tunnel shows: (1) Ability of cortex still attached to the nucleus to deform to fit the tunnel resulting in delivery of slightly oblong cataract; (2) Stripping cortex off the nucleus leaving debris in the AC with dependency on the tightness of the fit through the tunnel.
4.3.6 Epinucleus features
   4.3.6.1 Shows that epinuclear material behaves like dense cortical material
   4.3.6.2 Realistic interaction of epinuclear material with tunnel same as 4.3.5.3
   4.3.6.3 Able to deliver epinuclear material separately from nucleus
4.3.7 Nucleus features
   4.3.7.1 Shows a realistic look and feel of nucleus interaction with tunnel with dependency on the size of the nucleus
   4.3.7.2 Shows realistic interaction with the lens loop with dependency on the cradling of the nucleus for delivery (lens loop position 4 in the tech manual)
4.3.8 Iris features
   4.3.8.1 Realistic interaction with vitreous, nucleus, and viscoelastic same as 4.1.8 and 4.2.9
   4.3.8.2 Graphically realistic interaction of iris with lens loop including movement from direct contact and ability to capture iris between nucleus and the lens loop
   4.3.8.3 Shows iris prolapse with dependency on iris capture between the nucleus and lens loop during delivery
   4.3.8.4 Iris bleeding same as 4.1.8.10 - 4.1.8.11
4.3.9 Vitreous features see 4.1.12.1 - 4.1.12.8
4.3.10 Moves smoothly from Step 4.1 to Step 4.3 using only the required instrument changes
4.3.11 Shows realistic combined effect of lens loop speed and orientation, and Colibri stabilization and IOP based on comparison with the Sierra Leone data

### Supplementary Step 4.4 the multitear and linear capsulotomy

The features below are specific for addressing the hyper mature cortical and PSC cataract capsulotomy.
4.4.1 Long angled McPherson forceps features (use left haptic held in right hand to achieve proper grasping effect)
   4.4.1.1 Ability to grasp the cut free edge of anterior capsule shows dependency on forceps contact on both sides of the capsular flap either simultaneously or one before the other. Note that use of the McPherson will always require a cystotome cut to create a free edge to be grasped by the McPherson.
   4.4.1.2 Ability to maintain the grasp on the free edge of the capsule shows dependency on keeping the arms of the forceps closed.
   4.4.1.3 Ability to maintain the grasp on the free edge of the capsule shows dependency on force applied to the capsule.
   4.4.1.4 The movement of the free capsular edge is controlled by the movement of the tip of the McPherson at the exact point where it grasps the capsule.
   4.4.1.5 Ability to tear the free capsular edge shows same features as in 4.1.1.9, and 4.1.1.16 - 4.1.1.21
   4.4.1.6 As the point of grasping moves farther from the point of tearing, the linear component of the tear (in line with the direction of tearing) becomes more difficult to maintain due to space constraints in the AC.
   4.4.1.7 Shows that regrasping near the point of tearing is required to keep the direction of the tear in the desired direction.
   4.4.1.8 Shows run out of tear from misdirection of forceps or failure to regrasp. 4.4.1.9 Able to correct run out by adjusting vector of force on the free capsular edge at the grasping point. Produces the feeling of being able to "lead" the tear back on track.
   4.4.1.10 Able to convert to a multicut capsulotomy using the cystotome if run out occurs.
4.4.2 Westcott scissors
   4.4.2.1 Interaction between scissors and capsule shows realistic cutting with dependency on the capsule being immobilized at each end of the linear capsulotomy.
   4.4.2.2 The tunnel walls constrain the opening of the scissors when opened perpendicular to the tunnel plane (visual effect only since hand piece will not have active simulated feel).
4.4.3 Anterior capsule features
   4.4.3.1 Curvilinear tears of the capsule produce a convex edge and a concave edge. Shows the convex edge is free due to lack of stiffness and the concave edge is fixed due to inelasticity.
   4.4.3.2 Shows that a radial curvilinear tear from the center outward produces a free edge that can be folded over on itself as the starting point for the circumferential curvilinear tear (J stroke effect).
   4.4.3.3 Shows that a tear will not propagate through a curve. It will propagate only at the point of a cut.
   4.4.3.4 Shows the smooth propagation of a circular tear with dependency on the curve of the surface being torn.
   4.4.3.5 Shows increased risk for run out when the anterior capsule surface is convex and reduced risk when it is concave.
   4.4.3.6 Shows the smooth propagation of a circular tear with dependency on the centripetal vector inwards and away from the fixed edge.
   4.4.3.7 Shows the gapping of the linear capsulotomy allows leakage of liquid cortex and insertion of scissor blade
4.4.4 Moves smoothly from step 4.4 (alternate capsulotomy) to 4.2 (nucleus dislocation) using only the normal instrument changes.

### Supplementary Step 4.5 the sphincterotomy

The features below are specific for addressing the wooden pupil and posterior synechia.
4.5.1 Westcott scissors
   4.5.1.1 Interaction between the scissors and the iris shows realistic cutting. The iris should not require any immobilization to produce a successful cut (iris stiffness should be sufficient to hold iris in the scissor for cutting)
   4.5.1.2 Able to drag the pupil towards the tunnel without cutting the pupil using the partially closed scissor blades
   4.5.1.3 Able to cut the iris after dragging it into position
4.5.2 Iris features (same as 4.1.8.6.1 - 4.1.8.6.3) plus
   4.5.2.1 Shows stiff (wooden) pupil. Shows pupil is unresponsive to viscoelastic filling. Shows pupil is unresponsive to stretching with cystotome tip. Shows pigment debris resulting from interaction with attempted dilation with cystotome.
   4.5.2.2 Cutting the pupil with scissors shows realistic reaction. Cutting with scissor shows dependency on the amount of iris tissue between the scissor blades. During cutting, the iris retracts away from the scissor blades like they were stretched slightly. The amount of stretching shows dependency on the length of each cut of the iris. The amount of dilation shows dependency on the amount of stretching.
   4.5.2.3 Cut iris shows realistic behavior. Floppy iris is easily engaged by the aspiration port during I&A. Floppy edge of iris prolapses out of tunnel at lower IOP and with less proximity to the inner tunnel opening or premature entry site. Cut pupil reacts normally to viscoelastic displacement for dilation. Pupil dilation constrained by unlysed posterior synechia.
4.5.3 Fibrotic anterior capsule features (commonly associated with wooden pupil)
   4.5.3.1 Shows increased localized stiffness of capsule
   4.5.3.2 Shows adhesion between iris and anterior capsule associated with increased localized capsular stiffness
   4.5.3.3 Shows constraint on pupil dilation over areas of fibrotic capsule
4.5.4 Moves smoothly from Step 4.5 (sphincterotomy) to Step 4.1 (capsulotomy) and continue with procedure using only the required instrument changes

### Supplementary Step 4.6 Intracapsular cataract extraction (ICCE)

The features below are specific for addressing the zonular failure in more than 2 quadrants resulting in dislocated lens. This step assumes the cortex and nucleus are still in the intact capsular bag unless otherwise specified.
4.6.1 25 Gauge cannula features (right hand viscoelastic syringe)
   4.6.1.1 Ability to enter paracentesis shows dependency on slight downward pressure on the lower lip at the corneal entry site with the tip
   4.6.1.2 Ability to slide cannula through the paracentesis with dependency on angle of cannula relative to the upper and lower lip of the paracentesis.
   4.6.1.3 Shows increasing drag in paracentesis if the cannula is >20° off plane of entry (not the same as snagging which is a dead stop).
   4.6.1.4 Positioning of cannula is accurately constrained by the shape of the paracentesis
   4.6.1.5 Realistic interaction of the shaft with the cataract. Shows graphically and haptically that the shaft touches and follows the curve of the cataract precisely. Able to feel the slick smoothness of the capsular bag during interaction of the cannula with the cataract. Shows that the cataract will slide off the cannula if cannula not positioned centrally behind the cataract.
4.6.2 Lens loop features
   4.6.2.1 Realistic interaction with the cataract shows dependency on the shape of the lens loop profile and the shape of the cataract. Shows that to slide under the nucleus, the lens loop must follow the curve of the nucleus.
   4.6.2.2 Realistic interaction with the cataract shows dependency on the position of the cataract. Able to accurately show position of lens loop to slide under cataract is dependent on which way the cataract has tilted
   4.6.2.3 Shows that the intact zonules block the passing of the lens loop under the cataract
4.6.3 Zonular features
   4.6.3.1 Shows that the lens always tilts away from the supported quadrant.
   4.6.3.2 Shows hinge effect of zonules during interaction with lens loop and cannula
   4.6.3.3 Shows release of residual zonules with dependency on force applied to the cataract by the lens loop or cannula
4.6.4 Capsular bag features
   4.6.4.1 Ability to deform the capsular bag during delivery through the tunnel with dependency on the type of cataract. This is only needed for hyper mature cortical and hyper mature nuclear cataract types.
   4.6.4.2 Shows capsular bag rupture like a balloon bursting with dependency on excessive force
4.6.5 Cataract features with capsular bag intact
   4.6.5.1 Shows that the cataract tilts realistically with dependency on: (1) Patient age >70 having liquefied vitreous which allows more tilting; (2) Amount of viscoelastic injected under the cataract causing the cataract to float up; (3) The location of the residual zonules acting as a hinge in any one of the four quadrants.
   4.6.5.2 Shows that the cataract drops realistically with dependency on: (1) Trainer programmed event releasing the residual zonules; (2) Direction the cataract is tilting prior to dropping; (3) Patient age >70 having liquefied vitreous which allows faster dropping; (4) Amount of viscoelastic injected under the cataract slowing the drop.
   4.6.5.3 Shows that the cataract will flip over realistically with dependency on: (1) The amount of lifting force applied by the cannula; (2) The location of the hinge; (3) The use of bimanual technique to avoid dropping the nucleus. Shows flipping the cataract over onto the lens loop with the cannula.
   4.6.5.4 Shows that the capsular bag will rupture during delivery with dependency on: (1) Excessive force applied to the cataract in the tunnel; (2) Puncture due to contact with tip of cannula; (3) Presence of hyper mature cortical cataract.
4.6.6 Cataract features with capsular bag open
   4.6.6.1 Shows loss of capsular bag volume proportional to loss of intracapsular contents
   4.6.6.2 Shows debris from loss of cortical containment with dependency on type of cataract. Liquefied cortical material quickly leaks out and mixes with surrounding fluids. Formed cortex forms lumps around the lens
4.6.7 Vitreous features
   4.6.7.1 Shows realistic effect of a vitreous following the cataract out of the tunnel
   4.6.7.2 Shows vitreous support of the tilting nucleus. Tapping on the scleral wall will cause the cataract to bob up and down
4.6.8 Moves smoothly from Step 4.1 (identifying dislocated cataract) to Step 4.5 (ICCE) skipping step 4.2 and 4.3 using only the required instrument changes

### Supplementary Step 4.7 Removal of anterior or posterior capsular fibrosis

The features below are specific for addressing the problem of a thickened and stiff capsule requiring removal to complete the surgery. This may involve either the anterior or posterior capsule with similar features.
4.7.1 Cystotome features
   4.7.1.1 Shows the tip of the cystotome pierces, cuts, and tears the normal capsule adjacent to the fibrotic capsule (anterior or posterior)
   4.7.1.2 Shows the tip will not cut or pierce the fibrotic plaque (anterior or posterior)
4.7.2 IOL forceps features (long angled McPhersons)
   4.7.2.1 Able to grasp the fibrotic plaque when uncut but cannot tear it
   4.7.2.2 Able to tear the capsule at junction of normal capsule with fibrotic capsule but only after starting the tear by cutting an edge with the cystotome
4.7.3 Westcott scissor features
   4.7.3.1 Shows the scissors will cut the fibrotic capsule.
4.7.4 Capsular bag features
   4.7.4.1 Realistic training features of anterior capsular fibrosis includes. Shows the fibrotic plaque is part of the anterior capsule, not part of the cataract. Shows the fibrotic plaque is semitransparent. Shows fibrosis as an irregular plaque covering an area from the center to under the edge of the pupil. Shows plaque is stiff enough that it will not wad up or fold easily. Shows the plaque will not stay folded over unless held in position. Shows association with poorly dilated pupil. May be associated with any type of cataract.
   4.7.4.2 Realistic training features of posterior capsular fibrosis includes. Shows the plaque is part of the posterior capsule, not part of the cataract. Shows the plaque is completely opaque. Plaque is central and round with slightly irregular edges. Plaque is 50% thicker and stiffer than anterior capsule fibrosis. Plaque will not fold. Associated with PSC and hyper mature NS cataracts only

### Section 5: Cortex Removal and IOL Implantation Training Features Milestone Definition and Evaluation Criteria

The simulator is judged ready for training (RFT) based on the realism and training features of all the steps of the MSICS cortex removal and implantation of both PC and AC IOL (detailed below) in an anatomically correct model. RFT criteria will also include evaluation of the following variations and complications:
1. Able to perform standard cortex removal with normal variations in cortex (based on type of cataract), capsulotomy size and shape, pupil size and shape, and AC depth
2. Able to perform standard viscoelastic filling of capsular bag with normal variations in capsular bag and AC depth
3. Able to perform standard PC IOL insertion with normal variations in capsulotomy size and shape, pupil size and shape, and AC depth
4. Shows and manage inaccuracies in use of Simcoe including iris aspiration, posterior capsular bag aspiration, capsular bag rupture, loss of AC, constriction of the pupil from iris contact, iris prolapse, and failure to remove cortex
5. Shows and manage inaccuracies in viscoelastic filling including over-filling, incomplete filling, collapsing anterior capsule onto posterior capsule, excessively deepening AC, and failure to deepen AC
6. Shows and manage inaccuracies in PC IOL insertion including failure to insert leading haptic in the bag, failure to insert trailing haptic in the bag, and IOL haptic bending or breaking before inserting
7. Shows and manage PC rupture with and without vitreous loss including scissors vitrectomy and reforming remnant of capsular bag or anterior capsular shelf with viscoelastic
8. Shows and manage variations in zonular support including loss of 1, 2, or 3 quadrants of zonular support and dislocation of cataract
9. Able to insert anterior chamber IOL
10. Shows and manage advanced capsulotomy challenges including incomplete capsulotomy, hinged capsulotomy, small capsulotomy, fibrous capsule, and capsular tags
11. Shows and manage advanced iris challenges including iridodialysis following nucleus delivery, prolapsed iris with vitreous loss, and iris bleeding
12. Shows and manage advanced PC IOL challenges including sulcus fixation causing decentration, haptic bending or breakage in the AC resulting in need to explant the IOL, IOL damage to zonules resulting in tilted IOL and need for sulcus fixation
13. Manages increased posterior pressure with AC loss and high IOP

The steps of Section 5 will be integrated into a continuous scenario. Complications may be represented in some cases as an error message or as a visual representation of the complication. Alternatively, there will be an ability to repair or correct complications.

### Step 5.1 Cortex removal and preparation of capsular bag

5.1.1 Simcoe features (right hand)
   5.1.1.1 Shows realistic interaction of curved, rigid cannula with tunnel with dependency on position and curvature.
   5.1.1.2 Shows realistic metal on metal feel from contact with speculum
   5.1.1.3 Shows realistic feel of contact with upper lid
   5.1.1.4 Shows realistic flow of aspiration fluids perpendicular to the plane of the aspiration port (Perpendicular to cannula since port is on the side of the cannula)
   5.1.1.5 Shows realistic flow of irrigation fluid perpendicular to the plane of the irrigation port (parallel to the cannula since port is on the end of the cannula)
   5.1.1.6 Shows that flow at the aspiration port is dependent on direction and force applied to aspiration syringe plunger (the left hand device).
   5.1.1.7 Shows that flow at the aspiration port is constrained by the size of the opening.
   5.1.1.8 Shows that if tissue material (cortex, capsule, or iris) completely blocks the opening during aspiration, flow stops and vacuum builds.
   5.1.1.9 Shows that if there is viscoelastic material blocking the opening during aspiration, flow slows, pressure builds, but flow does not stop
   5.1.1.10 Shows that following aspiration of cortex, forceful irrigation ejects cortex from the port as a burst of cortical debris.
   5.1.1.11 Shows that flow from the aspiration port during irrigation is directed perpendicular to the opening.
   5.1.1.12 Shows that flow from the aspiration port can create a fluid wave that moves cortical debris, capsular flaps and tags, and iris
   5.1.1.13 Shows that flow at the irrigation port is dependent on the "height" of the virtual irrigation bottle
   5.1.1.14 Shows that flow at the irrigation port can be stopped by covering the port with cortex or iris
   5.1.1.15 Shows free flow of fluid from the irrigation port when outside the eye with dependency on the height of the virtual irrigation bottle.
   5.1.1.16 Shows realistic mechanical interaction of the cannula with iris.
   5.1.1.17 Shows that the Simcoe aspiration port can grab and manipulate capsular tags similar to how forceps manipulate tags but with dependency on aspiration force.
   5.1.1.18 Shows that the Simcoe aspiration port can grab and manipulate iris similar to how forceps manipulate iris but with dependency on aspiration force.
5.1.2 Cortex features
   5.1.2.1 Shows three types of residual cortex with dependency on degree of cohesiveness and viscosity:
      Type 1: high viscosity, high cohesiveness, typical in NSC and PSC cataract. Tends to peel off the capsular bag in large sheets and wide strands. The large sheets and strands tend to break off at the aspiration port because the mass of cortex is too great for the cohesiveness of the cortex to hold together. Smaller strands hold together for dragging.
      Type 2: high viscosity, low cohesiveness, typical in mature cortical and hypermature NSC. Tends to break into thin strands and clumps during removal. Requires more aspiration force to engage in aspiration port. Often removed or washed out in many pieces.
      Type 3: low viscosity, low cohesiveness (liquid), typical of the hypermature cortical cataract. Easily mixes in with liquids and viscoelastic in AC. Flows and aspirates like a liquid particulate suspension.
   5.1.2.2 Graphically realistic interaction of cortex with Simcoe shows dependency on type of cortex.
      5.1.2.2.1 NS and PSC cataract show residual clear to opaque, type 1 cortical material that sticks in the aspiration port with dependency on full contact across the aspiration port and the amount of vacuum applied to left hand piece.
      5.1.2.2.2 Mature cortical cataract shows residual white, type 2 cortical material that is removed in strands and chunks. Less sticky and slightly stiffer than NS and PSC residual cortex. Tends to jump into aspiration port when in close proximity. Contact with the cortex is not necessary. May have to wash more of this cortex out than cortex associated with NS or PSC cataract.
      5.1.2.2.3 Hypermature cortical cataract shows residual white type 3 cortex that leaks out of capsular bag proportional to the size hole. Easily aspirates into the aspiration port mixed with viscoelastic and/or saline.
   5.1.2.3 Graphically realistic interaction of cortex with capsular bag shows dependency on type of cortex
      5.1.2.3.1 NS and PSC cataract show residual clear to opaque type 1 cortical material that sticks to capsule as a sheet. Shows that shearing force for removal of cortex is high.
      5.1.2.3.2 Mature cortical cataract shows residual white type 2 cortical material that is removed in strands and chunks. Shows it sticks less tightly to the capsular bag and that the shearing force for removal is less than type 1 cortex.
      5.1.2.3.3 Hypermature cortical cataract shows residual white type 3 cortex that leaks out of capsular bag proportional to the size of the capsular hole. No stickiness but should be able to see the flow constrained by the hole and the viscoelastic in the AC.
5.1.3 Capsular bag features (See anterior capsular features 4.1.6.1, 4.1.6.16, 4.1.6.18 - 19)
   5.1.3.1 Realistic interaction of posterior capsule with Simcoe (similar to 4.2.7.9 cannula interactions). Shows that blunt pressure downward against clear capsule produces dimple with ring of light. Shows that blunt pressure tangentially against the posterior capsule produces a wrinkle. Shows that minimal force against the capsule with the Simcoe tip produces a round hole. Shows that contact of the posterior capsule with the aspiration port produces wrinkles in the capsule directed towards the port with dependency on the aspiration force applied. Shows that movement of the tip when wrinkles are present to the aspiration port results in posterior capsule hole
   5.1.3.2 Shows realistic behavior of the capsule as a bag. Shows that viscoelastic can realistically fill the bag. Shows that flow from the Simcoe can realistically fill the bag. Shows that the bag lined with cortex is stiffer than the bag without cortex. Shows that filling the bag exposes the residual cortex for removal by creating a smooth, stable surface. Filling the capsular bag essentially stretches the bag open and provides counter-force for shearing the cortex off the capsular surface. (See also 4.2.7.10 - 4.2.7.11).
5.1.4 Tunnel features (similar to cystotome features 4.1.5.1 - 4.1.5.5)
   5.1.4.1 Uplift of outer tunnel wall shows dependency on the tilting angle and curve of the Simcoe in the tunnel.
   5.1.4.2 Outer tunnel wall constrains tilting angle of Simcoe with dependency on the proximity to the lateral tunnel limits. 15° tilt possible in central tunnel; 5° tilt possible at lateral limits.
   5.1.4.3 Uplift of outer tunnel wall shows dependency on the location of the Simcoe in the tunnel.
   5.1.4.4 The inner tunnel opening is controlled by the force applied against the inner tunnel wall; the greater the force, the greater the gap in the inner tunnel opening.
   5.1.4.5 Inward gaping of the inner tunnel wall is constrained by contact with the lens iris diaphragm
   5.1.4.6 Shows that the Simcoe rotated 90° to the side creates twice the gap as the Simcoe in the upright position due to the oval profile (double cannula side by side)
5.1.5 AC features same as 4.1.9.1 - 4.1.9.3
   5.1.5.1 Shows responsiveness of AC volume to combined flow from aspiration and irrigation ports.
   5.1.5.2 Shows effect of combined volume of empty capsular bag plus the AC volume. Shows that capsular bag fills before shift in iris diaphragm. Shows that capsular bag fills first, then AC.
5.1.6 Saline flow features (See features 4.2.6.1 - 4.2.6.4)
   5.1.6.1 Shows free floating cortical material as swirling debris with dependency on saline flow
   5.1.6.2 Shows saline is able to displace viscoelastic
5.1.7 Iris features (see features 4.1.8.1 - 4.1.8.5, 4.1.8.10 - 4.1.8.11)
   5.1.7.1 Shows realistic V shaped deformation of the pupil edge caused by contact with Simcoe cannula shaft
   5.1.7.2 Shows iris is more floppy when not supported by presence of cataract.
5.1.8 Vitreous features same as 4.1.12
5.1.9 Irrigation/Aspiration syringe (for left hand)
   5.1.9.1 Downward force on plunger activates outflow from aspiration port proportional to amount of force on plunger
   5.1.9.2 Upward force on plunger activates aspiration at the aspiration port proportional to the amount of force on the plunger.

### Step 5.2 Implantation of PC IOL

5.2.1 PC IOL features
   5.2.1.1 Shows realistic, rigid, plastic on metal interaction with lid speculum
   5.2.1.2 Shows realistic, rigid, plastic on metal interaction with McPhersons including edge and central thickness and metal on plastic sliding
   5.2.1.3 Shows realistic compression and recoil of the IOL haptic "springs"
   5.2.1.4 Shows realistic breaking point for IOL haptic
   5.2.1.5 Shows realistic specular reflection off IOL larger than corneal specular because IOL is flatter than cornea
   5.2.1.6 Shows realistic behavior of IOL on the surface of the eye (if dropped from the forceps). IOL will slide freely on the tear film. IOL will slide off the cornea like sliding down a hill.
   5.2.1.7 Abe to show realistic behavior of IOL in the tunnel including: (1) Compression of haptic from tunnel resistance; (2) Deformation of outer tunnel in proportion to lifting of IOL; (3) Snagging of IOL haptic on inner tunnel limit if not angled at least parallel to iris.
   5.2.1.8 Shows realistic behavior of IOL in posterior chamber including: (1) Compression of haptic by iris less than tunnel (force partially absorbed by iris); (2) Compression of haptic by cut edge of anterior capsule about the same as iris; (3) Compression of haptic by posterior capsule less than iris (force mostly absorbed by posterior capsule); (4) Shows the iris or the anterior capsular edge can guide the IOL into the posterior chamber during rotation (dialing) of IOL.
   5.2.1.9 Shows that dialing the IOL haptic constrained by the iris puts the haptic into the space between the anterior capsule and the back surface of the iris (the sulcus)
   5.2.1.10 Shows that dialing the IOL haptic constrained by the anterior capsule edge or the posterior capsule, puts the haptic into the capsular bag.
   5.2.1.11 Shows accurate positioning of the IOL optic in the posterior chamber based on placement of IOL haptics in sulcus or capsular bag.
   5.2.1.12 Shows passive expulsion of IOL from AC with dependency on IOP and inner tunnel opening.
   5.2.1.13 Shows the IOL will not passively expulse from the eye once in the capsular bag or sulcus
   5.2.1.14 Shows that the IOL will dial only counterclockwise when right side up
   5.2.1.15 Shows that the IOL will only dial clockwise when inserted upside down
5.2.2 IOL forceps features (long angled McPhersons) (See forceps features 4.1.2.1 - 4.1.2.5, 4.1.5.7)
   5.2.2.1 Shows IOL maintains position relative to arms of forceps with dependency on maintaining forceps fully closed
   5.2.2.2 Shows that IOL slips (easily rotates) in forceps when forceps opening approaches the thickness of the IOL (approximately 0.4 to 0.5 mm).
   5.2.2.3 Shows that release of the IOL when forceps are opened just past the thickness of the IOL (0.6 mm).
   5.2.2.4 Shows realistic grasping of IOL from surface of eye with dependency on lower forceps arm under IOL and at least half way across and centered on the IOL optic.
   5.2.2.5 Shows that IOL acts as a rigid extension of the forceps when correctly grasped.
5.2.3 Sinskey hook features
   5.2.3.1 Shows realistic metal on metal feel from contact with speculum
   5.2.3.2 Shows realistic feel of contact with upper lid
   5.2.3.3 Shows Sinskey hook snags edge of optic when edge is within 15° of perpendicular to hook
   5.2.3.4 Shows Sinskey hook can rotate the IOL when hook is at the haptic-optic junction and within 15° of perpendicular to edge (dialing effect).
   5.2.3.5 Shows contact of Sinskey hook shaft with the top of the IOL prevents the IOL from lifting up
   5.2.3.6 Shows that contacts of the Sinskey hook shaft with bottom of the IOL causes the IOL to lift up
   5.2.3.7 Able to realistically pass the Sinskey hook through the paracentesis
   5.2.3.8 Able to realistically manipulate the iris with the Sinskey hook
5.2.4 Colibri features
   5.2.4.1 Able to feel contact of the IOL with the Colibri
   5.2.4.2 Able to grasp the IOL haptic with the Colibri to assist in grasping with the IOL forceps after the IOL is dropped
   5.2.4.3 Able to use left arm of closed Colibri to block IOL from slipping out of tunnel
5.2.5 Capsular bag features
   5.2.5.1 Shows forming of capsular bag by viscoelastic with dependency on the amount of viscoelastic
   5.2.5.2 Shows localized filling of the capsular bag by viscoelastic with dependency on the position viscoelastic cannula
   5.2.5.3 Able to see realistic cut anterior capsular edge when the bag is 50% or more filled with viscoelastic
   5.2.5.4 Shows realistic deformation of anterior capsular edge from contact with IOL
   5.2.5.5 Shows realistic deformation of posterior capsule from contact with IOL
   5.2.5.6 Shows IOL optic decenters when IOP is < 15 mmHg (bag is loose)
   5.2.5.7 Shows that IOL optic centers when IOP is >15 mmHg (bag is stretched)
   5.2.5.8 Shows that if 1 haptic is out of the capsular bag, the optic will decenter by 0.3 mm to that side
   5.2.5.9 Shows that an IOL haptic in the sulcus will collapse the anterior capsule against the posterior capsule on the side where the IOL is in the sulcus.
5.2.6 Viscoelastic cannula (25 gauge) features see 3.6.1
   5.2.6.1 Shows realistic look and feel to contact between cannula and IOL
   5.2.6.2 Able to direct the cannula above or below the IOL
5.2.7 Viscoelastic features (See features 3.6.3 - 3.6.4, 3.7.4 - 3.7.5)
   5.2.7.1 Shows viscoelastic sticks to surface of IOL
   5.2.7.2 Shows viscoelastic leakage out of AC if IOL forceps are opened wider than 0.7 mm
   5.2.7.3 Shows saline forms a single bead on the surface of the optic
5.2.8 Tunnel features
   5.2.8.1 Uplift of outer tunnel wall shows dependency on the angle of tilt of the IOL in the tunnel.
   5.2.8.2 Uplift of outer tunnel wall shows dependency on the angle of tilt of the IOL forceps in the tunnel.
   5.2.8.3 Shows that if the IOL forceps are opened more than 0.7 mm (just enough to allow release of the IOL), the inner tunnel allows escape of viscoelastic and shallowing of AC.
   5.2.8.4 Outer tunnel wall constrains tilting angle of IOL with dependency on the proximity to the lateral tunnel limits. 45° tilt possible in central tunnel; 10° tilt possible at lateral limits.
   5.2.8.5 Uplift of outer tunnel wall shows dependency on the location of the IOL in the tunnel.
   5.2.8.6 The inner tunnel opening is controlled by the force applied against the inner tunnel wall; the greater the force, the greater the gap in the inner tunnel opening.
   5.2.8.7 Inward gaping of the inner tunnel wall is constrained by contact with the lens iris diaphragm
   5.2.8.8 Basic tunnel features for IOL forceps same as 4.1.5.1 - 4.1.5.3
   5.2.8.9 The tunnel walls constrain the opening of the forceps when opened perpendicular to the tunnel plane (visual effect only since hand piece will not have active simulated feel)
5.2.9 Iris features
   5.2.9.1 Shows realistic interaction with the IOL
   5.2.9.2 Shows realistic interaction with the Sinskey hook
5.2.10 Zonular support same as 4.1.7 but with IOL
   5.2.10.1 Shows zonular breakage with dependency on localized force from the IOL haptic. Able to show that this force can be transmitted from the Sinskey hook during forceful rotation or forceful attempt to center the IOL.
   5.2.10.2 Shows tilt of IOL from one or more quadrant of broken zonules
   5.2.10.3 Shows decentration of IOL optic away from a quadrant of loose zonules

### Supplementary Step 5.3 Management of iris prolapse

The features below are specific for addressing the problems associated with iris prolapse through the main tunnel or premature entry site.
5.3.1 25 gauge cannula features
   5.3.1.1 Cannula features same as 3.6.1
   5.3.1.2 Visually realistic interaction of the shaft of the cannula with the prolapsed iris shows a soft, spongy response of iris tissue to minimal force from the cannula
   5.3.1.3 Able to pass cannula over the IOL but under the iris
   5.3.1.4 Able to see the cannula deforming the iris from the underside.
   5.3.1.5 Shows the cannula shaft moves the iris like a rolling pin with dependency on: (1) A line of contact perpendicular to the direction of movement +/- 10°. Able to show that contact with the cannula will deform the iris on contact but will not move it if the line of contact is not perpendicular to direction of movement; (2) Proximity of the line of contact to the AC angle and the line of entrapment. Shows that the more iris there is from the line of contact, the more it stretches.
   5.3.1.6 Shows the cannula tip punctures through the iris with minimal force
5.3.2 Viscoelastic in AC features same as 3.6.3 - 3.6.4, 3.7.4
5.3.3 Iris features same as 3.2.2.13, 4.1.8.1-4.1.8.5, 4.1.8.10-4.1.8.11, 4.2.9.3, 4.3.8.3, and 5.1.7.1
   5.3.3.1 Shows that iris prolapse with vitreous loss cannot be corrected by sweeping iris and lowering IOP. Shows that the iris is essentially held in place by vitreous.
   5.3.3.2 Shows realistic interaction of prolapsed iris with all instruments, nucleus, or IOL (PC or AC). Shows that the deformation of the iris by a solid object with edges is dependent on the type of contact and the direction of movement. Able to show that flat surfaces slide over iris and sharp edges engage the iris. Shows that contact with the edge of an instrument or IOL always stretches the iris when moving away from the point of iris fixation and folds up the iris when moving towards the point of fixation. Shows that deformation of the iris by movement of the nucleus is dependent on whether or not the nucleus is on top of or below the iris. If the nucleus is on top, it always slides over the iris since the nucleus has no edges. If the nucleus is under the iris, the iris will drape over the nucleus and restrict its movement.
   5.3.3.3 Shows iris repositioning in AC with dependency on: (1) IOP. Shows low IOP facilitates repositioning; (2) Vitreous in AC. Shows that vitreous will prevent iris from repositioning; (3) Sweeping all prolapsed iris into AC. Shows that until all the prolapsed iris is swept back into the AC, it will prolapse again.
   5.3.3.4 Shows that once the iris is back in the AC, restoring normal IOP causes the lens-iris diaphragm to fall back away from the tunnel entrance.
   5.3.3.5 Shows that recurrence of prolapse is dependent on: (1) the IOP. Shows IOP greater than 40 mm causes recurrence of prolapse; (2) the size of the premature entry. Shows that a premature entry longer than 2.5 mm allows recurrence until sutured (3) the proximity to the AC angle (shows that premature entry within 1 mm of the AC angel causes recurrence until sutured); (4) recurrence of vitreous loss (shows that incomplete vitrectomy with residual vitreous in AC leads to recurrence of prolapse).
5.3.4 AC features same as 3.7.5, and 4.1.9.
5.3.5 Vitreous features same as 4.1.12.1 - 4.1.12.8

### Supplementary Step 5.4 Scissors and Weck vitrectomy

The features below are specific for addressing the hyper mature cortical and PSC cataract capsulotomy.
5.4.1 Westcott scissor features same as 4.1.13
5.4.2 Weck sponge features same as 4.1.14
5.4.3 Vitreous features same as 4.1.12.1 - 4.1.12.8
5.4.4 Tunnel features same as 4.1.5.7 - 4.1.5.9
5.4.5 Iris features same as 4.1.8.1 - 4.1.8.4, 5.3.3.2.1 - 5.3.3.2.2
   5.4.5.1 Shows constriction of pupil with Acetylcholine injection through 27 gauge cannula with dependency on: (1) Amount of Ach; (2) Injecting Ach under the edge of the pupil.
5.4.5 AC features same as 4.1.9.1 - 4.1.9.3

### Supplementary Step 5.5 Implantation of AC IOL

The features below are specific for addressing the need for implantation of the AC IOL following vitrectomy.
5.5.1 AC IOL features same general features as PC IOL 5.2.1.1 - 5.2.1.8 but with the geometry of the AC IOL.
   5.5.1.1 Shows that passing the IOL haptic across the AC is constrained by the iris and the cornea
   5.5.1.2 Shows accurate positioning of the IOL haptic feet in the AC angle is based on the feet sliding down the peripheral cornea before contact with the iris.
   5.5.1.3 Shows passive expulsion of IOL from the AC with dependency on IOP >30, only 1 haptic in the AC, and size of the inner tunnel opening >1.5 mm.
   5.5.1.4 Shows the IOL will not passively expulse from the eye once properly seated in the AC angle
   5.5.1.5 Shows the IOL haptic feet can be repositioned in the AC angle only when pulled out of the angle and only one haptic at a time. Shows that the AC IOL will not rotate like the PC IOL. Instead of rotating into position, show it must be "walked" into position.
   5.5.1.6 Able to feel realistic compression force of the haptic while walking the IOL feet into the angle.
5.5.2 IOL forceps features same as 4.1.2.1 - 4.1.2.5, 4.1.5.7, 5.2.2
5.5.3 Sinskey hook features same as 5.2.3
5.5.4 Colibri features same as 5.2.4
5.5.4 Tunnel features same as 5.2.8
5.5.5 Iris feature same as 4.1.8.1.- 4.1.8.5, 5.3.3.2
   5.5.5.1 Shows the iris is snagged by movement of the IOL feet when in contact with the iris.
   5.5.5.2 Shows that the pupil is snagged by distal haptic foot if the foot us below the plane of the iris when in the pupillary space when it crosses the pupillary edge
5.5.6 Viscoelastic in AC features same as 3.6.3 - 3.6.4, 3.7.4
5.5.7 AC features same as 4.1.9.1 - 4.1.9.3

### Section 6: Restoring Physiologic Conditions Training Features Milestone Definition and Evaluation Criteria

The simulator is judged ready for training (RFT) based on the realism and training features of all the steps used to restore physiologic conditions in an anatomically correct model. RFT criteria also includes evaluation of the following variations and complications:
1. Residual sub incisional cortex requiring 27 gauge removal through paracentesis (M7)
2. Leaking paracentesis requiring corneal hydration (M7)
3. Leaking tunnel requiring suturing (M7)

### Step 6.1 Restoring optimal internal conditions

6.1.1 Viscoelastic washout features
   6.1.1.1 Simcoe features same as 5.1.1 and 5.1.9. Able to feel contact between Simcoe and surface of IOL. Shows realistic interaction between Simcoe, IOL, and PC. Shows that tapping on the IOL dislodges residual cortical material.
   6.1.1.2 Viscoelastic features same as 3.6.3 - 3.6.4, 3.7.4 - 3.7.5. Shows viscoelastic washes out as cohesive mass with dependency on: (1) Position of Simcoe tip (shows that the tip placed near the AC angle opposite the tunnel is most effective for washout); (2) Flow of saline (shows that high flow is most effective for washout; (3) Opening of tunnel (shows that pressing on inner tunnel wall increases flow).
   6.1.1.3 AC features same as 5.1.5.1 - 5.1.5.2
   6.1.1.4 Saline flow features same as 5.1.6
   6.1.1.5 Iris features same as 5.1.7
6.1.2 Normalize IOP to test tunnel and IOL centration
   6.1.2.1 AC features same as 4.1.9.1 - 4.1.9.3, 5.1.5.1
   6.1.2.2 Weck sponge features same as 4.1.14. Shows that pressure with the dry Weck behind the scleral groove always causes the tunnel to leak. Shows that the speed of swelling of the Weck sponge is proportional to the severity of leak. Rapid leak causes rapid swelling of sponge due to high volume of fluid.
   6.1.2.3 27 gauge cannula features same as 4.2.2.1 - 4.2.2.6.
      6.1.2.3.1 Positioning of cannula is accurately constrained by the shape of the paracentesis
      6.1.2.3.2 Ability to enter paracentesis shows dependency on slight downward pressure at the opening with the tip
      6.1.2.3.3 Ability to slide cannula through paracentesis shows dependency on angle of cannula relative to the angle of the paracentesis.
      6.1.2.3.4 Shows that saline flow originates exactly from the position of the tip.
      6.1.2.3.5 Shows that the direction of saline flow is controlled by orientation of cannula shaft
      6.1.2.3.6 Able to realistically tap the surface of the IOL
      6.1.2.3.7 Able to use tip of cannula to shift the position of IOL with dependency on the amount of force applied against the IOL
      6.1.2.3.8 Able to inject saline into corneal tissue with dependency on: (1) Contact of tip with corner of paracentesis; (2) Threshold force applied by the tip into the corner (Shows that must reach a set force before able to inject into the cornea; less than this force has no effect); (3) Application of moderate force on syringe plunger (shows that this force is greater than force used for injection into the AC).
   6.1.2.4 Corneal paracentesis features same as 3.5.3. Shows infusion of saline into corners of paracentesis causes localized whitening around the tip of the cannula. Shows that successful injection of saline into cornea stops paracentesis leak.
   6.1.2.5 PC IOL features same as 5.2.1.1 - 5.2.1.4, 5.2.1.11, 5.2.5.4 - 5.2.5.9

### Step 6.2 Restoring optimal external conditions (up to removal of the speculum)

6.2.1 Inject antibiotic
   6.2.1.1 Corneal paracentesis features same as 6.1.2.4
   6.2.1.2 27 gauge cannula features same as 6.1.2.3
6.2.2 Close conjunctiva
   6.2.2.1 Weck sponge features same as 6.1.2.2
   6.2.2.2 Colibri features same as 3.1.1
   6.2.2.3 Conjunctiva features same as 4.3.3

**Supplementary Step 6.3 Removal of sub incisional cortex through the paracentesis**

The features below are specific for addressing the problem of retained sub incisional cortex and the use of a cannula for removal.
6.3.1 27 gauge cannula features same as 6.1.2.3
   6.3.1.1 Shows that flow at the cannula tip is constrained by the size of the tip opening.
   6.3.1.2 Shows that if tissue material (cortex, capsule, or iris) completely blocks the opening during aspiration, flow stops and vacuum builds.
   6.3.1.3 Shows that if there is viscoelastic material blocking the opening during aspiration, flow slows, pressure builds, but flow does not stop
   6.3.1.4 Shows aspiration of cortex into the cannula tip with dependency on: (1) Tip contact with cortex. Shows that cortex must be in contact with cortical material to successfully engage it; (2) Activation of the aspiration plunger action.
   6.3.1.5 Shows that following aspiration of cortex, forceful irrigation ejects cortex from the port as a burst of cortical debris.
   6.3.1.6 Able to drag cortex out of capsular bag with dependency on: (1) Engagement of cortex in the cannula tip (6.3.1.4); (2) Speed of dragging (shows that slow steady drag is needed to successfully remove cortex from capsular bag); (3) Amount of force maintained on the aspiration plunger (shows that too much force aspirates cortex into cannula, too little force will not hold onto cortex during dragging.
   6.3.1.7 Able to direct the cannula above or below the IOL
6.3.2 Cortex features same as 5.1.2.1.1 (type 1 cortex only) and 5.1.2.3.1
6.3.3 Viscoelastic features same as 3.6.3 - 3.6.4, 3.7.4 - 3.7.5, 5.2.7.1
6.3.4 AC features same as 4.1.9.1 - 4.1.9.3, 5.1.5.1
   6.3.4.1 Shows rapid AC collapse when initiating aspiration without cortex blocking the aspiration port (due to absence of inflow when using the 27 gauge cannula)
   6.3.4.2 Able to create AC turbulence when injecting debris out of cannula tip
6.3.5 Corneal paracentesis features same as 3.5.3

## Claims

1. A system for simulating a surgery, the system comprising:
a first haptic arm;
a first haptic mechanism in mechanical communication with the first haptic arm, wherein the haptic arm and haptic mechanism are capable of providing haptic force feedback to a user of the system during simulation of a surgery;
a haptic control unit capable of signaling to the first haptic mechanism a level of haptic force feedback;
a first computer in electronic communication with the haptic control unit and comprising a software module capable of generating a physics-based model of a portion of the human body and calculating the haptic force feedback to be provided by the first haptic arm and first haptic mechanism, wherein the calculated haptic force feedback corresponds to substantially the same forces exerted on a surgical tool when it interacts with the portion of the human body during live surgery; and
a simulated microscope capable of displaying a three-dimensional image of the portion of the human body and a simulated first surgical tool simulated by the haptic arm.

2. The system of Claim 1 where in the portion of the human body modeled by the first computer is an eye.

3. The system of Claim 2 wherein rasterization is used to model the eye.

4. The system of Claim 2 wherein ray tracing is used to model the eye.

5. The system of Claim 1 wherein the haptic force feedback calculated by the first computer depends on an angle of the haptic arm relative to the modeled portion of the human body and a direction in which the haptic arm is moved relative to the modeled portion of the human body.

6. The system of Claim 1 wherein the first haptic arm has a first handle corresponding to the handle of a first surgical instrument.

7. The system of Claim 6 wherein the handle of the first haptic arm is adapted to be detachable from the first haptic arm and replaced by a second handle corresponding to the handle of a second surgical instrument.

8. The system of Claim 7 wherein the simulated surgical tool displayed in the simulated microscope displays a simulated second surgical tool when the second handle is attached to the haptic arm

9. A method for simulating manual small incision cataract surgery, the method comprising:
providing a first haptic arm capable of providing haptic force feedback;
displaying in a simulated microscope a three-dimensional simulated model of an eye;
displaying in the simulated microscope a simulated image of a surgical tool controlled by the first haptic arm;
generating a physics-based model of the eye wherein the model simulates interactions between an eye and a surgical tool;
calculating haptic force feedback to be provided by the first haptic arm, wherein the haptic force feedback is calculated based on the position of the haptic arm and the direction in which the haptic arm travels relative to the simulated eye; and
using the haptic arm to control the simulated image of a surgical tool, simulating the steps of creating a self-sealing tunnel into the simulated eye, removing a cataract from the simulated eye, and inserting a simulated intraocular lens into the simulated eye.

10. The method of Claim 9 wherein rasterization is used to generate the three-dimensional simulated model of the eye.

11. The method of Claim 9 wherein ray tracing is used to generate the three-dimensional simulated model of the eye.
